(19) Europäisches Patentamt
European Patent Office
Office européen des brevets

(11) EP 0 296 110 B1

(12) EUROPÄISCHE PATENTSCHRIFT

(45) Veröffentlichungstag und Bekanntmachung des Hinweises auf die Patenterteilung:
**21.02.1996 Patentblatt 1996/08**

(51) Int Cl.[6]: **C07D 498/22**, A61K 31/55 // (C07D498/00, 311:00, 273:00, 209:00, 209:00, 209:00)

(21) Anmeldenummer: **88810383.5**

(22) Anmeldetag: **09.06.1988**

# (54) An Methylamino-Stickstoff substituierte Derivate von Staurosporin

Staurosporine derivatives substituted for the nitrogen atom of the methylamino group

Dérivés de staurosporine substitués à l'azote du méthylamino

(84) Benannte Vertragsstaaten:
**AT BE CH DE ES FR GB GR IT LI LU NL SE**

(30) Priorität: **15.06.1987 CH 2244/87**
**19.04.1988 CH 1440/88**

(43) Veröffentlichungstag der Anmeldung:
**21.12.1988 Patentblatt 1988/51**

(73) Patentinhaber: **CIBA-GEIGY AG**
**CH-4002 Basel (CH)**

(72) Erfinder:
- **Caravatti, Giorgio, Dr.**
**CH-4123 Allschwil (CH)**
- **Fredenhagen, Andreas, Dr.**
**CH-4051 Basel (CH)**

(56) Entgegenhaltungen:
**WO-A-89/07105**
- **CHEMICAL ABSTRACTS, Band 104, 1986, Seite 269, Zusammenfassung Nr. 18288w, Columbus, Ohio, US; T. TAMAOKI et al.: "Staurosporine, a potent inhibitor of phospholipid/calcium dependent protein kinase"**
- **Römpps Chemie-Lexikon, 8. Aufl., Stuttgart, 1979, S. 60/61, 593**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft am Methylamino-Stickstoff substituierte Derivate von Staurosporin, insbesondere solche der allgemeinen Formel

$$[Stau]\text{-}N(CH_3)\text{-}R \qquad (I),$$

worin [Stau] der Rest der Teilformel

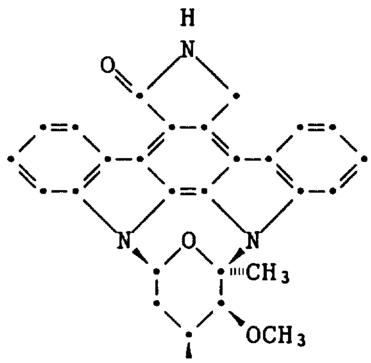

[Stau]

und R ein acylischer, carbocyclischer oder carbocyclisch-acyclischer Kohlenwasserstoffrest $R^0$ ist, der insgesamt höchstens 30 Kohlenstoffatome hat und gesättigt oder ungesättigt und unsubstituiert oder substituiert sein kann, wobei anstelle von einem, zwei oder mehreren Kohlenstoffatomen gleiche oder verschiedene Heteroatome ausgewählt aus Sauerstoff, Schwefel und Stickstoff vorliegen können; oder ein Acyl Ac, welches höchstens 30 Kohlenstoffatome aufweist, darstellt; sowie Salze von Verbindungen der Formel I mit salzbildenden Eigenschaften.

Die Erfindung betrifft auch Verfahren zur Herstellung der oben definierten Verbindungen, diese Verbindungen enthaltende pharmazeutische Präparate und deren Herstellung, medizinische Anwendung dieser Verbindungen und Präparate, sowie die entsprechende therapeutische Methode.

Staurosporin der Formel [Stau]-NH-$CH_3$ (II) (für die Bedeutung des Rests [Stau] siehe oben) als Grundstoff der erfindungsgemässen Verbindungen wurde bereits im Jahre 1977 aus den Kulturen von Streptomyces staurosporeus AWAYA, TAKAHASHI and Ō MURA, sp. nov. AM 2282, vgl. Omura, S.; Iwai, Y.; Hirano, A.; Nakagawa, A.; Awaya, J.; Tsuchiya, H.; Takahashi, Y.; und Masuma, R.: J. Antibiot. 30, 275-281 (1977), isoliert und auf antimikrobielle Wirksamkeit geprüft. Es wurde dabei auch gefunden, dass die Verbindung gegen hefeartige Mikroorganismen und Pilze wirksam ist (MIC von etwa 3-25 mcg/ml), wobei sie als Hydrochlorid eine $LD_{50}$ = 6,6 mg/kg (Maus, intraperitoneal) aufweist. In letzer Zeit hat sich bei ausgedehntem Screening gezeigt, vgl. Tamaoki, T.; Nomoto, H.; Takahashi, I; Kato, Y; Morimoto, M.; und Tomita, F.: Biochem. and Biophys. Research Commun. 135 (No. 2), 397-402 (1986), dass die Verbindung eine starke inhibierende Wirkung auf Proteinkinase C (aus Rattenhirn) ausübt.

Die von Phospholipiden und Calcium abhängige Proteinkinase C kommt innerhalb der Zelle in mehreren Formen vor und beteiligt sich an verschiedenen fundamentalen Vorgängen davon, wie Signalübertragung, Proliferation und Differenzierung, sowie auch Ausschüttung von Hormonen und Neurotransmittern. - Die Aktivierung dieser Enzyme erfolgt bekanntlich entweder durch eine über Rezeptoren vermittelte Hydrolyse von Phospholipiden der Zellenmembran oder durch eine direkte Interaktion mit gewissen Tumor-fördernden Wirkstoffen. Die Empfindlichkeit der Zelle gegenüber der rezeptorenvermittelten Signalübertragung kann durch die Abwandlung der Aktivität von Proteinkinase C (als Signalübertrager) wesentlich beeinflusst werden. Verbindungen, die fähig sind, die Aktivität der Proteinkinase C selektiv abzuwandeln, können ihre Anwendung als tumorhemmende, entzündungshemmende, immunomodulierende und antibakterielle Wirkstoffe finden und sogar als Mittel gegen Atherosklerose und Krankheiten des kardiovaskulären Systems und zentralen Nevensystems von Interesse sein.

Obwohl Staurosporin eine starke Hemmwirkung auf Proteinkinase C aufweist (siehe oben), hemmt es ebenso stark auch andere Proteinkinasen und hat deshalb nicht die Selektivität, welche für eine therapeutische Anwendung notwendig wäre. - Ueberraschenderweise hat es sich nun gezeigt, dass Entfernen des Wasserstoffs, in der Methylaminogruppe von Staurosporin durch Substitution zur Folge hat, dass derartig N-substituierte Derivate zwar die Hemmaktivität des Staurosporins gegenüber Proteinkinase C selektiv behalten, aber gegenüber anderen Proteinkinasen wesentlich weniger wirksam sind. Infolge dieser signifikant erhöhten Selektivität erfüllen die erfindungsgemässen Verbindungen somit auch die wichtige Bedingung für die therapeutische Anwendung in den oben erwähnten Indikationsbereichen, in erster Linie zur Beeinflussung der Zellenproliferation.

Zur Bestimmung der Proteinkinase-C-Hemmwirkung wurde zunächst Proteinkinase C aus Schweinehirn gewonnen und gemäss der von T. Uchida und C.R. Filburn in J. Biol. Chem. 259, 12311-4 (1984) beschriebenen Verfahrensweise

gereinigt. Zur Bestimmung der Proteinkinase-Hemmwirkung der Verbindungen der Formel I wurde die Methodik von D. Fabro et al. gemäss Arch. Biochem. Biophys. 239, 102-111 (1985) zugrundegelegt. Eine signifikante Proteinkinase-C-Hemmung erfolgte ab einer Konzentration von etwa 0,01 μM/l

Dementsprechend können die Verbindungen der Formel I und ihre pharmazeutisch verwendbaren Salze z.B. als Arzneimittel, insbesondere zur tumorhemmenden, entzündungshemmenden, immunomodulierenden, antibakteriellen Anwendung, ferner als Mittel gegen Artherosklerose, Krankheiten des kardiovaskulären Systems und des zentralen Nervensystems, verwendet werden. Ein weiterer Gegenstand der vorliegenden Erfindung ist die Verwendung der erfindungsgemässen Verbindungen zur Herstellung von Arzneimitteln, z.B. zur vorstehend aufgeführten Anwendung, zur therapeutischen und prophylaktischen Behandlung des menschlichen, ferner tierischen, Körpers. Dabei kann auch die gewerbsmässige Herrichtung der Wirksubstanzen eingschlossen sein.

Das Hydrocarbyl (Kohlenwasserstoffrest) $R^0$ ist ein acyclischer (aliphatischer), carbocyclischer oder carbocyclish-acyclischer Kohlenwasserstoffrest, der insgesamt vorzugsweise höchstens 30, insbesondere höchstens 18, Kohlenstoffatome hat und gesättigt oder ungesättigt, unsubstituiert oder substituiert sein kann. Er kann auch anstelle von einem, zwei oder mehreren Kohlenstoffatomen gleiche oder verschiedene Heteroatome, wie insbesondere Sauerstoff, Schwefel und Stickstoff, im acyclischen und/oder cyclischen Teil enthalten; im letzteren Fall wird er als ein heterocyclischer Rest (Heterocyclylrest) oder ein heterocyclisch-acyclischer Rest bezeichnet.

Ungesättigte Reste sind solche, die eine oder mehrere, insbesondere konjugierte und/oder isolierte, Mehrfachbindungen (Doppel- und/oder Dreifachbindungen) enthalten. Der Begriff cyclische Reste umfasst auch aromatische Reste, z.B. solche, worin mindestens ein 6-gliedriger carbocyclischer oder ein 5-bis 8-gliedriger heterocyclischer Ring die maximale Anzahl nichtcumulierter Doppelbindungen enthält. Carbocyclische Reste, worin mindestens ein Ring als ein 6-gliedriger aromatischer Ring (d.h. Benzolring) vorliegt, werden als Arylreste bezeichnet.

Wenn nicht anders angegeben, enthalten in der vorliegenden Offenbarung mit "nieder" bezeichnete organische Reste höchstens 7, vorzugsweise höchstens 4, Kohlenstoffatome.

Ein acyclischer unsubstituierter Kohlenwasserstoffrest ist inbesondere ein gerader oder verzweigter Niederalkyl-, Niederalkenyl-, Niederalkadienyl- oder Niederalkynylrest. Niederalkyl ist z.B. Methyl, Ethyl, n-Propyl, Isopropyl, n-Butyl, Isobutyl, sek-Butyl oder tert-Butyl, weiter auch n-Pentyl, Isopentyl, n-Hexyl, Isohexyl und n-Heptyl; Niederalkenyl ist z.B. Allyl, Propenyl, Isopropenyl, 2- oder 3-Methallyl und 2- oder 3-Butenyl; Niederalkadienyl ist z.B. 1-Penta-2,4-dienyl; Niederalkynyl ist z.B. Propargyl oder 2-Butynyl. In entsprechenden ungesättigten Resten ist die Doppelbindung insbesondere in höherer als der α-Stellung zur freien Valenz lokalisiert.

Ein carbocyclischer Kohlenwasserstoffrest ist insbesondere ein mono-, bi- oder polycyclischer Cycloalkyl-, Cycloalkenyl- oder Cycloalkadienylrest, oder ein entsprechender Arylrest. Bevorzugt sind Reste mit höchstens 14, insbesondere 12, Ringkohlenstoffatomen und 3- bis 8-, vorzugsweise 5-bis 7, vor allem 6-gliedrigen Ringen, wobei sie auch einen oder mehrere, z.B. zwei, acyclische Reste, z.B. die oben genannten, und insbesondere die Niederalkylreste, oder weitere carbocyclische Reste tragen können. Carbocyclisch-acyclische Reste sind solche, in welchen ein acyclischer Rest, insbesondere einer mit höchstens 7, vorzugsweise höchstens 4 Kohlenstoffatomen, wie vor allem Methyl, Ethyl und Vinyl, einen oder mehrere carbocyclische, gegebenenfalls aromatische Reste der obigen Definition trägt. Insbesondere sind Cycloalkyl-niederalkyl- und Arylniederalkylreste, sowie ihre im Ring und/oder Kette ungesättigten Analogen, welche den Ring am endständigen C-Atom der Kette tragen, zu erwähnen.

Cycloalkyl weist in erster Linie 3 bis und mit 10 C-Atome auf und ist z.B. Cyclopropyl, Cyclobutyl, Cyclopentyl, Cyclohexyl, Cycloheptyl und Cyclooctyl, sowie Bicyclo[2,2,2]octyl, 2-Bicyclo[2,2,1]heptyl und Adamantyl, welche auch durch 1, 2 oder mehrere, z.B. niedere, Alkylreste, vor allem Methylreste, substituiert sein können; Cycloalkenyl ist z.B. einer der bereits genannten monocyclischen Cycloalkylreste, der eine Doppelbindung in 1-, 2- oder 3-Stellung trägt. Cycloalkyl-niederalkyl oder -niederalkenyl ist z.B. ein durch einen der oben genannten Cycloalkylreste substituiertes -methyl, -1- oder -2-ethyl, -1- oder -2-vinyl, -1-, -2- oder -3-propyl bzw. -allyl, wobei die am Ende der linearen Kette substituierten bevorzugt sind.

Ein Arylrest ist in erster Linie ein Phenyl, ferner ein Naphthyl, wie 1-oder 2-Naphthyl, ein Biphenylyl, wie insbesondere 4-Biphenylyl, weiter auch ein Anthryl, Fluorenyl und Azulenyl, sowie deren jeweiliges aromatisches Analoges mit einem oder mehreren gesättigten Ringen. Bevorzugte Aryl-niederalkyl- und niederalkenyl-Reste sind z.B. Phenylniederalkyl oder Phenyl-niederalkenyl mit endständigem Phenylrest, wie z.B. Benzyl, Phenethyl, 1-, 2- oder 3-Phenylpropyl, Diphenylmethyl (Benzhydryl), Trityl und Cinnamyl, ferner auch 1- oder 2-Naphthylmethyl. Unter Arylresten, die acyclische Reste, wie Niederalkyl, tragen, sind insbesondere o-, m- und p-Tolyl und Xylylreste mit verschieden situierten Methylresten zu erwähnen.

Heterocyclische Reste, einschliesslich heterocyclisch-acyclische Reste, sind insbesondere monocyclische, aber auch bi- oder polycyclische, aza-, thia-, oxa-, thiaza-, oxaza-, diaza-, triaza- oder tetrazacyclische Reste aromatischen Charakters, sowie entsprechende partiell oder in erster Linie ganz gesättigte heterocyclische Reste dieser Art, wobei solche Reste gegebenenfalls, z.B. wie die obgenannten carbocyclischen oder Arylreste, weitere acyclische, carbocyclische oder heterocyclische Reste tragen können und/oder durch funktionelle Gruppen mono-, di- oder polysubstituiert sein können. Der acyclische Teil in heterocyclisch-acyclischen Resten hat z.B. die für die entsprechenden carbocyclischacyclischen Reste gegebene Bedeutung. Sofern sich ein Heterocyclyl als ein direkter Substituent $R^0$ an der Methylaminogruppe des Staurosporins befindet, so muss seine freie Valenz von einem seiner C-Atome ausgehen. In erster Linie sind es unsubstituierte oder substituierte monocyclische Reste mit einem Stickstoff-, Sauerstoff- oder Schwefelatom, wie 2-Aziridinyl, und insbesondere aromatische Reste dieser Art, wie Pyrryl, z.B. 2-Pyrryl oder 3-Pyrryl, Pyridyl, z.B. 2-, 3- oder 4-Pyridyl, ferner Thienyl, z.B. 2- oder 3-Thienyl, oder Furyl, z.B. 2-Furyl; analoge bicyclische Reste mit einem Stickstoff-, Sauerstoff- oder Schwefelatom sind z.B. Indolyl, wie 2- oder 3-Indolyl, Chinolyl, wie 2- oder 4-Chinolyl, Isochinolyl, wie 3- oder 5-Isochinolyl, Benzofuranyl, wie 2-Benzofuranyl, Chromenyl, wie 3-Chromenyl, oder Benzothienyl, wie 2- oder 3-Benzothienyl; bevorzugte monocyclische und bicyclische Reste mit mehreren Heteroatomen sind z.B. Imidazolyl, wie 2-Imidazolyl, Pyrimidinyl, wie 2-oder 4-Pyrimidinyl, Oxazolyl, wie 2-Oxazolyl, Isoxazolyl, wie 3-Isoxazolyl, oder Thiazolyl, wie 2-Thiazolyl, bzw. Benzimidazolyl, wie 2-Benzimidazolyl, Benzoxazolyl, wie 2-Benzoxazolyl, oder Chinazolyl, wie 2-Chinazolinyl. Entsprechende partiell oder, insbesondere, ganz gesättigte analoge Reste kommen auch in Betracht, wie 2-Tetrahydrofuryl, 2- oder 3-Pyrrolidyl, 2-, 3-, oder 4-Piperidyl, sowie auch 2-oder 3-Morpholinyl, 2- oder 3-Thiomorpholinyl, 2-Piperazinyl und N,N′-Bis-niederalkyl-2-piperazinyl-Reste. Diese Reste können auch einen oder mehrere acyclische, carbocyclische oder heterocyclische Reste, insbesondere die oben genannten, tragen. Heterocyclisch-acyclische Reste sind insbesondere von acyclischen Resten mit höchstens 7, vorzugsweise höchstens 4 Kohlenstoffatomen, z.B. von den oben genannten, abgeleitet und können einen, zwei oder mehrere heterocyclische Reste, z.B. die oben genannten, tragen, wobei der Ring zur Kette auch durch einen seiner Stickstoffatome gebunden werden kann.

Wie bereits erwähnt wurde, kann ein Hydrocarbyl (einschliesslich eines Heterocyclyls) durch einen, zwei oder mehrere gleichartige oder verschiedenartige Substituenten (funktionelle Gruppen) substituiert sein; die folgenden Substituenten kommen insbesondere in Betracht: freie, veretherte und veresterte Hydroxylgruppen; Mercapto- sowie Niederalkylthio- und gegebenenfalls substituierte Phenylthiogruppen; Halogenatome, wie Chlor und Fluor, aber auch Brom und Jod; Oxogruppen, welche in der Form von Formyl- (d.h. Aldehydo-) und Keto-gruppen, auch als entsprechende Acetale bzw. Ketale vorliegen; Azido- und Nitrogruppen; primäre, sekundäre und vorzugsweise tertiäre Aminogruppen, durch konventionelle Schutzgruppen geschützte primäre oder sekundäre Aminogruppen, Acylaminogruppen und Diacylaminogruppen, sowie gegebenenfalls funktionell abgewandelte Sulfogruppen, wie Sulfamoyl- oder in Salzform vorliegende Sulfogruppen. Alle diese funktionellen Gruppen dürfen sich nicht am C-Atom befinden, von dem die freie Valenz ausgeht, vorzugsweise sind sie von dieser durch 2- oder auch mehrere C-Atome getrennt. Der Hydrocarbylrest kann auch freie und funktionell abgewandelte Carboxylgruppen, wie in Salzform vorliegende oder veresterte Carboxylgruppen, gegebenenfalls einen oder zwei Kohlenwasserstoffreste tragende Carbamoyl-, Ureido- oder Guanidinogruppen, und Cyangruppen tragen.

Eine als Substituent im Hydrocarbyl vorliegende veretherte Hydroxylgruppe ist z.B. eine Niederalkoxygruppe, wie die Methoxy-, Ethoxy-, Propoxy-, Isopropoxy-, Butoxy- und tert-Butoxygruppe, welche auch substituiert sein kann. So kann eine solche Niederalkoxygruppe durch Halogenatome, z.B. ein-, zwei- oder mehrfach, insbesondere in 2-Stellung, wie im 2,2,2-Trichlorethoxy-, 2-Chlorethoxy- oder 2-Jodethoxyrest, oder durch Hydroxy bzw. Niederalkoxyreste, jeweils vorzugsweise einfach, insbesondere in 2-Stellung, wie im 2-Methoxyethoxyrest, substituiert sein. Eine besonders bevorzugte Ausgestaltung der veretherten Hydroxylgruppen liegt in Oxaalkylresten vor, in welchen in einem, vorzugsweise linearen, Alkyl ein oder mehrere C-Atome durch Sauerstoffatome ersetzt sind, die vorzugsweise durch mehrere (vor allem 2) C-Atome voneinander getrennt sind, so dass sie eine, gegebenenfalls mehrfach sich wiederholende Gruppe

$$-(-O-CH_2CH_2-)_n-,$$

worin n = 1 bis 14 ist, bilden. Ferner sind solche veretherte Hydroxylgruppen auch gegebenenfalls substituierte Phenoxyreste und Phenylniederalkoxyreste, wie vor allem Benzyloxy, Benzhydryloxy und Triphenylmethoxy (Trityloxy), sowie Heterocyclyloxyreste, wie insbesondere 2-Tetrahydropyranyloxy. Als eine besondere veretherte Hydroxylgruppe ist die Gruppierung Methylendioxy und Ethylendioxy zu bezeichnen, wobei erstere in der Regel 2 benachbarte C-Atome überbrückt, insbesondere in Arylresten, und letztere an einem und demselben C-Atom gebunden und als Schutzgruppe für Oxo anzusehen ist.

Unter veretherten Hydroxylgruppen sind in diesem Zusammenhang auch silylierte Hydroxylgruppen zu verstehen, wie sie z.B. in Triniederalkylsilyloxy, wie Trimethylsilyloxy und Dimethyl-tert-butylsilyloxy, oder Phenyldiniederalkylsilyloxy bzw. Niederalkyl-diphenylsilyloxy vorliegen.

Eine als Substituent im Hydrocarbyl vorliegende veresterte Hydroxylgruppe trägt einen weiter unten definierten Acylrest Ac, insbesondere einen mit höchstens 12 C-Atomen, oder ist durch eine im Hydrocarbyl auch anwesende Carboxylgruppe lactonisiert.

Eine als Substituent im Hydrocarbyl vorliegende veresterte Carboxylgruppe ist eine solche, in welcher das Wasserstoffatom durch einen der oben charakterisierten Kohlenwasserstoffreste, vorzugsweise einen Niederalkyl- oder Phenylniederalkylrest, ersetzt ist; als Beispiel einer veresterten Carboxylgruppe ist z.B. Niederalkoxycarbonyl oder gegebenenfalls im Phenylteil substituiertes Phenylniederalkoxycarbonyl, insbesondere die Methoxy-, Ethoxy-, tert-Butoxy- und Benzyloxycarbonylgruppe, sowie auch eine lactonisierte Carboxylgruppe zu nennen.

Eine primäre Aminogruppe $-NH_2$ als Substituent des Hydrocarbyls kann auch in geschützter Form als eine dieser Gruppe entsprechende Acylaminogruppe der Formel -NH-Ac°, worin Ac° die nachstehend charakterisierte Bedeutung hat, vorliegen. Eine sekundäre Aminogruppe trägt anstelle eines der beiden Wasserstoffatome einen Hydrocarbylrest, vorzugsweise einen unsubstituierten, wie einen der oben genannten, insbesondere Niederalkyl, und kann auch in einer geschützten Form als eine davon abgeleitete Acylaminogruppe mit einem nachstehend charakterisierten monovalenten Acylrest Ac° vorliegen.

Der als Amino-Schutzgruppe dienende Acylrest Ac° leitet sich vorzugsweise von einem Kohlensäurehalbderivat ab und ist vorzugsweise ein gegebenenfalls, insbesondere durch Niederalkyl, Niederalkoxy, Nitro und/oder Halogen, substituiertes Niederalkoxycarbonyl oder Arylniederalkoxycarbonyl, wie Methoxycarbonyl, Ethoxycarbonyl, tert-Butoxycarbonyl, 2,2,2-Trichlorethoxycarbonyl, 2-Jodethoxycarbonyl, Benzyloxycarbonyl, 2-Phenyl-2-propoxycarbonyl, 2-p-Tolyl-2-propoxycarbonyl, 2-(p-Biphenylyl)-2-propoxycarbonyl oder 9-Fluorenylmethoxycarbonyl.

Eine als Substituent im Hydrocarbyl vorkommende tertiäre Aminogruppe trägt 2 verschiedene oder, vorzugsweise, gleiche Hydrocarbylreste (einschliesslich der heterocyclischen Reste), wie die oben charakterisierten unsubstituierten Hydrocarbylreste, insbesondere Niederalkyl.

Eine bevorzugte Aminogruppe ist eine solche der Formel

$$R^1-\overset{|}{N}-R^2 ,$$

worin $R^1$ und $R^2$ unabhängig je Wasserstoff, unsubstituiertes acyclisches $C_1$-$C_7$-Hydrocarbyl (wie insbesondere ein $C_1$-$C_4$-Alkyl oder $C_1$-$C_4$-Alkenyl) oder monocyclisches, gegebenenfalls durch $C_1$-$C_4$-Alkyl, $C_1$-$C_4$-Alkoxy, Halogen und/oder Nitro substituiertes Aryl, Aralkyl oder Aralkenyl mit höchstens 10 C-Atomen darstellen, wobei die kohlenstoffhaltigen Reste durch eine Kohlenstoff-Kohlenstoff-Bindung oder ein Sauerstoff-, Schwefel- oder gegebenenfalls durch Hydrocarbyl substituiertes Stickstoffatom untereinander gebunden sein können. In einem solchen Fall bilden sie zusammen mit dem Stickstoffatom der Aminogruppe einen stickstoffhaltigen heterocyclischen Ring. Als Beispiel von besonders bevorzugten freien Aminogruppen sind die folgenden zu nennen: Diniederalkylamino, wie Dimethylamino, Diethylamino, Pyrrolidino, Piperidino, Morpholino, Thiomorpholino und Piperazino oder 4-Methylpiperazino, gegebenenfalls, insbesondere im Phenylteil, z.B. durch Niederalkyl, Niederalkoxy, Halogen und/oder Nitro substituierte Diphenylamino und Dibenzylamino; unter den geschützten dann insbesondere Niederalkoxycarbonylamino, wie tert-Butoxycarbonylamino, Phenylniederalkoxycarbonylamino, wie 4-Methoxybenzyloxycarbonylamino, sowie 9-Fluorenylmethoxycarbonylamino.

Sofern nicht abweichend aufgeführt können vor- und nachfolgend aromatische carbocyclische und heterocyclische Hydroxycarbylreste ein- oder mehrfach, wie zwei- oder dreifach, substituiert sein, insbesondere durch $C_1$-$C_4$-Alkyl, $C_1$-$C_4$-Alkoxy, Halogen, Nitro, Trifluormethyl, ferner Carboxy, $C_1$-$C_4$-Alkoxycarbonyl, Methylendioxy, und/oder Cyano. Vor- und nachstehend angegebene reduzierte Angaben über Substituenten sind als Bevorzugungen anzusehen.

Bevorzugte erfindungsgemässe Verbindungen der Formel I sind z.B. solche, worin R ein Hydrocarbyl $R^0$ mit den folgenden bevorzugten Bedeutungen eines acyclischen Hydrocarbyls darstellt: ein $C_1$-$C_{20}$-Alkyl, ein $C_2$-$C_{20}$-Hydroxyalkyl, dessen Hydroxylgruppe sich in beliebiger Stellung ausser 1-Stellung, vorzugsweise in 2-Stellung befindet, ein Cyano-[$C_1$-$C_{20}$]-alkyl, dessen Cyanogruppe sich vorzugsweise in 1- oder ω-Stellung befindet, oder ein Carboxy-[$C_1$-$C_{20}$]-alkyl, dessen Carboxylgruppe sich vorzugsweise in 1- oder ω-Stellung befindet und gegebenenfalls auch in Salzform oder als ein $C_1$-$C_4$-Alkylester ($C_1$-$C_4$-Alkoxycarbonyl) oder Benzylester (Benzyloxycarbonyl) vorliegen kann, sowie ein $C_3$-$C_{20}$-Alkenyl, dessen freie Valenz sich nicht an demselben C-Atom wie die Doppelbindung befindet, wobei alle genannten Reste, ausgenommen diejenigen mit der $C_3$-$C_5$-Alkyl-Grundstruktur, eine lineare (unverzweigte) Alkylkette aufweisen; weiter auch ein lineares (Mono-, Di- bis Hexa)-oxaalkyl mit 4-20 Kettengliedern, worin eines oder mehrere der C-Atome, von C-3 an, eines linearen $C_4$-$C_{20}$-Alkyls durch Sauerstoffatome, welche voneinander durch mindestens 2 C-Atome getrennt sind und vorzugsweise sich in Stellungen 3-, 6-, 9-, 12-, 15- und 18- befinden, ersetzt ist.

Bevorzugte erfindungsgemässe Verbindungen der Formel I sind auch solche, worin R ein Hydrocarbyl $R^0$ mit den folgenden bevorzugten Bedeutungen eines carbocyclischen oder heterocyclischen, sowie auch carbocyclisch-acyclischen bzw. heterocyclisch-acyclischen Hydrocarbyls darstellt: ein bicyclisches oder vorzugsweise monocyclisches Aryl, vor allem Phenyl, ferner Naphthyl, welches einen oder mehrere der folgenden Substituenten tragen kann: Halogenatome, insbesondere Fluor, Chlor und Brom, $C_1$-$C_4$-Alkylreste, insbesondere Methyl, $C_1$-$C_4$-Alkoxygruppen, insbesondere Methoxy, Methylendioxy, Nitrogruppen und/oder Carboxylgruppen, welche frei, in einer Salzform, oder als $C_1$-$C_4$-Alkylester, insbesondere Methoxycarbonyl oder Ethoxycarbonyl, vorliegen können. Vorzugsweise tragen die Arylreste nicht mehr als 2 Substituenten, insbesondere solche gleicher Art, oder nur einen einzigen; vor allem sind sie unsubstituiert. Als ein bevorzugtes heterocyclisches Hydrocarbyl (Heterocyclyl) kommt z.B. ein solches in Betracht, welches den oben hervorgehobenen Arylresten analog ist und anstelle eines oder 2 C-Atome je ein Heteroatom, insbesondere Stickstoff, enthält, wie ein Pyridyl oder Chinolyl, bzw. Chinazolyl, wobei die freie Valenz an einem C-Atom lokalisiert ist, und auch dementsprechend substituiert sein kann. Bevorzugte carbocyclisch-acyclische und heterocyclisch-acyclische Hydrocarbylreste sind solche, worin zwei oder drei, vorzugsweise aber nur einer, der oben definierten cyclischen Reste, vorzugsweise der unsubstituierten, durch ein $C_1$-$C_3$-Alkyl getragen werden, wobei sie vorzugsweise alle an einem C-Atom, vorzugsweise dem endständigen, lokalisiert sind; am meisten bevorzugt ist unsubstituiertes Benzyl.

Besonders bevorzugte Verbindungen der Formel I sind solche, worin $R^0$ $C_1$-$C_7$-Alkyl, insbesondere $C_1$-$C_4$-Alkyl, Hydroxy-$C_2$-$C_{18}$-alkyl, insbesondere Hydroxy-$C_2$-$C_{14}$-alkyl, Cyano-$C_1$-$C_7$-alkyl, insbesondere Cyano-$C_1$-$C_4$-alkyl, Carboxy-$C_1$-$C_7$-alkyl, insbesondere Carboxy-$C_1$-$C_4$-alkyl, C1-C7-Alkoxy-carbonyl-$C_1$-$C_7$-alkyl, insbesondere $C_1$-$C_4$-Alkoxy-carbonyl-$C_1$-$C_4$-alkyl, Benzyloxy-carbonyl-$C_1$-$C_7$-alkyl, insbesondere Benzyloxycarbonyl-$C_1$-$C_4$-alkyl, $C_3$-$C_7$-Alkenyl, Phenyl, Naphthyl, Pyridyl, Chinolyl, bzw. Chinazolyl, oder Phenyl-$C_1$-$C_7$-alkyl, insbesondere Phenyl-$C_1$-$C_3$-alkyl, bedeutet, wobei die jeweiligen aromatischen Reste ferner auch durch $C_1$-$C_7$-Alkyl, insbesondere $C_1$-$C_4$-Alkyl, $C_1$-$C_7$-Alkoxy, insbesondere $C_1$-$C_4$-Alkoxy, Halogen, Nitro, Trifluormethyl, ferner Carboxy, $C_1$-$C_4$-Alkoxy-carbonyl, Methylendioxy und/oder Cyano substituiert sein können, wobei die Hydroxygruppe im entsprechend substituierten Alkylrest insbesondere in 2-Stellung und wobei die Cyano-, Carboxy-, Alkoxy-carbonyl-, Benzyloxy-carbonyl- bzw. Phenylgruppe im entsprechend substituierten Alkylrest insbesondere in 1-oder ω-Stellung lokalisiert ist.

Besonders bevorzugte Verbindungen der Formel I sind solche, worin $R^0$ $C_1$-$C_4$-Alkyl, wie Methyl oder Ethyl, Hydroxy-$C_2$-$C_{14}$-alkyl, wie 2-Hydroxy-propyl, -hexyl, -decyl oder -tetradecyl, Cyano-$C_1$-$C_4$-alkyl, wie 2-Cyano-ethyl, Carboxy-$C_1$-$C_4$-alkyl, wie Carboxymethyl, $C_1$-$C_4$-Alkoxycarbonyl-$C_1$-$C_4$-alkyl, wie Methoxycarbonyl-methyl oder -ethyl, $C_3$-$C_7$-Alkenyl, wie Allyl, oder Phenyl bedeutet, wobei die Hydroxygruppe im entsprechend substituierten Alkyl vorzugsweise in 2-Stellung und die Cyano-, Carboxy bzw. Alkoxycarbonylgruppe insbesondere in 1- oder ω-Stellung lokalisiert ist.

Der Acylrest Ac leitet sich von einer gegebenenfalls funktionell abgewandelten Carbonsäure, einer organischen Sulfonsäure oder einer gegebenenfalls veresterten Phosphorsäure, wie Pyro- oder Orthophosphorsäure, ab und weist vorzugsweise höchstens 30 C-Atome auf.

Ein von einer gegebenenfalls funktionell abgewandelten Carbonsäure abgeleitetes Acyl, das als $Ac^1$ bezeichnet wird, ist insbesondere eines der Teilformel Z-C(=W)-, worin W Sauerstoff, Schwefel oder Imino und Z Wasserstoff, Hydrocarbyl $R^0$, Hydrocarbyloxy $R^0O$, eine Aminogruppe, insbesondere eine der Formel

$$R^1-\overset{|}{N}-R^2 ,$$

oder, falls W für Sauerstoff oder Schwefel steht, auch Chlor darstellen kann. Die Bedeutungen von $R^0$, $R^1$ und $R^2$ entsprechen den oben angegebenen allgemeinen und hervorgehobenen, wobei die letzteren auch hier im allgemeinen die bevorzugte Auswahl darstellen.

Ein von einer organischen Sulfonsäure abgeleitetes Acyl, das als $Ac^2$ bezeichnet wird, ist insbesondere eines der Teilformel $R^0$-$SO_2$-, worin $R^0$ ein Hydrocarbyl der oben angegebenen allgemeinen und hervorgehobenen Bedeutungen darstellt, wobei die letzteren auch hier im allgemeinen die bevorzugte Auswahl darstellen.

Ein von einer gegebenenfalls veresterten Phosphorsäure abgeleitetes Acyl, das als $Ac^3$ bezeichnet wird, ist insbesondere eines der Teilformel

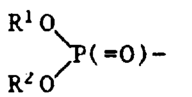 ,

worin $R^1$ und $R^2$ unabhängig die oben angegebenen allgemeinen und hervorgehobenen Bedeutungen haben. Vorzugsweise haben $R^1$ und $R^2$ gleiche Bedeutung.

Bevorzugte Acylreste $Ac^1$ sind Acylreste einer Carbonsäure, die durch die Teilformel $R_b^o$-CO- charakterisiert sind, worin $R_b^o$ entweder für Wasserstoff steht (und somit den Formylrest bildet) oder eine der obgenannten allgemeinen und bevorzugten Bedeutungen des Hydrocarbylrestes $R^o$ hat, und somit sich von einer gegebenenfalls substituierten acyclischen, carbocyclischen, carbocyclisch-acyclischen, heterocyclischen oder heterocyclisch-acyclischen Monocarbonsäure ableiten. Ein bevorzugtes Hydrocarbyl in einem solchen Acyl ist z.B. ein $C_1$-$C_{19}$-Alkyl, in erster Linie ein $C_1$-$C_7$- oder $C_1$-$C_4$-Alkyl, insbesondere ein solches, das bei mehr als 5 C-Atomen eine lineare Kette aufweist und welches auch die folgenden Substituenten tragen kann: eine Carboxylgruppe, die gegebenenfalls auch in Salzform oder als eine Cyanogruppe oder ein $C_1$-$C_4$-Alkylester ($C_1$-$C_4$-Alkoxycarbonylgruppe) vorliegen kann und welche sich vorzugsweise in ω-Stellung befindet, eine Aminogruppe der oben definierten Formel

$$R^1-\overset{|}{N}-R^2 ,$$

vorzugsweise eine solche, worin $R^1$ und $R^2$ je Wasserstoff ist und sich dann vorzugsweise in 1-Stellung befindet, oder ein oder mehrere Halogenatome, insbesondere Fluor oder Chlor, die sich vorzugsweise in der Nachbarschaft der Carbonylgruppe befinden. Ein anderes bevorzugtes Acyl ist ein bicyclisches oder insbesondere monocyclisches Aroyl, vor allem Benzoyl, welches auch einen oder mehrere der folgenden Substituenten tragen kann: Halogenatome, insbesondere Chlor oder Fluor, Nitrogruppen, $C_1$-$C_4$-Alkylreste, insbesondere Methyl, Hydroxylgruppen und veretherte Hydroxylgruppen, insbesondere $C_1$-$C_4$-Alkoxy, wie Methoxy, Phenoxy und Methylendioxy, sowie Carboxylgruppen, welche auch in der Salzform oder als eine Cyanogruppe oder ein $C_1$-$C_4$-Alkylester ($C_1$-$C_4$-Alkoxycarbonyl) vorliegen können. Vorzugsweise tragen die Aroylreste nicht mehr als 2, vor allem nur einen solchen Substituenten. Bevorzugt sind auch analoge Heteroaroylreste, insbesondere solche, die sich von Pyridin, Furan, Thiophen und Imidazol, und von ihren Analogen mit kondensiertem Benzoring (wie Chinolin, Isochinolin, Benzofuran und Benzimidazol) ableiten und gegebenenfalls auch, wie oben angegeben substituiert sind. Bevorzugte Acylreste dieser Art leiten sich auch von monocyclischen Aryl-alkenyl, z.B. entsprechendem Aryl-$C_2$-$C_5$-Alkenyl, wie Benzyl und Styryl (d.h. Phenacetyl und Cinnamoyl), ab, sie können auch in der oben angegebenen Weise substituiert sein. -Derartige Acylreste bilden mit der Grundstruktur des Staurosporins entsprechende Acylamide, wobei diejenigen mit den obengenannten Bedeutungen von $Ac^1$ besonders bevorzugt sind. Beispielsweise sind Staurosporin-amide zu nennen, die sich von den folgenden Carbonsäuren ableiten: aliphatische Monocarbonsäuren mit höchstens 20 Kohlenstoffatomen, wie Niederalkancarbonsäuren, z.B. die Propion-, Butter-, Isobutter-. Valerian-, Isovalerian, Capron-, Trimethylessig-, Oenanth- und Diethylessigsäure und vor allem die Essigsäure, sowie Laurin-, Myristin-, Palmitin- und Stearinsäure, sowie Oelsäure, Elaidinsäure, Linolsäure und Linolensäure, aber auch entsprechende halogenierte Niederalkancarbonsäuren, wie die Chloressigsäure, Trifluor- oder Trichloressigsäure, Bromessig- oder $\alpha$-Bromisovaleriansäure, carbocyclische oder carbocyclisch-acyclische Monocarbonsäuren, z.B. die Cyclopropan-, Cyclopentan- und Cyclohexan-carbonsäure bzw. die Cyclopentan oder Cyclohexan-essigsäure oder -propionsäure; aromatische carbocyclische Carbonsäuren, z.B. Benzoesäure, die einfach oder mehrfach, wie oben angegeben, substituiert sein kann; Aryl- oder Aryloxy-niederalkancarbonsäuren und deren in der Kette ungesättigte Analoga, z.B. gegebenenfalls, wie oben für die Benzoesäure angegeben, substituierte Phenylessig- bzw. Phenoxyessigsäuren, Phenylpropionsäuren und Zimtsäuren; und heterocyclische Säuren, z.B. Furan-2-carbonsäure, 5-tert-Butylfuran-2-carbonsäure, Thiophen-2-carbonsäure, Nicotin- oder Isonicotinsäure, 4-Pyridinpropionsäure, und gegebenenfalls durch Niederalkylreste substituierte Pyrrol-2-oder -3-carbonsäuren; ferner auch entsprechende $\alpha$-Aminosäuren, insbesondere die in der Natur vorkommenden $\alpha$-Aminosäuren der L-Reihe, z.B. Glycin, Phenylglycin, Alanin, Phenylalanin, Prolin, Leucin, Serin, Valin, Tyrosin, Arginin, Histidin und Asparagin, vorzugsweise in einer N-geschützten Form, d.h. in einer solchen, in welcher die Aminogruppe durch eine konventionelle, z.B. eine der obengenannten, Aminoschutzgruppe substituiert ist; weiter auch Dicarbonsäuren, wie Oxalsäure, Malonsäure, Mono- oder Di-niederalkylmalonsäuren, Bernsteinsäure, Glutarsäure, Adipinsäure, Erucasäure, Maleinsäure, eine durch Halogen, wie Fluor, Chlor oder Brom, und/oder Niederalkyl, Hydroxy, Niederalkoxy und Nitro gegebenenfalls substituierte Phthal-, Chinolin-, Isochinolin oder Phenylbernsteinsäure, sowie auch Glutaminsäuren und Asparginsäure, wobei die zwei letztgenannten Säuren vorzugsweise mit geschützten Aminogruppen vorliegen. Wie erwähnt, kann die zweite Carboxylgruppe nicht nur frei, sondern auch funktionell abgewandelt, z.B als ein $C_1$-$C_4$-Alkylester oder als ein Salz, vorzugsweise als ein physiologisch verträgliches Salz, mit einer salzbildenden basischen Komponente vorhanden sein. In Betracht kommen in erster Linie Metall- oder Ammoniumsalze, wie Alkalimetall-und Erdalkalimetall-, z.B. Natrium-, Kalium-, Magnesium- oder Calciumsalze, bzw. Ammoniumsalze mit Ammoniak oder geeigneten organischen Aminen.

Ein weiteres bevorzugtes Acyl $Ac^1$ leitet sich von Monoestern der Kohlensäure ab und ist durch die Teilformel $R^0$-O-CO- charakterisiert. Mit der Grundstruktur des Staurosporins bildet dieses Acyl dann entsprechende N-disubstituierte Urethane. Unter besonders bevorzugten Hydrocarbylresten $R^0$ in diesen Derivaten sind beispielsweise die folgenden zu nennen: acyclisches Hydrocarbyl, insbesondere ein $C_1$-$C_{20}$-Alkyl, vorzugsweise ein lineares, welches durch eine Carboxylgruppe, vorzugsweise in einer funktionell abgewandelter Form, wie Salz, Cyano oder $C_1$-$C_4$-Alkylester, die sich vorzugsweise in der $\omega$-Stellung befindet, substituiert sein kann, oder ein analoges lineares (Mono- bis Hexa-) -oxaalkyl mit 4-20 Kettengliedern, insbesondere ein solches, das als besonders bevorzugt oben charakterisiert wurde. Bevorzugt sind in dieser Bedeutung von $R^0$ auch gegebenenfalls substituierte Phenyl- und Benzylreste, z.B. die oben als bevorzugt erwähnten.

Ein noch weiteres bevorzugtes Acyl $Ac^1$ leitet sich von Amiden der Kohlensäure (oder auch Thiokohlensäure) ab und ist durch die Formel

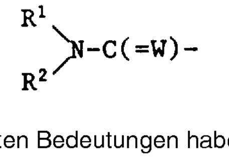

charakterisiert, worin $R^1$ und $R^2$ die obengenannten Bedeutungen haben und W für Schwefel und insbesondere Sauerstoff steht. Mit der Grundstruktur des Staurosporins bildet dieser Acylrest dann entsprechende Harnstoffe bzw. Thioharnstoffe.

Unter bevorzugten erfindungsgemässen Verbindungen, die dieses Acyl tragen, sind insbesondere solche hervorzuheben, worin W für Sauerstoff steht, einer der Reste $R^1$ und $R^2$ Wasserstoff ist und der andere ein $C_1$-$C_7$-Alkyl bedeutet, welches durch Hydroxyl, Mercapto, Methylthio, Phenyl, p-Hydroxyphenyl, p-Methoxyphenyl, 2-Indolyl, 2-Imidazolyl und vor allem durch Carboxyl (frei oder in einer funktionell abgewandelter Form, wie $C_1$-$C_4$-Alkoxycarbonyl, Carb-

amoyl oder Amidino), wovon eine sich vorzugsweise in 1-Stellung befindet, substituiert sein kann und vorzugsweise einem Radikal entspricht, dessen freie Valenz anstelle der Aminogruppe in einer geläufigen Aminsäure, wie β-Alanin, γ-Aminobuttersäure oder Norvalin, und insbesondere einer in der Natur als Peptid-Baustein vorkommenden α-Aminosäure der L-Reihe, oder einem Antipoden davon, steht. Hervorzuheben sind auch Verbindungen mit Acyl der letztgenannten Art, worin W für Schwefel steht, einer der Reste $R^1$ und $R^2$ Wasserstoff ist und der andere ein $C_1$-$C_7$-Alkyl oder insbesondere ein $C_3$-$C_7$-Alkenyl, in welchem die freie Valenz von einem anderen C-Atom als die Doppelbindung ausgeht, wie Allyl, darstellt.

Hervorzuheben sind auch die erfindungsgemässen Verbindungen der Formel I, worin R für Chloroformyl oder Thiochloroformyl steht, welche sich insbesonder als vorteilhafte Zwischenprodukte zur Herstellung von modifizierten Kohlensäure-acylestern auszeichnen.

Der Acylrest $Ac^2$ leitet sich von einer acyclischen, carbocyclischen, oder heterocyclischen, ferner auch einer carbocyclisch-acyclischen oder heterocyclisch-acyclischen Sulfonsäure ab und entpricht der erwähnten Teilformel $R^0$-$SO_2$-, worin $R^0$ für Hydrocarbyl der oben erwähnten allgemeinen und, insbesondere, der bevorzugten Bedeutungen steht. Unter den erfindungsgemässen Verbindungen, die den Rest $Ac^2$ tragen, sind solche besonders hervorzuheben, worin $R^0$ ein $C_1$-$C_7$-Alkyl, insbesondere ein lineares, ein bicyclisches oder insbesondere monocyclisches Aryl, wie insbesondere Phenyl, welches analog, wie oben für hervorgehobene Aroylreste geschildert wurde, substituiert sein kann. Hervorzuheben sind auch analog gebaute bicyclische und monocyclische aromatische Heterocyclylreste, in welchen eines oder zwei der C-Atome durch Heteroatome ersetzt sind, wie Pyrimidyl, z.B. 2- oder 4-Pyrimidyl, Chinolyl- oder Isochinolyl. Auch die Heterocyclylreste können Substituenten, insbesondere die für Aroyl hervorgehobenen, tragen (dabei ist z.B. ein Hydroxylderivat durch tautomere Verschiebung der Doppelbindung einem Dihydro-oxo-Derivat gleich).

Der von einer Phosphorsäure abgeleitete Acylrest $Ac^3$ ist beispielsweise einer von Pyrophosphorsäure oder vor allem von Orthophosphorsäure abgeleitete, der auch in einer funktionell abgewandelter Form, z.B. als ein Salz, Hydrocarbylester oder Amid, vorliegen kann. Von den erfindungsgemässen Verbindungen der Formel I, worin R für $Ac^3$ steht, sind insbesondere solche hervorzuheben, in welchen $Ac^3$ der Teilformel

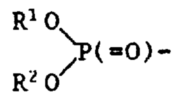

entspricht, worin $R^1$ und $R^2$ die oben angegebenen allgemeinen und besonders hervorgehobenen Bedeutungen haben und vorzugsweise beide gleich sind und Wasserstoff oder in unsubstituiertes $C_1$-$C_7$-Alkyl, insbesondere ein lineares, wie vor allem Methyl oder Ethyl, oder aber ein gegebenenfalls, insbesondere durch $C_1$-$C_4$-Alkyl, $C_1$-$C_4$-Alkoxy, Halogene und/oder Nitro substituiertes Phenyl darstellen.

Besonders bevorzugt sind solche Verbindungen der Formel I, worin R ein Acyl der Teilformel Z-C(=W)- ist, worin W Sauerstoff, ferner Schwefel, ist und Z $C_1$-$C_7$-Alkyl bedeutet, welches auch durch Halogen, Carboxy oder $C_1$-$C_4$-Alkoxy-carbonyl substituiert sein kann.

Besonders bevorzugt sind solche Verbindungen der Formel I, worin R ein Acyl der Teilformel Z-C(=W)- ist, worin W Sauerstoff, ferner Schwefel, ist und Z Phenyl, ferner Pyridyl, Furyl, Thienyl, Imidazolyl, Chinolyl, Isochinolyl, Benzofuranyl oder Benzimidazolyl bedeutet, welches jeweils unsubstituiert oder durch $C_1$-$C_4$-Alkyl, $C_1$-$C_4$-Alkoxy, Halogen, Nitro, Trifluormethyl, Carboxy, $C_1$-$C_4$-Alkoxy-carbonyl, Methylendioxy und/oder Cyano substituiert ist.

Besonders bevorzugt sind solche Verbindungen der Formel I, worin R ein Acyl der Teilformel $R_b^o$ -CO- ist, worin $R_b^o$ $C_1$-$C_7$-Alkyl, insbesondere $C_1$-$C_4$-Alkyl, wie Methyl oder tert-Butyl, bedeutet, welches auch durch Halogen, wie Fluor oder Chlor, Carboxy oder $C_1$-$C_4$-Alkoxy-carbonyl, wie Methoxycarbonyl, substituiert sein kann, wie Trifluor- oder Trichlormethyl, 2-Carboxy- oder 2-Methoxycarbonyl-ethyl.

Besonders bevorzugt sind solche Verbindungen der Formel I, worin R ein Acyl der Teilformel $R_b^o$ -CO- ist, worin $R_b$ Phenyl ist, welches unsubstituiert oder ferner durch $C_1$-$C_4$-Alkyl, $C_1$-$C_4$-Alkoxy, Halogen, wie Fluor oder Chlor, Nitro, Trifluormethyl, Carboxy oder $C_1$-$C_4$-Alkoxy-carbonyl substituiert sein kann.

Besonders bevorzugt sind solche Verbindungen der Formel I, worin R ein Acyl der Teilformel $R^0$-$SO_2$- ist, worin $R^0$ $C_1$-$C_7$-Alkyl, insbesondere $C_1$-$C_4$-Alkyl, bedeutet.

Besonders bevorzugt sind solche Verbindungen der Formel I, worin R ein Acyl der Teilformel $R^0$-$SO_2$- ist, worin $R^0$ Phenyl, ferner Pyridyl, Furyl, Thienyl, Imidazolyl, Chinolyl, Isochinolyl, Benzofuranyl oder Benzimidazolyl bedeutet, welches jeweils unsubstituiert oder durch $C_1$-$C_4$-Alkyl, $C_1$-$C_4$-Alkoxy, Halogen, Nitro, Trifluormethyl, Carboxy, $C_1$-$C_4$-Alkoxy-carbonyl, Methylendioxy und/oder Cyano substituiert ist.

Besonders bevorzugt sind solche Verbindungen der Formel I, worin R ein Acyl der Teilformel $R^0$-$SO_2$- ist, worin $R^0$ Phenyl oder durch $C_1$-$C_4$-Alkyl oder Halogen substituiertes Phenyl oder Isochinolyl, wie 5-Isochinolyl, bedeutet.

Besonders bevorzugt sind solche Verbindungen der Formel I, worin R ein Acyl der Teilformel $R^0$-O-CO- ist, worin $R^0$ $C_1$-$C_7$-Alkyl, insbesondere $C_1$-$C_4$-Alkyl, bedeutet.

Besonders bevorzugt sind solche Verbindungen der Formel I, worin R ein Acyl der Teilformel $R^0$-O-CO- ist, worin $R^0$ Phenyl, ferner Pyridyl, Furyl, Thienyl, Imidazolyl, Chinolyl, Isochinolyl, Benzofuranyl oder Benzimidazolyl bedeutet, welches jeweils unsubstituiert oder durch $C_1$-$C_4$-Alkyl, $C_1$-$C_4$-Alkoxy, Halogen, Nitro, Trifluormethyl, Carboxy, $C_1$-$C_4$-Alkoxy-carbonyl, Methylendioxy und/oder Cyano substituiert ist.

Besonders bevorzugt ist die Verbindung der Formel I, worin R ein Acyl der Teilformel $R^0$-O-CO- ist, worin $R^0$ unsubstituiertes Phenyl ist.

Besonders bevorzugt sind solche Verbindungen der Formel I, worin R ein Acyl der Teilformel

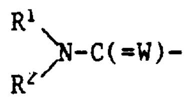

ist, worin W für Schwefel oder insbesondere Sauerstoff steht, $R_1$ Wasserstoff ist und $R_2$ $C_1$-$C_7$-Alkyl, insbesondere $C_1$-$C_4$-Alkyl, $C_3$-$C_7$-Alkenyl oder Phenyl, ferner Pyridyl, Furyl, Thienyl, Imidazolyl, Chinolyl, Isochinolyl, Benzofuranyl oder Benzimidazolyl bedeutet, welches jeweils unsubstituiert oder durch $C_1$-$C_4$-Alkyl, $C_1$-$C_4$-Alkoxy, Halogen, Nitro, Trifluormethyl, Carboxy, $C_1$-$C_4$-Alkoxy-carbonyl, Methylendioxy und/oder Cyano substituiert ist.

Besonders bevorzugt sind solche Verbindungen der Formel I, worin R von einer α-Aminosäure, insbesondere einer in der Natur vorkommenden α-Aminosäure der L-Reihe, abgeleitet ist.

Besonders bevorzugt sind solche Verbindungen der Formel I, worin R von einer α-Aminosäure ausgewählt aus Glycin, Phenylglycin, Alanin, Phenylalanin, Prolin, Leucin, Serin, Valin, Tyrosin, Arginin, Histidin und Asparagin abgeleitet ist.

Besonders bevorzugt sind solche Verbindungen der Formel I, worin R von einer α-Aminosäure ausgewählt aus Glycin, Alanin, Phenylalanin, Serin, Arginin und Histidin abgeleitet ist.

Ihrer Natur nach können die erfindungsgemässen Verbindungen, sofern sie salzbildende Gruppen enthalten auch in Form von Salzen, insbesondere von pharmazeutisch verwendbaren, d.h. physiologisch verträglichen, Salzen, vorliegen. Zur Isolierung oder Reinigung können auch pharmazeutisch ungeeignete Salze verwendet werden. Zur therapeutischen Anwendung gelangen nur pharmazeutisch verwendbare Salze, die bevorzugt sind.

So können z.B. Verbindungen mit freien Säuregruppen, wie beispielsweise einer freien Sulfo-, Phosphoryl- oder Carboxylgruppe, insbesondere einer, die sich im Acylrest Ac befindet, als ein Salz, vorzugsweise als ein physiologisch verträgliches Salz, mit einer salzbildenden basischen Komponente vorliegen. In Betracht kommen in erster Linie Metall- oder Ammoniumsalze, wie Alkalimetall- und Erdalkalimetall-, z.B. Natrium-, Kalium-, Magnesium- oder Calciumsalze, bzw. Ammoniumsalze mit Ammoniak oder geeigneten organischen Aminen, insbesondere tertiären Monoaminen und heterocyclischen Basen z.B. Triethylamin, Tri-(2-hydroxyethyl)-amin, N-Ethylpiperidin, oder N,N′-Dimethylpiperazin. - Falls sich eine solche Säuregruppe in einem Hydrocarbylrest $R^0$ befindet, so kann sie auch mit dem Aminostickstoff der Staurosporin-Grundstruktur, oder auch mit einer anderen gegebenenfalls vorhandenen Aminogruppe, ein inneres Salz bilden.

Erfindungsgemässe Verbindungen basischen Charakters können auch als Additionssalze vorliegen, insbesondere als Säureadditionssalze mit anorganischen und organischen Säuren, aber auch als quaternäre Salze. So können z.B. Verbindungen der Formel I, die im Rest Ac eine basische Gruppe, wie eine Aminogruppe, als Substituent tragen, Säureadditionssalze mit geläufigen Säuren bilden. Besonders hervorzuheben sind Additionssalze der Formel

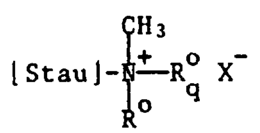

(IA)

worin [Stau] und $R^o$ die eingangs angegebenen Bedeutungen haben, $R_q^o$ Wasserstoff oder ein unsubstituiertes, vorzugsweise lineares $C_1$-$C_4$-Alkyl, wie insbesondere Ethyl oder vor allem Methyl, oder Benzyl ist und $X^-$ Anion einer anorganischen oder organischen Säure oder eines in Rest $R^o$ vorliegenden Carboxyls darstellt, wobei physiologisch verträgliche Salze bevorzugt sind. Unter quaternären Salzen der Formel IA sind solche bevorzugt, worin im Hydrocarbyl $R^o$ das erste Kohlenstoffatom als Methylen vorliegt.

Zur Bildung des Anions $X^-$ sind beispielsweise u.a. die folgenden geläufigen Säuren geeignet: Halogenwasserstoffsäuren, z.B. Chlor-und Bromwasserstoffsäure, Schwefelsäure, Phosphorsäure, Salpetersäure oder Perchlorsäure, bzw. aliphatische, alicyclische, aromatische oder heterocyclische Carbon- oder Sulfonsäuren, wie Ameisen-, Essig-, Propion-, Bernstein-, Glykol-, Milch-, Aepfel-, Wein-, Zitronen-, Fumar-, Malein-, Hydroxymalein-, Oxal-, Brenztrauben-, Phenylessig-, Benzoe-, p-Aminobenzoe-, Anthranil-, p-Hydroxybenzoe-, Salicyl-, p-Aminosalicylsäure, Embonsäure, Methansulfon-, Ethansulfon-, Hydroxyethansulfon-, Ethylendisulfon-, Halogenbenzolsulfon-, Toluolsulfon-, Naphthalensulfonsäuren oder Sulfanilsäure, ferner Methionin, Tryptophan, Lysin oder Arginin, sowie Ascorbinsäure. In quaternären Salzen sind als $X^-$ Anione starker anorganischer Säuren, wie Halogenwasserstoffsäuren, insbesondere Bromide und Jodide, oder organischer Sulfonsäuren, wie Methansulfonate (Mesylate), p-Toluolsulfonate (Tosylate), p-Brombenzolsulfonate (Brosylate) und p-Nitrobenzolsulfonate, bevorzugt.

Besonders bevorzugte Verbindungen der Formel I bzw. IA sind die in den Beispielen beschriebenen.

Die erfindungsgemässen Verbindungen der Formel I und ihre Salze werden durch an sich bekannte allgemeine Verfahren der organischen Chemie hergestellt, insbesondere dadurch, dass man Staurosporin der Formel [Stau] $-NH-CH_3$ (II), worin [Stau] die obgenannte Bedeutung hat, oder ein Säureadditionssalz davon entweder

a) mit einem Reagens der Formel R-Y (III), worin R die obgenannten Bedeutungen hat und Y eine reaktionsfähig aktivierte Hydroxylgruppe oder eine zusätzliche einfache Bindung, derer anderes Ende einen Wasserstoff im Rest R ersetzt, darstellt, oder,

b) zur Herstellung von Verbindungen der Formel I, in welchen R den Rest der Teilformel $H-R^{o}_{a}-$ darstellt, worin $R^{o}_{a}$ einen zweiwertigen, der allgemeinen Struktur von $R^{o}$ entsprechenden Kohlenwasserstoffrest (Hydrocarbylrest) aliphatischen Charakters (d.h. einen solchen, in welchem das funktionalisierte Kohlenstoffatom durch einfache Bindungen mit benachbarten Kohlenstoff- und/oder Wasserstoffatomen verbunden ist) darstellt, mit einem Carbonylreagens der Formel $R^{o}_{a}=0$ (IV), worin $R^{o}_{a}$ die obenstehende Bedeutung hat, und zugleich oder nachfolgend mit einem Reduktionsmittel umsetzt, und

gewünschtenfalls eine erhaltene Verbindung der Formel I in eine andere Verbindung der Formel I umwandelt und/oder eine in freier Form erhaltene Verbindung der Formel I in ein Salz davon überführt und/oder eine als Salz erhaltene Verbindung der Formel I in ihre freie Form oder in ein anderes Salz überführt.

Die erfindungsgemässe Umsetzung von Staurosporin mit einem Reagens des Typ a), d.h. einem der Formel III, erfolgt unter bekannten Verfahrensbedingungen, die in der organischen Chemie allgemein für die Substitution von Aminen gebräuchlich sind, üblicherweise bei Temperaturen zwischen dem Gefrierpunkt und dem Siedepunkt des Reaktionsgemisches, wie im Temperaturbereich von etwa -10 bis etwa +160°, insbesondere von etwa +20 bis etwa +50°, beim atmosphärischen oder erhöhten Druck, in heterogener Phase (wie Suspension) unter Rühren oder Umschütteln, oder vornehmlich in homogener flüssiger Phase, wie in einem Ueberschuss von flüssigem Reagens oder insbesondere in Anwesenheit von Lösungsmitteln, insbesondere organischen Lösungsmitteln, und gegebenenfalls in Gegenwart von säurebindenden anorganischen oder organischen Mitteln. - Geeignete Lösungsmittel sind beispielsweise aprotische organische Lösungsmittel niedriger Polarität, wie aliphatische und aromatische Kohlenwasserstoffe vom Typ Pentan, Hexan, Heptan und Cyclohexan bzw. Benzol, Toluol und Xylole, sowie halogenierte, insbesondere chlorierte, aliphatische Kohlenwasserstoffe, wie Chloroform und Dichlormethan, und insbesondere polare aprotische Lösungsmittel, wie aliphatische und cyclische Ether, z.B. Diethylether, 1,2-Dimethoxyethan und Diisopropylether bzw. Dioxan und Tetrahydrofuran, niederaliphatische Ester und Amide, wie Ethylacetat bzw. Formamid, Acetamid, N,N-Dimethylacetamid und Dimethylformamid, sowie Acetonitril, Dimethylsulfoxid und Hexamethylphosphortriamid; unter gewissen Bedingungen ist als Lösungsmittel auch Wasser oder ein protisches organisches Lösungsmittel, wie ein Niederalkanol, z.B. Methanol, Ethanol, Isopropylalkohol und tert-Butylalkohol, sowie Glykol-oder Diglykolmonoether, z.B. 2-Methoxyethanol, vorteilhaft. In diesem Falle ist es oft vorteilhaft, die Reaktionsgeschwindigkeit durch erhöhten Druck, z.B. durch Arbeiten in geschlossenen Gefässen, zwecks Erhöhung der Siede- und Reaktionstemperatur, zu fördern. Die Lösungsmittel können auch in zweckmässigen Kombinationen, z.B. zur Erhöhung der Löslichkeit von Komponenten, eingesetzt werden. -

Als säurebindende Mittel können im Prinzip beliebige basische Verbindungen zugezogen werden, wie einerseits organische stickstoffhaltige Basen, z.B. tertiäre Amine vom Typ Triethylamin, Ethyldiisopropylamin, N,N-Dimethylanilin, N-Ethylpiperidin oder N,N'-Dimethylpiperazin, oder aromatische heterocyclische Basen vom Typ Pyridin, Collidin, Chinolin oder 4-Dimethylaminopyridin, andererseits basisch reagierende anorganische Verbindungen, insbesondere Alkalimetallhydroxide, -carbonate und -hydrocarbonate, sowie Salze von Carbonsäuren, wie Natrium- oder Kaliumacetat.

Schliesslich können, diese Rolle auch neutral reagierende Stickstoffhaltige Verbindungen übernehmen, die zugleich oft auch vorteilhafte Lösungsmittel darstellen, z.B. Carbonsäureamide, insbesondere niederaliphatische Carbonsäureamide, wie die oben genannten, und cyclische Amide, wie N-Methylpyrrolidon, sowie Amidoderivate der Kohlensäure, wie Urethane und Harnstoff.

Obwohl der Austauschreaktion immer dasselbe Prinzip zugrundeliegt und die Umsetzung nach einem einheitlichen Grundschema erfolgt, ist es für ein optimales Resultat notwendig, bei praktischer Durchführung die Eigenart der Reaktionskomponenten, in erster Linie die des jeweiligen Reaktionsmittels der Formel III, zu berücksichtigen.

Gemäss der vorangehenden Definition kann der Rest R ein Hydrocarbyl $R^0$ der oben angegebenen allgemeinen und hervorgehobenen Bedeutungen sein. In diesem Falle stellt Y in erster Linie eine reaktionsfähige veresterte Hydroxylgruppe (als eine besondere Form der obgenannten reaktionsfähig aktivierter Hydroxylgruppe) dar, d.h. eine solche, die mit einer starken anorganischen Säure, wie einer Halogenwasserstoffsäure, (z.B. Chlor-, Brom- und Jodwasserstoffsäure), einer sauerstoffhaltigen Mineralsäure, wie Phosphorsäure und insbesondere Schwefelsäure, oder einer starken organischen, wie aliphatischen oder aromatischen Sulfonsäure, (z.B. Methan- und Ethan- bzw. Benzol-, p-Toluol-, p-Nitrobenzol- und p-Chlorbenzolsulfonsäure) verestert wird. Falls $R^0$ den aliphatischen Charakter hat, d.h. seine freie Valenz von einem Kohlenstoffatom ausgeht, welches nur durch einfache Bindungen mit benachbarten C- bzw. H-Atomen verbunden ist, so kann man unter den oben beispielsweise genannten veresterten Hydroxylgruppen frei wählen; falls jedoch $R^0$ den aromatischen Charakter hat, d.h. seine freie Valenz von einem Kohlenstoffatom ausgeht, welches ein Bestandteil eines aromatischen carbocyclischen oder heterocyclischen Rings ist, sind Ester von Halogenwasserstoffsäuren, insbesondere Bromide und Jodide, bevorzugt.

Ein Reagens der Formel III, worin Y eine zusätzliche einfache Bindung zu einem Hydrocarbylrest $R^0$ (unter Ersatz eines Wasserstoffs davon) dargestellt, ist beispielsweise ein Alken, insbesondere ein solches, dessen Doppelbindung durch eine strukturelle Besonderheit, wie im 2-Methylpropen, oder Substitution, wie insbesondere im Acrylnitril, zusätzlich aktiviert wird. Inbegriffen in der Definition von Y ist auch eine solche einfache Bindung, derer anderes Ende nicht unmittelbar mit einem C-Atom des Hydrocarbylrests $R^o$, sondern mit einem als Substituent vorkommenden Heteroatom, wie Sauerstoff (d.h. einem von einer Hydroxylgruppe) oder Stickstoff (von einer Aminogruppe), gebunden ist (wobei es einen Wasserstoff dieser Gruppe ersetzt); besonders bevorzugt sind Reagentien der Formel $R^oY$, welche die Gruppierung des $\alpha$-Epoxids (Oxirans) oder $\alpha$-Imins (Aziridins) enthalten und als vorteilhafte Quelle von Resten $R^o$ mit einer 2-Hydroxy- bzw. 2-Amino-alkyl-Gruppierung dienen. Die Umsetzung mit diesen Reagentien erfolgt vorzugsweise in Anwesenheit von Niederalkanolen bei erhöhter Temperatur, z.B. bei etwa +100 bis etwa 150°C, und gegebenenfalls (zwecks Erhöhung der Siedetemperatur des Reaktionsgemisches) unter erhöhtem Druck, oder im basischen Milieu und insbesondere mit einem Ueberschuss an Reagens.

Gemäss der eingangs angegebenen Definition kann der Rest R für ein Acyl Ac stehen und demnach einem Acylierungsmittel der Formel AcY zugrundeliegen, in welchem sowohl Ac wie Y die bereits angegebenen, wie für $Ac^1$, $Ac^2$ und $Ac^3$, allgemeinen und hervorgehobenen Bedeutungen haben. Bevorzugt bedeutet Y Halogen, insbesondere Chlor, Brom und Jod.

In Acylierungsmitteln, die sich vom oben definierten Acylrest $Ac^1$ einer Carbonsäure ableiten, kann Y beispielsweise für eine reaktionsfähig aktivierte Hydroxylgruppe stehen. Eine solche liegt bereits in der freien Carboxylgruppe einer Carbonsäure der Formel $R^o$-COOH vor, wenn sie durch Besonderheiten der Struktur, wie in der Trifluoressigsäure und vor allem der Ameisensäure, eine genügende Reaktivität hat, aber insbesondere dann, wenn sie durch die Einwirkung von Aktivierungsreagentien, z.B. Carbodiimiden, wie insbesondere Dicyclohexylcarbodiimid oder Di-(2-imidazolyl)-carbodiimid und andere analoge Verbindungen, und gegebenenfalls in Anwesenheit von Aktivester-bildenden Hilfsstoffen, wie substituierten Phenolen und insbesondere N-Hydroxyamino-Verbindungen vom Typ 1-Hydroxybenzotriazol, N-Hydroxyphthalimid und N-Hydroxymaleinimid oder -succinimid, vorüber aktiviert wird.

Eine bei Acylresten aller Art, z.B. bei $Ac^1$, $Ac^2$ und $Ac^3$, vorteilhafte aktivierte Hydroxylgruppe ist eine reaktionsfähige, durch starke Säuren veresterte Hydroxylgruppe, wie die oben im Zusammenhang mit dem Hydrocarbyl $R^0$ definierte, welche mit dem Acylrest ein gemischtes Säureanhydrid bildet. Darunter besonders hervorzuheben sind gemischte Anhydride mit Halogenwasserstoffsäuren, insbesondere mit der Bromwasserstoffsäure und vor allem der Chlorwasserstoffsäure, d.h. Säurebromide bzw. Säurechloride, z.B. diejenigen der Formeln Z-C(=W)-Hal, $R^0$-$SO_2$-Hal und

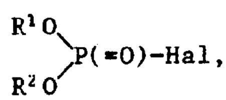

worin Hal Brom und vorzugsweise Chlor bedeutet und Z, W, $R^0$, $R^1$ und $R^2$ die oben genannten Bedeutungen haben, als eine besondere Ausgestaltung ist Phosgen und Thiphosgen zu erwähnen.

Bei Acylresten $Ac^1$ von Carbonsäuren, (einschliesslich Acylreste einer funktionell abgewandelten Kohlensäure) kann die reaktionsfähige veresterte Hydroxylgruppe auch entweder durch den Rest einer anderen Carbonsäure, insbesondere einer stärkeren Carbonsäure, wie der Ameisensäure, Chloressigsäure oder vornehmlich der Trifluoressigsäure, verestert werden und einem gemischten Anhydrid zugrundeliegen, oder aber durch denselben Acylrest verestert werden und ein symmetrisches Carbonsäureanhydrid der Formel $Ac^1$-O-$Ac^1$, insbesondere eines der Formeln $R^0$-CO-O-CO-$R^0$ oder $R^0$-O-CO-O-CO-O-$R^0$ (oder eines Schwefelanalogen davon), bilden.

Bei Acylierungen mit oben geschilderten Acylierungsmitteln arbeitet man vorzugsweise in Anwesenheit eines säurebindenden Mittels, wie eines oben erwähnten, welches man vornehmlich in äquivalenter Menge oder einem kleineren Ueberschuss (der normalerweise 2 Aequivalente nicht übersteigt) einsetzt.

Acylierungsmittel der Formel AcY, in welchen Y eine zusätzliche Bindung zum Rest Ac darstellt, leiten sich insbesondere von Acylresten $Ac^1$ der Carbonsäuren, speziell von jenen, die am Nachbaratom zur Carbonylgruppe (d.h. am benachbarten Kohlenstoff- oder Stickstoffatom) einen Wasserstoff tragen; sie gehören der Kategorie der Ketene bzw.

Isocyanate und entsprechen den Formeln $R_a^o$=C=0 bw. $R^1$-N=C=O, worin $R_a^o$ die oben definierte Bedeutung eines zweiwertigen, dem Rest $R^0$ entsprechenden Hydrocarbyls aliphatischen Charakters hat und $R^1$ die oben angegebenen allgemeinen und besonders hevorgehobenen Bedeutungen mit Ausnahme von Wasserstoff hat. Zu erwähnen ist auch ein analoges schwefelhaltiges Acylierungsmittel-Isothiocyanat der Formel $R^1$-N=C=S, worin $R^1$ die obgenannten allgemeinen und hervorgehobenen Bedeutungen ausser Wasserstoff hat. Die Acylierung mit derartigen Mitteln kann, ihrer Natur nach, auch ohne säurebindende Mittel erfolgen, ein strikter Ausschluss von Feuchtigkeit und/oder protischen Lösungsmitteln ist zu empfehlen.

Verfahrensvariante b) ist unter dem Begriff "reduktive Alkylierung" allgemein bekannt und häufig angewendet. Zu Carbonylreagentien der oben definierten Formel $R_a^o$=O [IV] gehören in erster Linie Aldehyde, aber auch Ketone, einschliesslich cyclischer Ketone, in welchen die Carbonylgruppe ein Glied eines alicyclischen Rings ist, vorzugsweise leiten sich diese Reagentien von unsubstituierten Hydrocarbylresten, oder mindestens tragen sie Substituenten, die gegen Reduktion beständig sind. - Als Reduktionsmittel kommen komplexe Metallhydride in Betracht, wie Alkalimetallaluminium- und insbesondere -borohydride, z.B. Lithiumaluminiumhydrid, Kaliumborohydrid, Lithiumborohydrid und vor allem Natriumborohydrid, sowie ihre Derivate, worin ein oder mehrere Wasserstoffatome durch Alkoxyreste oder Cyano ersetzt sind, z.B. Methoxynatriumborohydrid, Tri-(tert-butoxy)-lithiumborohydrid oder Di-(2-Methoxyethoxy)-dinatriumlithiumhydrid bzw. Natriumcyanoborohydrd, sowie auch Diboran. Diese Reduktionsmittel werden vornehmlich erst in zweiter Phase der Alkylierung, d.h. nach der primären Zugabe des Carbonylreagens, zugegeben. - Ein anderes häufig gebrauchtes Reduktionsmittel ist elementarer Wasserstoff, der unter den üblichen Bedingungen der katalytischen Hydrierung, bei Temperaturen von etwa +20 bis etwa +100° und erforderlichenfalls Ueberdruck bis zu etwa 150 Atm. gleichzeitig mit der Carbonylkomponente eingesetzt wird. Als Katalysator wird üblicherweise Raney-Nickel, aber auch Palladium oder Platin, vorzugsweise auf einem inerten Träger, wie Calciumcarbonat, Bariumsulfat oder Aluminiumoxid, verwendet. - Eine andere Variante der reduktiven Alkylierung verwendet als Reduktionsmittel die Ameisensäure, sie ist insbesondere für die Methylierung mit Formaldehyd geeignet.

Erfindungsgemäss kann, wenn erwünscht, eine erhaltene Verbindung der Formel I in eine andere Verbindung der Formel I übergeführt werden; dementsprechend wird insbesondere eine im Rest R vorliegende funktionelle Gruppe in eine andere umgewandelt, z.B. eine funktionell abgewandelte, insbesondere eine geschützte, Hydroxyl-, Caboxyl- oder Aminogruppe in ihre freie Form übergeführt, oder ein reaktionsfähigen Chloratom (wie jenes im Chloroformylrest) gegen den Rest $R^0$-O- oder $R^1$-N(-$R^2$)- ausgetauscht. - Die Freisetzung einer funktionell abgewandelten Gruppe ist beispielsweise die Umwandlung einer veresterten Carboxylgruppe in die freie Carboxylgruppe, welche im allgemeinen durch konventionelle Hydrolyse, vor allem unter Einwirkung von Basen (wie vornehmlich von Alkalimetall-hydroxiden, -cabonaten der -hydrocarbonaten), oder aber, bei geeigneten Estern, wie solchen von tertiären Alkoholen (z.B. tert-Butylalkohol), durch Acidolyse, z.B. mittels Fluorwasserstoff oder Trifluoressigsäure, durchgeführt werden kann. Ester mit Benzylalkoholen können auch durch konventionelle Hydrogenolyse abgespalten werden. Da Veresterung zu den gewöhnlichsten Methoden zum Schutz von Carboxylgruppen zählt, stellt die obige Umwandlung zugleich eine wirksame Methode zum Entfernen von Carboxyl-Schutzgruppen dar.

Die zu vorübergehendem Schutz von Hydroxylgruppen anzuwendenden Gruppen und Abspaltungsmethoden sind auch allgemein bekannt, z.B. aus der Synthese von Peptiden. Insbesondere schützt man Hydroxylgruppen in der Form von Estern mit Carbonsäuren, wie mit Niederalkansäuren oder mit Monoestern der Carbonsäure (z.B. Formiate oder Acetate einerseits oder tert-Butoxy- oder Benzyloxy-carbonate andererseits), oder aber in der Form von Ethern, wie insbesondere solchen von tertiären Alkoholen (z.B. tert-Butylalkohol), oder auch in der Form von Acetalen (z.B. insbesondere als 2-Tetrahydropyranylether). Die ersteren Schutzgruppen werden üblicherweise analog wie veresterte Carbonylgruppen abgespalten; beide letzteren werden vornehmlich durch Acidolyse entfernt.

Die zum vorübergehenden Schutz von primären und sekundären Aminogruppen verwendbaren Schutzgruppen entsprechen denjenigen, die bei der Synthese von Peptiden eingehend untersucht wurden und die breiteste Anwendung finden, vorzugsweise werden die eingangs angegebenen Amino-Schutzgruppen angewendet - ihre Abspaltung, welche sich generell nach ihrer spezifischen Natur richtet, erfolgt unter allgemein bekannten Bedingungen der Hydrolyse (insbesondere der basischen Hydrolyse), Acidolyse bzw. Hydrogenolyse.

Vornehmlich werden die allgemeinen Bedingungen der konventionellen Abspaltung der funktionell abgewandelten Gruppen so gewählt, dass weder die Bindung zwischen dem Rest R und der Methylaminogruppe des Staurosporins noch dessen Grundstruktur beeinträchtigt wird; da diese Strukturmerkmale sich im allgemeinen durch gute Stabilität auszeichnen, kann man geläufige Reaktionsbedingungen ohne besondere Vorsichtsmassnahmen anwenden.

Eine gewünschtenfalls durchführbare erfindungsgemässe nachträgliche Umwandlung eines reaktionsfähigen Chloratoms findet insbesondere Ueberführung der Chloroformylgruppe (Cl-CO-) in die Hydrocarbyloxycarbonylgruppe ($R^0$-O-CO-) oder Aminocarbonyl-(Carbamoyl-)-gruppe [$R^1$-N(-$R^2$)-CO-] statt. Diese Umwandlung erfolgt unter an sich bekannten Bedingungen, indem man N-Chloroformylstaurosporin mit einem Alkohol der Formel $R^0$-OH bzw. einem Amin

(einschliesslich Ammoniak) der Formel $R^1$-NH-$R^2$, vorzugsweise in Anwesenheit eines säurebindenden Mittels, wie einer organischen Base (z.B. eines der oben genannten tertiären Amine) umsetzt. Die allgemeinen Reaktionsbedingungen sind analog denjenigen, die oben für die Umsetzungen mit Reagentien mit reaktionsfähig veresterter Hydroxylgruppe (insbesondere für Säurechloride) eingehend beschrieben wurden.

Die gewünschtenfalls durchzuführende Salzbildung und Freisetzen der Grundformen der Verbindungen der Formel I aus ihren Salzen erfolgt in an sich allgemein bekannter, konventioneller Weise. - So werden Carboxyltragende Acylderivate der Formel I in entsprechende Salze mit Basen, vor allem Alkalimetallsalze, durch Behandeln mit einer entsprechenden Base, insbesondere einer alkalisch reagierenden Verbindung, wie Hydroxid, Carbonat oder Bicarbonat, übergeführt; die Salze können in freie Carboxylverbindungen durch Ansäurern, z.B. mit anorganischen Säuren, wie insbesondere Halogenwasserstoffsäuren, umgewandelt werden. - Basisch reagierende Endstoffe, z.B. tertiäre und quaternäre Amine der Formeln I bzw. IA können in ihre Salze mit Säuren z.B. durch Behandeln mit einer zur Salzbildung geeigneten Säure, wie einer der oben genannten, umgewandelt werden; umgekehrt wird durch Behandeln mit basisch reagierenden Mitteln, wie mit anorganischen Hydroxiden, Carbonaten und Bicarbonaten, oder organischen Basen und Ionenaustauschern eine solche basische Grundform eines tertiären Amins der Formel I freigesetzt.

Geeignete Verbindungen der vorliegenden Erfindung können auch innere Salze, z.B. durch übliches acid-basisches Titrieren zum Neutralpunkt bzw. zum isoelektrischen Punkt, oder, z.B. durch Behandeln mit einem dem Rest $R^o_q$ entsprechenden Quaternisierungsmittel, wie einem reaktions-fähigen Ester einer entsprechenden Hydroxyverbindung mit einer starken Säure, wie einer Halogenwasserstoffsäure, Schwefelsäure oder einer starken organischen Sulfonsäure, quaternäre Ammoniumsalze der Formel IA bilden.

Diese oder andere Salze der neuen Verbindungen, wie z.B. die Pikrate, können auch zur Reinigung der erhaltenen Verbindungen dienen, indem man die freien Verbindungen in Salze überführt, diese abtrennt und aus den Salzen wiederum die freien Verbindungen gewinnt. Infolge der engen Beziehung zwischen den Verbindungen in freier Form und in Form ihrer Salze sind im vorausgegangenen und nachfolgend unter den freien Verbindungen sinn- und zweckgemäss gegebenenfalls auch die entsprechenden Salze (einschliesslich quaternärer Salze) zu verstehen.

Bestimmte Carbonylfunktionen können beispielsweise mit Hilfe geeigneter Reagentien, die den Austausch von O gegen S bewirken, in die entsprechende Thioform übergeführt werden. So kann man z.B. durch Umsetzung mit dem Lawesson-Reagens [2,4-Bis-(4-methoxyphenyl)-2,4-dithioxo-1,3,2,4-dithiadiphosphetan] in Verbindungen der Formel I und ihren Salzen, die als Carbonylfunktion z.B. eine Carboxamid-, Keton- und Lactongruppierung aufweisen, das O-Atom gegen das S-Atom austauschen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates, z.B. Salzes, verwendet oder unter den Reaktionsbedingungen bildet.

Bei den Verfahren der vorliegenden Erfindung werden bekannte oder nach bekannten Methoden erhältliche Ausgangsstoffe verwendet, vorzugsweise solche, welche zu den eingangs als besonders wertvoll geschilderten Verbindungen führen.

Im Hinblick auf die oben beschriebenen pharmakologischen Eigenschaften der neuen Verbindungen umfasst die vorliegende Erfindung auch die Verwendung der erfindungsgemässen Wirkstoffe allein, gegebenenfalls zusammen mit Hilfsstoffen, oder in Kombination mit anderen Wirkstoffen, z.B. Antibiotika oder Chemotherapeutika, als Mittel zur Behandlung von Krankheiten, bei denen, wie oben beschrieben worden ist, Zellenwachstum von Bedeutung ist, und zwar sowohl prophylaktisch, wie auch kurativ. Bei der Verwendung als Heilmittel werden die erfindungsgemässen Wirkstoffe in prophylaktisch bzw. kurativ wirksamen Mengen, vorzugsweise in Form von pharmazeutischen Zusammensetzungen zusammen mit konventionellen pharmazeutischen Trägermaterialien oder Hilfsstoffen verabreicht. Dabei werden z.B. an Warmblüter mit einem Körpergewicht von etwa 70 kg je nach Spezies, Körpergewicht, Alter und individuellem Zustand, sowie je nach Applikationsweise und insbesondere auch je nach den jeweiligen Krankheitsbild, tägliche Dosen von etwa 1 bis 1000 mg, die in akuten Fällen noch überschritten werden dürfen, verabreicht. Sinngemäss umfasst die Erfindung auch die entsprechende Methode zur medizinischen Behandlung.

Die Erfindung betrifft im weiteren pharmazeutische Zusammensetzungen, welche die Verbindungen der vorliegenden Erfindung als Wirkstoffe enthalten, sowie Verfahren zur Herstellung dieser Zusammensetzungen.

Bei den erfindungsgemässen pharmazeutischen Zusammensetzungen handelt es sich z.B. um solche zur enteralen, wie peroralen oder rektalen, sowie zur parenteralen Verabreichung an Warmblüter. Entsprechende Dosiseinheitsformen, insbesondere zur peroralen Verabreichung, z.B. Dragées, Tabletten oder Kapseln, enthalten vorzugsweise von etwa 5 bis 500 mg, insbesondere von etwa 10 bis 100 mg, des Wirkstoffs zusammen mit pharmazeutisch verwendbaren Träger- oder Hilfsstoffen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister (unter Verwendung z.B. von Mais-, Weizen-, Reis- oder Kartoffelstärke), Gelatine, Traganth,

Methylcellulose und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykole und/oder Titandioxid enthalten, oder Lacklösungen in geeigneten organischen Lösungsmitteln oder Lösungsmittelgemischen, oder, zur Herstellung Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmzeutische Präparate sind Steckkapseln aus Gelatine, sowie weiche geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Lactose, Bindemitteln, wie Stärken und/oder Gleitmitteln, wie Talk oder Magnesiumstearat, und gegebenenfalls von Stabilisatoren enthalten. In weichen Kapseln ist der Wikstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffes mit einer Grundmasse enthalten; als Grundmassenstoffe kommen z.B. flüssige Triglyceride, Polyethylenglykole oder Paraffinkohlenwasserstoffe in Frage.

Zur parenteralen Verabreichung eignen sich im erster Linie wässrige Lösungen einer in Wasser löslichen Form des Wirkstoffes, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatore enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Die pharmazeutischen Zusammensetzungen der vorliegenden Erfindung können in an sich bekannter Weise, z.B. mittels konventioneller Misch-, Granulier-, Dragier-, Lösungs- oder Lyophilisierungsverfahren, hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Die nachfolgenden Beispiele illustrieren die oben beschriebene Erfindung, schränken jedoch deren Umfang in keiner Weise ein. Temperaturen werden in Celsiusgraden angegeben.

Die Nomenklatur der Produkte wird von der vollständigen Struktur von Staurosporin ([Stau]-NH-$CH_3$,

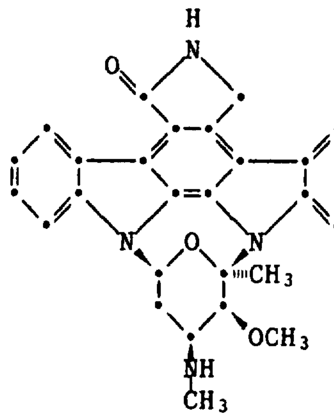

II)

abgeleitet, wobei der mit N- bezeichnete Substituent sich am Stickstoff der Methylaminogruppe befindet.

Beispiel 1: N-Methoxycarbonylmethyl-staurosporin

Eine Mischung von 233 mg (0.5 mMol) Staurosporin, 0,1 ml (0,59 mMol) N,N-Diisopropyl-ethylamin und 2 ml Dimethylformamid wird bei Raumtemperatur mit 0,056 ml (0,6 mMol) Bromessigsäure-methylester versetzt. Das Reaktionsgemisch wird im verschlossenen Kolben 48 Stunden bei Raumtemperatur gerührt; durch Zugabe von 1 ml Wasser wird das Produkt ausgefällt und anschliessend aus Methanol umkristallisiert. Smp. ~210° (Zersetzung, ab 170° Braun-

färbung).

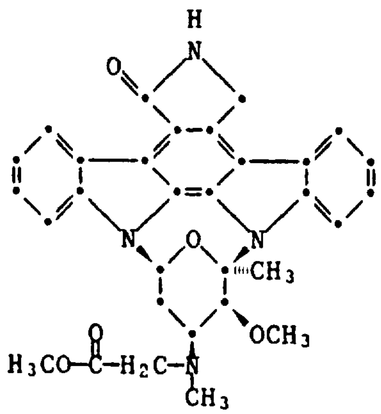

Beispiel 2: N-Carboxymethyl-staurosporin

269 mg (0,5 mMol) des N-Methoxycarbonylmethyl-staurosporins (Beispiel 1) wird in 15 ml Methanol und 0,3 ml 2N Natronlauge während 18 Stunden unter Rückfluss gekocht. Nach dem Abkühlen auf Raumtemperatur wird mit 0,1 ml Essigsäure neutralisiert und das Produkt durch Zugabe von 15 ml Wasser gefällt. Smp. >230° (Zersetzung, ab ca. 220° Braunfärbung).

Beispiel 3: N-(1-Methoxycarbonylethyl)-staurosporin

Eine Mischung von 233 mg (0,5 mMol) Staurosporin, 0,12 ml (0,71 mMol) N,N-Diisopropyl-ethylamin und 2 ml Dimethylformamid wird bei Raumtemperatur mit 0,085 ml (0,75 mMol) α-Brompropionsäure-methylester versetzt. Das Reaktionsgemisch wird im verschlossenen Kolben 20 Stunden bei Raumtemperatur gerührt. Nach Zugabe von weiteren 0,044 ml (0,038 mMol) α-Brompropionsäure-methylester wird noch 20 Stunden auf 80° erwärmt. Nach dem Abkühlen auf Raumtemperatur wird das Produkt durch Zugabe von 2 ml Wasser gefällt. Das Rohprodukt wird durch Chromatographie an Kieselgel gereinigt (Eluiermittel: Methylenchlorid/Ethanol 9:1). Smp. ~150° (Zersetzung).

Beispiel 4: N-Benzyl-staurosporin

Eine Mischung von 116,5 mg (0,25 mMol) Staurosporin, 0,06 ml (0,35 mMol) N,N-Diisopropyl-ethylamin und 1 ml Dimethylformamid wird bei Raumtemperatur mit 0,048 ml (0,38 mMol) Benzylbromid versetzt und das Reaktionsgemisch im verschlossenen Kolben 6 Stunden bei Raumtemperatur gerührt. Das Produkt wird durch Zugabe von 1 ml Wasser gefällt, abfiltriert und aus Methanol umkristallisiert. Smp. ~170° (Zersetzung).

Beispiel 5: N-Allyl-staurosporin

Eine Mischung von 116,5 mg (0,25 mMol) Staurosporin, 0,06 ml (0,35 mMol) N,N-Diisopropyl-ethylamin und 1 ml Dimethylformamid wird bei Raumtemperatur mit 0,032 ml (0,38 mMol) Allylbromid versetzt und das Reaktionsgemisch im verschlossenen Kolben 6 Stunden bei Raumtemperatur gerührt. Das Produkt wird durch Zugabe von 1 ml Wasser gefällt und abfiltriert. Die Reinigung erfolgt durch Chromatographie an Kieselgel mit Methylenchlorid/Ethanol 9:1 als Eluiermittel. Smp. 160° (Zersetzung).

Beispiel 6: N,N-Dimethyl-stauroporiniumjodid (N-Methyl-staurosporinjodmethylat)

Eine Mischung von 233 mg (0,5 mMol) Staurosporin, 0,12 ml (0,71 Mol) N,N-Diisopropyl-ethylamin und 2 ml Dimethylformamid wird bei Raumtemperatur mit 0,046 ml (0,75 mMol) Methyljodid versetzt und das Reaktionsgemisch im verschlossenen Kolben bei Raumtemperatur gerührt. Nach ca. einer Stunde bildet sich ein Niederschlag. Nach Zugabe von weiteren 0,023 ml (0,038 mMol) Methyljodid und 0,06 ml (0,038 mMol) N,N-Diisopropyl-ethylamin wird noch 4 Stunden bei Raumtemperatur gerührt und nach Zufügen von 2 ml Wasser filtriert; das feste Rohprodukt wird in warmem Methanol aufgeschlämmt und nach dem Abkühlen erneut filtriert und getrocknet. Smp. 260° (Zersetzung).

Beispiel 7: N-Ethyl-staurosporin

Eine Mischung von 116,5 mg (0,25 mMol) Staurosporin, 0,06 ml (0,35 mMol) N,N-Diisopropyl-ethylamin und 2 ml Dimethylformamid wird bei Raumtemperatur mit 0,029 ml (0,38 mMol) Ethyljodid versetzt und im verschlossenen Kolben 24 Stunden bei Raumtemperatur gerührt. Das Produkt wird durch Zugabe von 2 ml Wasser gefällt und abfiltriert. Smp. 170° (Zersetzung).

Beispiel 8: N,N-Ethyl-methyl-staurosporin-iumjodid (N-Ethylstaurosporinjodmethylat)

Eine Mischung von 115 mg (0,2 mMol) N-Ethyl-staurosporin (Beispiel 7) und 2 ml Dimethylformamid wird bei Raumtemperatur mit 0,018 ml (0,3 mMol) Methyljodid versetzt. Nach 16 Stunden bei Raumtemperatur und 16 Stunden bei 50° wird weiteres 0,018 ml (0,3 mMol) Methyljodid zugegeben und 6 Stunden bei 80° gerührt. Das Rohprodukt wird durch Fällung mit 2 ml Wasser erhalten und aus Dimethylformamid/Chloroform umkristallisiert. Smp. 265° (Zersetzung).

Beispiel 9: N-(2-Hydroxyhexyl)-staurosporin

Eine Suspension von 116,5 mg (0,25 mMol) Staurosporin und 0,054 ml (0,45 mMol) 1-Hexenoxid in 3,5 ml absolutem Ethanol wird 36 Stunden im Bombenrohr auf 110° erhitzt. Das abgekühlte Reaktionsgemisch wird mit Wasser verdünnt und mit Methylenchlorid extrahiert. Die organische Phase wird über Magnesiumsulfat getrocknet, eingedampft und an Kieselgel chromatographiert (Eluiermittel Methylenchlorid/Ethanol 9:1); Umkristallisation aus Ether/Petrolether ergibt das Produkt vom Smp. 110° (Zersetzung).

Beispiel 10: N-(2-Hydroxytetradecyl)-staurosporin

Eine Suspension von 116,5 mg (0,25 mMol) Staurosporin und 0,075 ml (0,30 mMol) 1-Tetradecenoxid in 3,5 ml absolutem Ethanol wird 68 Stunden im Bombenrohr auf 110° erhitzt. Das abgekühlte Reaktionsgemisch wird mit Wasser verdünnt und mit Methylenchlorid extrahiert. Die organische Phase wird über Magnesiumsulfat getrocknet, eingedampft und an Kieselgel chromatographiert (Eluiermittel Methylenchlorid/Ethanol 9:1). Umkristallisation aus Ether/Petrolether ergibt das Produkt vom Smp. 120° (Zersetzung).

Beispiel 11: N-(2-Hydroxydecyl)-staurosporin

Eine Suspension von 116,5 mg (0,25 mMol) Staurosporin und 0,055 ml (0,30 mMol) 1-Decenoxid in 3,5 ml absolutem Ethanol wird 43 Stunden im Bombenrohr auf 110° erhitzt. Das abgekühlte Reaktionsgemisch wird mit Wasser verdünnt und mit Methylenchlorid extrahiert. Die organische Phase wird über Magnesiumsulfat getrocknet, eingedampft und an Kieselgel chromatographiert (Eluiermittel Methylenchlorid/Ethanol 9:1). Umkristallisation aus Ether/Petrolether ergibt das Produkt vom Smp. 140°.

Beispiel 12: N-(2-Cyanoethyl)-staurosporin

Eine Suspension von 116,5 mg (0,25 mMol) Staurosporin in 2,5 ml (38 mMol) Acrylonitril wird im Bombenrohr während 70 Stunden auf 140° erhitzt. Nach dem Abkühlen wird das Reaktionsgemisch eingedampft und an Kieselgel chromatographiert (Eluiermittel Methylenchlorid/Ethanol 9:1). Umkristallisation aus Chloroform/Methanol ergibt das Produkt vom Smp. ~210°.

Beispiel 13: N-Acetyl-staurosporin

Eine Lösung von 116,5 mg (0,25 mMol) Staurosporin und 0,065 ml (0,38 mMol) N,N-Diisopropyl-ethylamin in 2 ml Chloroform wird bei Raumtemperatur mit 0,03 ml (0,3 mMol) Essigsäureanhydrid versetzt und 2 Stunden im verschlossenen Kolben gerührt. Das Reaktionsgemisch wird mit Chloroform verdünnt, mit Natriumbicarbonatlösung gewaschen, über Magnesiumsulfat getrocknet und eingedampft. Das Produkt wird aus Chloroform/Methanol umkristallisiert; Smp. 240°.

Beispiel 14: N-(3-Carboxypropionyl)-staurosporin

Eine Lösung von 116,5 mg (0,25 mMol) Staurosporin und 0,065 ml (0,38 mMol) N,N-Diisopropyl-ethylamin in 2 ml Chloroform wird bei Raumtemperatur mit 40 mg (0,4 mMol) Bersteinsäureanhydrid versetzt und 28 Stunden im verschlossenen Kolben gerührt. Das Reaktionsgemisch wird mit Chloroform verdünnt, mit 0,1N Salzsäurelösung gewaschen, über Magnesiumsulfat getrocknet und eingedampft. Das Rohprodukt wird an Kieselgel chromatographiert (Eluiermittel Methylenchlorid/Ethanol 9:1); Smp. 140°.

Beispiel 15: N-(5-Isochinolinsulfonyl)-staurosporin

Eine Lösung von 116,5 mg (0,25 mMol) Staurosporin und 0,118 ml (0,69 mMol) N,N-Diisopropyl-ethylamin in 2 ml Chloroform wird bei Raumtemperatur mit 105 mg (0,4 mMol) 5-Isochinolinsulfonylchlorid versetzt und 29 Stunden im verschlossenen Kolben gerührt. Das Reaktionsgemisch wird mit Chloroform verdünnt, mit Natriumbicarbonatlösung gewaschen, über Magnesiumsulfat getrocknet und eingedampft. Das Produkt wird aus Chloroform/Methanol umkristallisiert. Smp. 240° (Zersetzung).

Beispiel 16: N-Methylsulfonyl-staurosporin

Eine Lösung von 116,5 mg (0,25 mMol) Staurosporin und 0,065 ml (0,38 mMol) N,N-Diisopropyl-ethylamin in 2 ml Chloroform wird bei Raumtemperatur mit 0,023 ml (0,38 mMol) Methansulfochlorid versetzt und 24 Stunden im verschlossenen Kolben gerührt. Das Reaktionsgemisch wird mit Chloroform verdünnt, mit Natriumbicarbonatlösung, 1N Salzsäure und gesättigter Natriumchloridlösung gewaschen, über Magnesiumsulfat getrocknet und eingedampft. Das Rohprodukt wird an Kieselgel chromatographiert (Eluiermittel Methylenchlorid/ Ethanol 9:1) und aus Chloroform/Methanol umkristallisiert; Smp. 230°.

Beispiel 17: N-(p-Tosyl)-staurosporin

Eine Lösung von 116,5 mg (0,25 mMol) Staurosporin und 0,065 ml (0,38 mMol) N,N-Diisopropyl-ethylamin in 2 ml Chloroform wird bei Raumtemperatur mit 57 mg (0,3 mMol) p-Toluolsulfochlorid versetzt und 68 Stunden in verschlossenen Kolben gerührt. Das Reaktionsgemisch wird mit Chloroform verdünnt, mit Natriumbicarbonatlösung gewaschen, über Magnesiumsulfat getrocknet und eingedampft. Das Rohprodukt wird an Kieselgel chromatographiert (Eluiermittel Methylenchlorid/Ethanol 9:1) und aus Chloroform/Methanol umkristallisiert; Smp. 245°.

Beispiel 18: N-Benzoyl-staurosporin

Eine Lösung von 116,5 mg (0,25 mMol) Staurosporin und 0,065 ml (0,38 mMol) N,N-Diisopropyl-ethylamin in 2 ml Chloroform wird bei Raumtemperatur mit 0,035 ml (0,3 mMol) Benzoylchlorid versetzt und 10 Minuten gerührt. Das Reaktionsgemisch wird mit Chloroform verdünnt, mit Natriumbicarbonatlösung gewaschen, über Magnesiumsulfat getrocknet und eingedampft. Das Rohprodukt wird an Kieselgel chromatographiert (Eluiermittel Methylenchlorid/Ethanol 30:1); Smp. 235-247° unter Braunfärbung.

Beispiel 19: N-Trifluoracetyl-staurosporin

Eine Lösung von 233 mg (0,5 mMol) Staurosporin und 0,13 ml (0,6 mMol) N,N-Diisopropyl-ethylamin in 2 ml Chloroform wird bei Raumtemperatur mit 0,5 ml (3,57 mMol) Trifluoressigsäureanhydrid versetzt und 15 Minuten gerührt. Das Reaktionsgemisch wird mit Chloroform verdünnt, mit Natriumbicarbonatlösung gewaschen, über Magnesiumsulfat getrocket und eingedampft. Das Rohprodukt wird an Kieselgel chromatographiert (Eluiermittel Methylenchlorid/ Ethanol 20:1); Smp. >220°.

Beispiel 20: N-Phenoxycarbonyl-staurosporin

Eine Lösung von 116,5 mg (0,25 mMol) Staurosporin und 0,065 ml (0,38 mMol) N,N-Diisopropyl-ethylamin in 2 ml Chloroform wird bei Raumtemperatur mit 0,035 ml (0,28 mMol) Chlorameisensäure-phenylester versetzt und 30 Minuten im verschlossenen Kolben gerührt. Das Reaktionsgemisch wird mit Chloroform verdünnt, mit Natriumcarbonatlösung gewaschen, über Magnesiumsulfat getrocknet und eingedampft. Der Rückstand wird mit heissem Methanol verrieben und nach dem Abkühlen abfiltriert und getrocknet. Smp. >210° (Zersetzung).

Beispiel 21: N-Methoxycarbonyl-staurosporin

Eine Lösung von 116,5 mg (0,25 mMol) Staurosporin und 0,065 ml (0,38 mMol) N,N-Diisopropyl-ethylamin in 2 ml Chloroform wird bei Raumtemperatur mit 0,025 ml (0,32 mMol) Chlorameisensäure-methylester versetzt und 1 Stunde im verschlossenen Kolben gerührt. Das Reaktionsgemisch wird mit Chloroform verdünnt, mit Natriumbicarbonatlösung gewaschen, über Magnesiumsulfat getrocknet und eingedampft. Das Rohprodukt wird aus Methanol umkristallisiert;

Smp. >220° (Zersetzung).

Beispiel 22: N-Allylaminothiocarbonyl-staurosporin (N-Allylthiocarbamoyl-staurosporin)

Eine Lösung von 116,5 mg (0,25 mMol) Staurosporin in 2,5 ml Chloroform wird mit 0,029 ml (0,3 mMol) Allylisothiocyanat versetzt und 12 Stunden im verschlossenen Kolben bei Raumtemperatur gerührt. Das Reaktionsgemisch wird eingedampft und das Rohprodukt aus Chloroform/Methanol umkristallisiert; Smp. 220°.

Beispiel 23: N-Methylaminothiocarbonyl-staurosporin (N-Methylthiocarbamoyl-staurosporin)

Eine Lösung von 116,5 mg (0,25 mMol) Staurosporin in 2,5 ml Chloroform wird mit 0,022 mg (0,3 mMol) Methylisothiocyanat versetzt und 12 Stunden im verschlossenen Kolben bei Raumtemperatur gerührt. Das Reaktionsgemisch wird eingedampft und das Rohprodukt aus Chloroform/Methanol umkristallisiert; Smp. 235-38°.

Beispiel 24: N-Phenylcarbamoyl-staurosporin

Eine Lösung von 116,5 mg (0,25 mMol) Staurosporin in 2,5 ml Chloroform wird mit 0,033 ml (0,3 mMol) Phenylisocyanat versetzt und 15 Minuten bei Raumtemperatur gerührt. Das Reaktionsgemisch wird eingedampft und das Rohprodukt aus Chloroform/Methanol umkristallisiert; Smp. 225-229° (Braunfärbung).

Beispiel 25: N-Trichloroacetyl-staurosporin

Eine Lösung von 116,5 mg (0,25 mMol) Staurosporin und 0,1 ml (0,58 mMol) N,N-Diisopropyl-ethylamin in 1 ml Chloroform wird bei Raumtemperatur mit 0,04 ml (0,35 mMol) Trichloracetylchlorid versetzt und eine Stunde gerührt. Das Reaktionsgemisch wird mit Methylenchlorid verdünnt und mit Natriumbicarbonatlösung gewaschen, über Magnesiumsulfat getrocknet und eingedampft.
Das Rohprodukt wird an Kieselgel chromatographiert (Eluiermittel Ethylacetat); IR: 1682 (stark); FAB-MS: 611.

Beispiel 26: N-(3-Chlorobenzoyl)-staurosporin

Eine Lösung von 116,5 mg (0,25 mMol) Staurosporin und 0,065 ml (0,38 mMol) N,N-Diisopropyl-ethylamin in 2 ml Chloroform wird bei Raumtemperatur mit 0,038 ml (0,38 mMol) 3-Chlorbenzoylchlorid versetzt und eine Stunde gerührt. Das Reaktionsgemisch wird mit Chloroform verdünnt und mit Natriumbicarbonatlösung, 1 normaler Salzsäure und gesättigter Natriumchloridlösung gewaschen, über Magnesiumsulfat getrocknet und eingedampft.
Das Rohprodukt wird an Kieselgel chromatographiert (Eluiermittel Methylenchlorid-Ethanol 95:5); Smp. 240 Grad (Zersetzung).

Beispiel 27: N-(2-Chlorobenzoyl)-staurosporin

Eine Lösung von 116,5 mg (0,25 mMol) Staurosporin und 0,065 ml (0,38 mMol) N,N-Diisopropyl-ethylamin in 2 ml Chloroform wird bei Raumtemperatur mit 0,038 ml (0,38 mMol) 2-Chlorbenzoylchlorid versetzt und eine Stunde gerührt. Das Reaktionsgemisch wird mit Chloroform verdünnt und mit Natriumbicarbonatlösung, 1 normaler Salzsäure und gesättigter Natriumchloridlösung gewaschen, über Magnesiumsulfat getrocknet und eingedampft.
Das Rohprodukt wird an Kieselgel chromatographiert (Eluiermittel Methylenchlorid-Ethanol 95:5); Smp. 255 Grad (Zersetzung).

Beispiel 28: N-(3-Nitrobenzoyl)-staurosporin

Eine Lösung von 116,5 mg (0,25 mMol) Staurosporin und 0,065 ml (0,38 mMol) N,N-Diisopropyl-ethylamin in 2 ml Chloroform wird bei Raumtemperatur mit 55,5 mg (0,30 mMol) 3-Nitrobenzoylchlorid versetzt und eine Stunde gerührt. Das Reaktionsgemisch wird mit Chloroform verdünnt und mit Natriumbicarbonatlösung, 1 normaler Salzsäure und gesättigter Natriumchloridlösung gewaschen, über Magnesiumsulfat getrocknet und eingedampft.
Das Rohprodukt wird an Kieselgel chromatographiert (Eluiermittel Methylenchlorid-Ethanol 95:5); Smp. 230 Grad.

Beispiel 29: N-(4-Methoxybenzoyl)-staurosporin

Eine Lösung von 116,5 mg (0,25 mMol) Staurosporin und 0,065 ml (0,38 mMol) N,N-Diisopropyl-ethylamin in 2 ml Chloroform wird bei Raumtemperatur mit 0,083 ml (0,38 mMol) einer 58 prozentigen 4-Methoxybenzoylchloridlösung

in Toluol versetzt und eine Stunde gerührt. Das Reaktionsgemisch wird mit Chloroform verdünnt und mit Natriumbicarbonatlösung, 1 normaler Salzsäure und gesättigter Natriumchloridlösung gewaschen, über Magnesiumsulfat getrocknet und eingedampft.
Das Rohprodukt wird an Kieselgel chromatographiert (Eluiermittel Methylenchlorid-Ethanol 95:5); Smp. 220 Grad.

Beispiel 30: N-(4-Fluorobenzoyl)-staurosporin

Eine Lösung von 116,5 mg (0,25 mMol) Staurosporin und 0,065 ml (0,38 mMol) N,N-Diisopropyl-ethylamin in 2 ml Chloroform wird bei Raumtemperatur mit 0,036 ml (0,30 mMol) 4-Fluorobenzoylchlorid versetzt und eine Stunde gerührt. Das Reaktionsgemisch wird mit Chloroform verdünnt und mit Natriumbicarbonatlösung, 1 normaler Salzsäure und gesättigter Natriumchloridlösung gewaschen, über Magnesiumsulfat getrocknet und eingedampft.
Das Rohprodukt wird an Kieselgel chromatographiert (Eluiermittel Methylenchlorid-Ethanol 95:5); Smp. 225 Grad (Zersetzung).

Beispiel 31: N-(4-Chlorobenzoyl)-staurosporin

Eine Lösung von 233 mg (0,5 mMol) Staurosporin und 0,13 ml (0,76 mMol) N,N-Diisopropyl-ethylamin in 4 ml Chloroform wird bei Raumtemperatur mit 0,077 ml (0,6 mMol) 4-Chlorobenzoylchlorid versetzt und eine Stunde gerührt. Das Reaktionsgemisch wird mit Chloroform verdünnt und mit Natriumbicarbonatlösung, 1 normaler Salzsäure und gesättigter Natriumchloridlösung gewaschen, über Magnesiumsulfat getrocknet und eingedampft.
Das Rohprodukt wird an Kieselgel chromatographiert (Eluiermittel Methylenchlorid-Ethanol 95:5); Smp. 220 Grad (Zersetzung).

Beispiel 32: N-(3-Fluorobenzoyl)-staurosporin

Eine Lösung von 233 mg (0,5 mMol) Staurosporin und 0,13 ml (0,76 mMol) N,N-Diisopropyl-ethylamin in 4 ml Chloroform wird bei Raumtemperatur mit 0,072 ml (0,6 mMol) 3-Fluorobenzoylchlorid versetzt und eine Stunde gerührt. Das Reaktionsgemisch wird mit Chloroform verdünnt und mit Natriumbicarbonatlösung, 1 normaler Salzsäure und gesättigter Natriumchloridlösung gewaschen, über Magnesiumsulfat getrocknet und eingedampft.
Das Rohprodukt wird an Kieselgel chromatographiert (Eluiermittel Methylenchlorid-Ethanol 95:5); Smp. 240 Grad (Zersetzung).

Beispiel 33: N-(4-Nitrobenzoyl)-staurosporin

Eine Lösung von 233 mg (0,5 mMol) Staurosporin und 0,13 ml (0,76 mMol) N,N-Diisopropyl-ethylamin in 4 ml Chloroform wird bei Raumtemperatur mit 0,11 ml (0,6 mMol) 4-Nitrobenzoylchlorid versetzt und eine Stunde gerührt. Das Reaktionsgemisch wird mit Chloroform verdünnt und mit Natriumbicarbonatlösung, 1 normaler Salzsäure und gesättigter Natriumchloridlösung gewaschen, über Magnesiumsulfat getrocknet und eingedampft.
Das Rohprodukt wird an Kieselgel chromatographiert (Eluiermittel Methylenchlorid-Ethanol 95:5); Smp. 255 Grad.

Beispiel 34: N-(4-Methoxycarbonylbenzoyl)-staurosporin

Eine Lösung von 466 mg (1 mMol) Staurosporin und 0,26 ml (1,52 mMol) N,N-Diisopropyl-ethylamin in 8 ml Chloroform wird bei Raumtemperatur mit 237 mg (1,2 mMol) 4-Methoxycarbonylbenzoylchlorid versetzt und eine Stunde bei Raumtemperatur gerührt. Das Reaktionsgemisch wird mit Chloroform verdünnt und mit Natriumbicarbonatlösung, 1 normaler Salzsäure und gesättigter Natriumchloridlösung gewaschen, über Magnesiumsulfat getrocknet und eingedampft.
Das Rohprodukt wird an Kieselgel chromatographiert (Eluiermittel Methylenchlorid-Ethanol 95:5); Smp. 240 Grad (Zersetzung).

Beispiel 35: N-Thiobenzoyl-staurosporin

Eine Gemisch von 180 mg (0,31 mMol) N-Benzoyl-Staurosporin (Beispiel 18) und 132 mg (0,326 mMol) Lawesson-Reagens (Fluka AG) in 2 ml Toluol wird 48 Stunden bei Raumtemperatur gerührt. Zur Aufarbeitung wird mit Methylenchlorid verdünnt, mit gesättigter Natriumbicarbonat-Lösung und gesättigter Natriumchlorid-Lösung gewaschen, über Magnesiumsulfat getrocknet und eingedampft. Das Rohprodukt wird an Kieselgel chromatographiert (Eluiermittel Essigsäure-ethylester); FD-MS: 586; H-NMR (300 MHz in CDCl3): 2,99 s (3H); 2,62 s (3H); 2,56 s (3H).

Beispiel 36: N-tert.-Butoxycarbonyl-staurosporin

Eine Lösung von 116,5 mg (0,25 mMol) Staurosporin in 2 ml Tetrahydrofuran wird bei Raumtemperatur mit einer Lösung von 65 mg (0,297 mMol) Di-tert.-butyl-dicarbonat in 1 ml Tetrahydrofuran versetzt und 9 Stunden gerührt. Das Reaktionsgemisch wird eingedampft und an Kieselgel chromatographiert (Eluiermittel Methylenchlorid/Ethanol 95:5); Smp. ~160 Grad.

Beispiel 37: N-(4-Carboxybenzoyl-staurosporin-natriumsalz

Eine Gemisch von 314 mg (0,5 mMol) N-(4-Methoxycarbonylbenzoyl)-staurosporin (Beispiel 34) 10 ml Methanol und 0,3 ml 2 normale Natronlauge wird während 24 Stunden unter Rückfluss erhitzt. Nach dem Abkühlen wird filtriert, mit 10 ml Wasser verdünnt und mit 0,1 ml Essigsäure neutralisiert; dabei fällt die Titelverbindung als Säure aus (Smp. 275 Grad). Zur Herstellung des Natriumsalzes wird die Säure in 10 ml Methanol suspendiert und mit einem Aequivalent (5 ml) einer 0,1 normalen Natronlauge versetzt. Die resultierende Lösung wird eingedampft und der Rückstand aus Methanol/Ether umkristallisiert; FAB-MS: 637 (M+M)+; 659 (M+Na)+.

Beispiel 38: N-(3,5-Dinitrobenzoyl)-staurosporin

Eine Lösung von 233 mg (0,5 mMol) Staurosporin und 0,13 ml (0,76 mMol) N,N-Diisopropyl-ethylamin in 4 ml Chloroform wird bei Raumtemperatur mit 138 mg (0,6 mMol) 3,5-Dinitrobenzoylchlorid versetzt und eine Stunde gerührt. Das Reaktionsgemisch wird mit Chloroform verdünnt und mit Natriumbicarbonatlösung, 1 normaler Salzsäure und gesättigter Natriumchloridlösung gewaschen, über Magnesiumsulfat getrocknet und eingedampft.
Das Rohprodukt wird an Kieselgel chromatographiert (Eluiermittel Methylenchlorid-Ethanol 95:5); Smp. ~250 Grad (Zersetzung).

Beispiel 39:N-[(tert.-Butoxycarbonylamino)-acetyl]-staurosporin

699 mg (1,5 mMol) Staurosporin in 4o ml trockenem Chloroform werden mit 264 mg (1,5 mMol) BOC-Glycin (Fluka AG) und 340 mg (1.65 mMol) Dicyclohexylcarbodiimid versetzt und 1,5 Stunden bei Raumtemperatur gerührt. Anschliessend wird das Reaktionsgemisch mit Chloroform verdünnt und mit Natriumbicarbonatlösung und gesättigter Natriumchloridlösung gewaschen, über Magnesiumsulfat getrocknet und eingedampft. Der Rückstand wird in wenig Methylenchlorid aufgeschlemmt und filtriert (Entfernen des Dicyclohexylharnstoffs). Das Filtrat wird eingedampft und getrocknet; Smp. 190 Grad.

Beispiel 40: N-(2-Aminoacetyl)-staurosporin

Eine Lösung von 187 mg (0,3 mMol) N-[(tert.-Butoxycarbonylamino)-acetyl]-staurosporin (Beispiel 39) in 1 ml Essigsäure-ethylester wird bei Raumtemperatur mit 1 ml einer gesättigten Lösung Salzsäure in Essigsäure-ethylester versetzt. Dabei entsteht sofort ein Niederschlag. Die Suspension wird noch 10 Stunden nachgerührt und das Produkt abfiltriert und mit Essigsäure-ethylester gewaschen; Smp. 280 Grad (Zersetzung).

Beispiel 41: N-(2-Hydroxy-propyl)-staurosporin

Ein Gemisch von 23,3 mg (50 µmol) Staurosporin in 1 ml Dioxan, 0,5 ml (0,05 M) Boratpuffer (pH 10,0) und 100 µl (1,5 Mmol) Propylenoxid wird 13 Tage gerührt. Das Gemisch wird zweimal mit Dichlormethan extrahiert. Die organische Phase wird über $Na_2SO_4$ getrocknet und das Lösungsmittel im Vakuum entfernt. Das Reaktionsprodukt wird durch halbpräparative HPLC (Lichrosorb Si 60,5 µm, 8 x 250 mm) gereinigt, wobei mit Wasser gesättigtes Dichlormethan/2-Propanol (98:2, v/v) bei einer Fliessgeschwindigkeit von 5 ml/Minute und ein Detektor mit 295 nm verwendet wird. Zwanzig Injektionen werden durchgeführt. Die Retentionszeit des Produkts beträgt 15,4 Minuten; Struktur durch EI-MS und $^1$H-NMR (360 Hz) gesichert.

Beispiel 42: N-Phenyl-staurosprorin

Eine Lösung von 2,4 mg (51 µMol) Staurosporin in 0,5 ml Dioxan und 50 µl einer 1N Phenyldiazoniumchlorid-Lösung (Organikum, 13 Ed. Deutscher Verlag der Wissenschaften, Berlin, 1974, Seite 583) wird 1 Stunde gerührt und zu einem Gemisch von 1 ml 1N NaHC03, 50 ml Dichlormethan und 5 ml Methanol zugegeben. Die organische Phase wird über $Na_2SO_4$ getrocknet, das Lösungsmittel entfernt und das Produkt durch halbpräparative HPLC unter den Bedingungen von Beispiel 41 gereinigt. Die Retentionszeit des Produkts beträgt 5,1 Minuten; Struktur durch EI-MS und $^1$H-NMR (360

Hz) gesichert.

Beispiel 43: In analoger Weise wie in einem der vorstehenden Ausführungsbeispielen beschrieben kann man herstellen:

N-Alanyl-staurosporin,
N-Arginyl-staurosporin,
N-Phenylalanyl-staurosporin,
N-Histidyl-staurosporin,
N-Seryl-staurosporin.

Beispiel 44: Tabletten enthaltend 20 mg an Wirkstoff, z.B. N-Methoxycarbonylmethyl-staurosporin, werden in folgender Zusammensetzung in üblicher Weise hergestellt:

Zusammensetzung:

| | |
|---|---|
| Wirkstoff | 20 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 145 mg |

Herstellung:

Der Wirkstoff wird mit einem Teil der Weizenstärke, mit Milchzucker und kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 145 mg Gewicht mit Bruchkerbe verpresst.

Beispiel 45:

Tabletten enthaltend 1 mg an Wirkstoff, z.B. N-Methoxycarbonylmethyl-staurosporin, werden in folgender Zusammensetzung in üblicher Weise hergestellt:

Zusammensetzung :

| | |
|---|---|
| Wirkstoff | 1 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 126 mg |

Herstellung:

Der Wirkstoff wird mit einem Teil der Weizenstärke, mit Milchzucker und kolloidaler Kieselsäure gemischt und die

Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 126 mg Gewicht mit Bruchkerbe verpresst.

Beispiel 46: Kapseln enthaltend 10 mg an Wirkstoff, z.B. N-Methoxycarbonylmethyl-staurosporin, werden wie folgt auf übliche Weise hergestellt:

Zusammensetzung:

| | |
|---|---|
| Wirkstoff | 2500 mg |
| Talkum | 200 mg |
| Kolloidale Kieselsäure | 50 mg |

Herstellung:

Die aktive Substanz wird mit Talkum und kolloidaler Kieselsäure innig gemischt, das Gemisch durch ein Sieb mit 0,5 mm Maschenweite getrieben und dieses in Portionen von jeweils 11 mg in Hartgelatinekapseln geeigneter Grösse abgefüllt.

Beispiel 47: Anstelle der in den Beispielen 44 bis 46 beschriebenen Verbindung können auch pharmazeutische Präparate als Wirkstoff, enthaltend eine andere der in den Beispielen 1 bis 43 beschriebenen Verbindungen hergestellt werden.

## Patentansprüche

### Patentansprüche für folgende Vertragsstaaten : AT, BE, CH, DE, FR, GB, IT, LI, LU, NL, SE

**1.** N-substituierte Derivate von Staurosporin der allgemeinen Formel

$$[Stau]\text{-}N(CH_3)\text{-}R \quad (I),$$

worin [Stau] der Rest der Teilformel

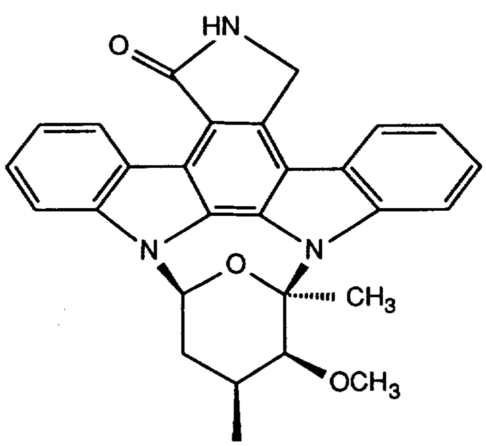

[Stau]

und R ein acyclischer, carbocyclischer oder carbocyclisch-acyclischer Kohlenwasserstoffrest $R^0$ ist, der insgesamt höchstens 30 Kohlenstoffatome hat und gesättigt oder ungesättigt und unsubstituiert oder substituiert sein kann, wobei anstelle von einem, zwei oder mehreren Kohlenstoffatomen gleiche oder verschiedene Heteroatome ausgewählt aus Sauerstoff, Schwefel und Stickstoff vorliegen können; oder ein Acyl Ac, welches höchstens 30 Kohlenstoffatome aufweist, darstellt; sowie Salze von Verbindungen der Formel I mit salzbildenden Eigenschaften.

**2.** Eine Verbindung der Formel I gemäss Anspruch 1, worin R ein $C_1$-$C_{20}$-Alkyl, ein $C_1$-$C_{20}$-Hydroxyalkyl, dessen Hydroxygruppe sich in beliebiger Stellung ausser 1-Stellung befindet, ein Cyano-[$C_1$-$C_{20}$]-alkyl, ein Carboxy-[$C_1$-$C_{20}$]-alkyl, dessen Carboxylgruppe auch in Salzform oder als ein $C_1$-$C_4$-Alkylester oder Benzylester vorliegen kann, oder ein $C_1$-$C_{20}$-Alkenyl, dessen freie Valenz sich nicht an demselben C-Atom wie die Doppelbindung befindet, darstellt.

**3.** Eine Verbindung gemäss Anspruch 1, worin R bicyclisches oder monocyclisches Aryl, oder ein analoges Heterocyclyl mit einem oder zwei Heteroatomen, wobei die freie Valenz an einem C-Atom lokalisiert ist, oder einen dieser Reste, der einen oder mehrere der folgenden Substituenten trägt: Halogenatome, $C_1$-$C_4$-Alkylreste, $C_1$-$C_4$-Alkoxygruppen, Methylendioxy, Nitrogruppen und/oder Carboxygruppen, welche frei, in einer Salzform oder als$C_1$-$C_4$-Alkylester vorliegen können, darstellt.

**4.** Eine Verbindung gemäss Anspruch 1, worin $R^0$ $C_1$-$C_7$-Alkyl, Hydroxy-$C_2$-$C_{18}$-alkyl, Cyano-$C_1$-$C_7$-alkyl, Carboxy-$C_1$-$C_7$-alkyl, $C_1$-$C_7$-Alkoxy-carbonyl-$C_1$-$C_7$-alkyl, Benzyloxycarbonyl-$C_1$-$C_7$-alkyl, $C_3$-$C_7$-Alkenyl, Phenyl, Naphthyl, Pyridyl, Chinolyl, Chinazolyl oder Phenyl-$C_1$-$C_7$-alkyl bedeutet, wobei die jeweiligen aromatischen Reste auch durch $C_1$-$C_7$-Alkyl, $C_1$-$C_7$-Alkoxy, Halogen, Nitro, Trifluormethyl, Carboxy, $C_1$-$C_4$-Alkoxy-carbonyl, Methylendioxy und/oder Cyano substituiert sein können, oder Salze davon, sofern salzbildende Gruppen vorliegen.

**5.** Verbindung gemäss Anspruch 1, worin R° $C_1$-$C_4$-Alkyl, Hydroxy-$C_2$-$C_{14}$-alkyl, Cyano-$C_1$-$C_4$-alkyl, Carboxy-$C_1$-$C_4$-alkyl, Carboxy-$C_1$-$C_4$-alkyl, $C_1$-$C_4$-Alkoxy-carbonyl-$C_1$-$C_4$-alkyl, $C_3$-$C_7$-Alkenyl oder Phenyl bedeutet, oder Salze davon, sofern salzbildende Gruppen vorliegen.

**6.** Eine Verbindung gemäss Anspruch 1, worin R ein Acyl mit höchstens 30 Kohlenstoffatomen der Teilformel Z-C(=W) - ist, worin W Sauerstoff, Schwefel oder Imino und Z Wasserstoff; Hydrocarbyl oder Hydrocarbyloxy, worin Hydrocarbyl ein acyclischer, carbocyclischer oder carbocyclisch-acyclischer Kohlenwasserstoffrest ist, der insgesamt höchstens 18 Kohlenstoffatome hat und gesättigt oder ungesättigt und unsubstituiert oder substituiert sein kann, wobei anstelle von einem, zwei oder mehreren Kohlenstoffatomen gleiche oder verschiedene Heteroatome ausgewählt aus Sauerstoff, Schwefel und Stickstoff vorliegen können; oder eine Aminogruppe ist; oder, falls W für Sauerstoff oder Schwefel steht, auch Chlor sein kann, oder Salze davon, sofern salzbildende Gruppen vorliegen.

**7.** Verbindung gemäss Anspruch 6, worin R ein Acyl der Teilformel Z-C(=W)- ist, worin W Sauerstoff oder Schwefel ist und Z $C_1$-$C_7$-Alkyl bedeutet, welches auch durch Halogen, Carboxy oder $C_1$-$C_4$-Alkoxycarbonyl substituiert sein kann, Phenyl, Pyridyl, Furyl, Thienyl, Imidazolyl, Chinolyl, Isochinolyl, Benzofuranyl oder Benzimidazolyl bedeutet, welches jeweils unsubstituiert oder durch $C_1$-$C_4$-Alkyl, $C_1$-$C_4$-Alkoxy, Halogen, Nitro, Trifluormethyl, Carboxy, $C_1$-$C_4$-Alkoxy-carbonyl, Methylendioxy und/oder Cyano substituiert ist, oder Salze davon, sofern salzbildende Gruppen vorliegen.

**8.** Eine Verbindung gemäss Anspruch 1, worin R ein von einer organischen Sulfonsäure abgeleitetes Acyl der Teilformel $R^0$-$SO_2$-, worin $R^0$ ein acyclischer, carbocyclischer oder carbocyclisch-acyclischer Kohlenwasserstoffrest ist, der insgesamt höchstens 30 Kohlenstoffatome hat und gesättigt oder ungesättigt und unsubstituiert oder substituiert sein kann, wobei anstelle von einem, zwei oder mehreren Kohlenstoffatomen gleiche oder verschiedene Heteroatome ausgewählt aus Sauerstoff, Schwefel und Stickstoff vorliegen können, darstellt, oder Salze davon, sofern salzbildende Gruppen vorliegen.

**9.** Eine Verbindung gemäss Anspruch 1, worin R ein von einer gegebenenfalls veresterten Phosphorsäure abgeleitetes Acyl der Teilformel

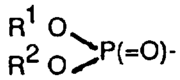

darstellt, worin $R^1$ und $R^2$ unabhängig voneinander je ein Wasserstoff, ein unsubstituiertes $C_1$-$C_7$-Alkyl oder ein unsubstituiertes oder durch $C_1$-$C_4$-Alkyl, $C_1$-$C_4$-Alkoxy, Halogen und/oder Nitro substituiertes Phenyl darstellen, oder Salze davon, sofern salzbildende Gruppen vorliegen.

**10.** Eine Verbindung gemäss Anspruch 1, worin R ein Acyl der Teilformel $R_b^°$-CO- ist, worin $R_b^°$ Wasserstoff, ein

$C_1$-$C_{19}$-Alkyl, das bei mehr als 5 C-Atomen eine lineare Kette aufweist, oder ein solches Alkyl substituiert durch Amino, Halogen und/oder Carboxyl in Form freier Säure, des Salzes, der Cyanogruppe oder eines $C_1$-$C_4$-Alkylesters darstellt, oder Salze davon, sofern salzbildende Gruppen vorliegen.

**11.** Verbindung gemäss Anspruch 1, worin R ein Acyl der Teilformel $R_b^o$-CO- ist, worin $R_b^o$ $C_1$-$C_7$-Alkyl bedeutet, welches unsubstituiert oder durch Halogen, Carboxy oder $C_1$-$C_4$-Alkoxycarbonyl substituiert sein kann, oder Phenyl ist, welches unsubstituiert oder durch $C_1$-$C_4$-Alkyl, $C_1$-$C_4$-Alkoxy, Halogen, Nitro, Trifluormethyl, Carboxy oder $C_1$-$C_4$-Alkoxycarbonyl substituiert sein kann, oder Salze davon, sofern salzbildende Gruppen vorliegen.

**12.** Eine Verbindung gemäss Anspruch 1, worin R ein bicyclisches oder monocyclisches Aroyl, welches auch einen oder mehrere der folgenden Substituenten tragen kann:
Halogenatome, Nitrogruppen, $C_1$-$C_4$-Alkylreste, Hydroxylgruppen, $C_1$-$C_4$-Alkoxy-, Phenoxy-, Methylendioxy- und/oder Carboxylgruppen (diese auch in der Salzform, als eine Cyanogruppe oder ein $C_1$-$C_4$-Alkylester), oder ein analoges Heteroaroyl, abgeleitet von Pyridin, Furan, Thiophen oder Imidazol oder von ihren Analogen mit kondensiertem Benzoring, darstellt, oder Salze davon, sofern salzbildende Gruppen vorliegen.

**13.** Eine Verbindung gemäss Anspruch 1, worin R ein Acyl der Teilformel $R^0$-$SO_2$- ist, worin $R^0$ $C_1$-$C_7$-Alkyl, Phenyl, Pyridyl, Furyl, Thienyl, Imidazolyl, Chinolyl, Isochinolyl, Benzofuranyl oder Benzimidazolyl bedeutet, welches jeweils unsubstituiert oder durch $C_1$-$C_4$-Alkyl, $C_1$-$C_4$-Alkoxy, Halogen, Nitro, Trifluormethyl, Carboxy, $C_1$-$C_4$-Alkoxycarbonyl, Methylendioxy und/oder Cyano substituiert ist, oder Salze davon, sofern salzbildende Gruppen vorliegen.

**14.** Eine Verbindung gemäss Anspruch 1, worin R ein von einem Monoester der Kohlensäure abgeleitetes Acyl der Teilformel $R^0$-O-CO- darstellt, worin $R^0$ ein $C_1$-$C_{20}$-Alkyl, welches unsubstituiert oder durch eine Carboxylgruppe (auch als Salz, Cyano oder $C_1$-$C_4$-Alkylester) substituiert sein kann, oder ein analoges lineares (Mono- bis Hexa-) -oxaalkyl mit 4-20 Kettengliedern, oder aber gegebenenfalls substituiertes Phenyl oder Benzyl ist.

**15.** Verbindung gemäss Anspruch 1, worin R ein Acyl der Teilformel $R^0$-O-CO- ist, worin $R^0$ $C_1$-$C_7$-Alkyl, Phenyl, Pyridyl, Furyl, Thienyl, Imidazolyl, Chinolyl, Isochinolyl, Benzofuranyl oder Benzimidazolyl bedeutet, welches jeweils unsubstituiert oder durch $C_1$-$C_4$-Alkyl, $C_1$-$C_4$-Alkoxy, Halogen, Nitro, Trifluormethyl, Carboxy, $C_1$-$C_4$-Alkoxycarbonyl, Methylendioxy und/oder Cyano substituiert ist, oder Salze davon, sofern salzbildende Gruppen vorliegen.

**16.** Eine Verbindung gemäss Anspruch 1, worin R ein Amid der Kohlensäure der Formel

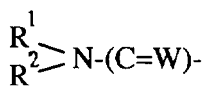

darstellt, worin W für Sauerstoff steht, einer der Reste $R^1$ und $R^2$ Wasserstoff ist und der andere ein $C_1$-$C_7$-Alkyl bedeutet, welches unsubstituiert oder durch Hydroxyl, Mercapto, Methylthio, Phenyl, p-Hydroxyphenyl, p-Methoxyphenyl, 2-Indolyl, 2-Imidazolyl und/oder Carboxyl (frei oder als $C_1$-$C_4$-Alkoxycarbonyl, Carbamoyl oder Amidino), wovon eines sich in 1-Stellung befindet, substituiert sein kann, oder Salze davon, sofern salzbildende Gruppen vorliegen.

**17.** Verbindung gemäss Anspruch 1, worin R ein Acyl der Teilformel

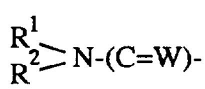

ist, worin W für Schwefel oder Sauerstoff steht, $R^1$ Wasserstoff ist und $R^2$ $C_1$-$C_7$-Alkyl, $C_3$-$C_7$-Alkenyl, Phenyl, Pyridyl, Furyl, Thienyl, Imidazolyl, Chinolyl, Isochinolyl, Benzofuranyl oder Benzimidazolyl bedeutet, welches jeweils unsubstituiert oder durch $C_1$-$C_4$-Alkyl, $C_1$-$C_4$-Alkoxy, Halogen, Nitro, Trifluormethyl, Carboxy, $C_1$-$C_4$-Alkoxy-carbonyl, Methylendioxy und/oder Cyano substituiert ist, oder Salze davon, sofern salzbildende Gruppen vorliegen.

**18.** Verbindung gemäss Anspruch 1, worin R von einer α-Aminosäure ausgewählt aus Glycin, Phenylglycin, Alanin, Phenylalanin, Prolin, Leucin, Serin, Valin, Tyrosin, Arginin, Histidin und Asparagin abgeleitet ist, oder ein Salz davon.

**19.** Verbindung ausgewählt aus der Gruppe bestehend aus

N-(3-Carboxypropionyl)-staurosporin,
N-Benzoyl-staurosporin,
N-Trifluoracetyl-staurosporin,
N-Methylaminothiocarbonyl-staurosporin und
N-Phenylcarbamoyl-staurosporin.

**20.** Verbindung ausgewählt aus der Gruppe bestehend aus

N-(3-Nitrobenzoyl)-staurosporin,
N-(3-Fluorobenzoyl)-staurosporin,
N-tert.-Butoxycarbonyl-staurosporin,
N-(4-Carboxybenzoyl)-staurosporin-natriumsalz,
N-(3,5-Dinitrobenzoyl)-staurosporin,
N-[(tert.-Butoxycarbonylamino)-acetyl]-staurosporin und
N-(2-Aminoacetyl)-staurosporin.

**21.** Eine Verbindung gemäss Anspruch 1, welche ein Additionssalz der Formel

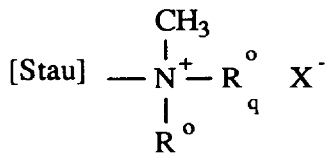

(IA)

ist, worin [Stau] und $R^0$ die in einem der Ansprüche 1 bis 7 angegebenen Bedeutungen haben, $R^o_q$ Wasserstoff oder ein unsubstituiertes $C_1$-$C_4$-Alkyl oder Benzyl ist und $X^-$ ein Anion einer anorganischen oder organischen Säure oder eines im Rest $R^0$ vorliegenden Carboxyls darstellt.

**22.** N-Carboxymethylstaurosporin gemäss Anspruch 1, oder ein pharmazeutisch verwendbares Salz davon.

**23.** N-Ethyl-staurosporin gemäss Anspruch 1, oder ein pharmazeutisch verwendbares Salz davon.

**24.** N-Benzoyl-staurosporin gemäss Anspruch 1.

**25.** N-Trifluoracetyl-staurosporin gemäss Anspruch 1.

**26.** N-(Phenylcarbamoyl)-staurosporin gemäss Anspruch 1.

**27.** N-Glycyl-staurosporin gemäss Anspruch 1, oder ein pharmazeutisch verwendbares Salz davon.

**28.** N-Alanyl-staurosporin gemäss Anspruch 1, oder ein pharmazeutisch verwendbares Salz davon.

**29.** Eine Verbindung gemäss einem der Ansprüche 1 bis 28 zur Anwendung als Hemmer der Proteinkinase C.

**30.** Verfahren zur Herstellung einer in Anspruch 1 definierten Verbindung der Formel I oder eines Salzes davon, dadurch gekennzeichnet, dass man Staurosporin der Formel [Stau]-NH-$CH_3$, worin [Stau] die im Anspruch 1 genannten Bedeutungen hat, oder ein Säureadditionssalz davon entweder

a) mit einem Reagens der Formel R-Y (III), worin R die in Anspruch 1 genannten Bedeutungen hat und Y eine reaktionsfähig aktivierte Hydroxylgruppe oder eine zusätzliche einfache Bindung, deren anderes Ende einen Wasserstoff im Rest R ersetzt, darstellt, oder

b) zur Herstellung von Verbindungen der Formel I, in welchen R den Rest der Teilformel H- $R^o_a$- darstellt, worin $R^o_a$ einen zweiwertigen, der allgemeinen Struktur des im Anspruch 1 definierten acyclischen Kohlenwasserstoff-

restes $R^0$ entsprechenden aliphatischen Rest bedeutet, mit einem Carbonylreagens der Formel $R_a^o$=O (IV), worin $R_a^o$ die obenstehende Bedeutung hat, und zugleich oder nachfolgend mit einem Reduktionsmittel umsetzt, und

gewünschtenfalls eine erhaltene Verbindung der Formel I in eine andere Verbindung der Formel I umwandelt und/oder eine in freier Form erhaltene Verbindung der Formel I in ein Salz davon überführt und/oder eine als Salz erhaltene Verbindung der Formel I in ihre freie Form oder in ein anderes Salz überführt.

**31.** Eine pharmazeutische Zusammensetzung enthaltend als Wirkstoff mindestens eine in einem der Ansprüche 1 bis 28 definierte Verbindung und einen oder mehrere pharmazeutisch verwendbare Hilfsstoffe.

**32.** Verwendung einer in einem der Ansprüche 1 - 28 definierten Verbindung zur Herstellung von pharmazeutischen Präparaten zur präventiven oder kurativen Behandlung von Krankheiten, bei welchen die Hemmung von Proteinkinase C von Bedeutung ist.

**Patentansprüche für folgende Vertragsstaaten : ES, GR**

**1.** Verfahren zur Herstellung N-substituierter Derivate von Staurosporin der allgemeinen Formel

$$[Stau]\text{-}N(CH_3)\text{-}R \qquad (I),$$

worin [Stau] der Rest der Teilformel

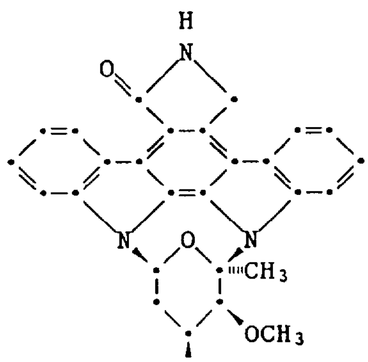

[Stau]

und R ein acyclischer, carbocyclischer oder carbocyclisch-acyclischer Kohlenwasserstoffrest $R^0$ ist, der insgesamt höchstens 30 Kohlenstoffatome hat und gesättigt oder ungesättigt und unsubstituiert oder substituiert sein kann, wobei anstelle von einem, zwei oder mehreren Kohlenstoffatomen gleiche oder verschiedene Heteroatome ausgewählt aus Sauerstoff, Schwefel und Stickstoff vorliegen können; oder ein Acyl Ac, welches höchstens 30 C-Atome aufweist, darstellt, sowie von Salzen von Verbindungen der Formel I mit salzbildenden Eigenschaften, dadurch gekennzeichnet, dass man Staurosporin der Formel [Stau]-NH-$CH_3$ (II), worin [Stau] die im Anspruch 1 genannte Bedeutung hat, oder ein Säureadditionssalz davon entweder

a) mit einem Reagens der Formel R-Y (III), worin R die im Anspruch 1 genannten Bedeutungen hat und Y eine reaktionsfähig aktivierte Hydroxylgruppe oder eine zusätzliche einfache Bindung, derer anderes Ende einen Wasserstoff im Rest R ersetzt, darstellt, oder,

b) zur Herstellung von Verbindungen der Formel I, in welchen R den Rest der Teilformel H-$R_a^o$- darstellt, worin $R_a^o$ einen zweiwertigen, der allgemeinen Struktur des im Anspruch 1 definierten acyclischen Kohlenwasserstoffrestes $R^0$ entsprechenden aliphatischen Rest darstellt, mit einem Carbonylreagens der Formel $R_a^o$ =O (IV), worin $R_a^o$ die obenstehende Bedeutung hat, und zugleich oder nachfolgend mit einem Reduktionsmittel umsetzt, und

gewünschtenfalls eine erhaltene Verbindung der Formel I in eine andere Verbindung der Formel I umwandelt

und/oder eine in freier Form erhaltene Verbindung der Formel I in ein Salz davon überführt und/oder eine als Salz erhaltene Verbindung der Formel I in ihre freie Form oder in ein anderes Salz überführt.

2. Verfahren gemäss Anspruch 1 zur Herstellung von Verbindungen der Formel I, worin R ein $C_1$-$C_{20}$-Alkyl, ein $C_2$-$C_{20}$-Hydroxyalkyl, dessen Hydroxylgruppe sich in beliebiger Stellung ausser 1-Stellung befindet, ein Cyano-[$C_1$-$C_{20}$]-alkyl, ein Carboxy-[$C_1$-$C_{20}$]-alkyl, dessen Carboxylgruppe auch in Salzform oder als ein $C_1$-$C_4$-Alkylester oder Benzylester vorliegen kann, oder ein $C_1$-$C_{20}$-Alkenyl, dessen freie Valenz sich nicht an demselben C-Atom wie die Doppelbindung befindet, darstellt.

3. Verfahren gemäss Anspruch 1 zur Herstellung von Verbindungen der Formel I, worin R bicyclisches oder monocyclisches Aryl, oder ein analoges Heterocyclyl mit einem oder 2 Heteroatomen, wobei die freie Valenz an einem C-Atom lokalisiert ist, oder einen dieser Reste, der einen oder mehrere der folgenden Substituenten trägt: Halogenatome, $C_1$-$C_4$-Alkylreste, $C_1$-$C_4$-Alkoxygruppen, Methylendioxy, Nitrogruppen und/oder Carboxylgruppen, welche frei, in einer Salzform, oder als $C_1$-$C_4$-Alkylester vorliegen können, darstellt.

4. Verfahren gemäss Anspruch 1 zur Herstellung von Verbindungen der Formel I, worin $R^0$ $C_1$-$C_7$-Alkyl, Hydroxy-$C_2$-$C_{18}$-alkyl, Cyano-$C_1$-$C_7$-alkyl, Carboxy-$C_1$-$C_7$-alkyl, $C_1$-$C_7$-Alkoxy-carbonyl-$C_1$-$C_7$-alkyl, Benzyloxy-carbonyl-$C_1$-$C_7$-alkyl, $C_3$-$C_7$-Alkenyl, Phenyl, Naphthyl, Pyridyl, Chinolyl, Chinazolyl oder Phenyl-$C_1$-$C_7$-alkyl bedeutet, wobei die jeweiligen aromatischen Reste auch durch $C_1$-$C_7$-Alkyl, $C_1$-$C_7$-Alkoxy, Halogen, Nitro, Trifluormethyl, Carboxy, $C_1$-$C_4$-Alkoxy-carbonyl, Methylendioxy und/oder Cyano substituiert sein können, oder Salzen davon, sofern salzbildende Gruppen vorliegen, dadurch gekennzeichnet, dass man entsprechend substituierte Ausgangsmaterialien verwendet.

5. Verfahren gemäss Anspruch 1 zur Herstellung von Verbindungen der Formel I, worin $R^0$ $C_1$-$C_4$-Alkyl, Hydroxy-$C_2$-$C_{14}$-alkyl, Cyano-$C_1$-$C_4$-alkyl, Carboxy-$C_1$-$C_4$-alkyl, $C_1$-$C_4$-Alkoxy-carbonyl-$C_1$-$C_4$-alkyl, $C_3$-$C_7$-Alkenyl oder Phenyl bedeutet, oder Salzen davon, sofern salzbildende Gruppen vorliegen, dadurch gekennzeichnet, dass man entsprechend substituierte Ausgangsmaterialien verwendet.

6. Verfahren gemäss Anspruch 1 zur Herstellung von Verbindungen der Formel I, worin R ein Acyl mit höchstens 30 C-Atomen der Teilformel Z-C(=W)- ist, worin W Sauerstoff, Schwefel oder Imino und Z Wasserstoff, Hydrocarbyl oder Hydrocarbyloxy, worin Hydrocarbyl ein acylischer, carbocyclischer oder carbocyclisch-acyclischer Kohlenwasserstoffrest ist, der insgesamt höchstens 18 Kohlenstoffatome hat und gesättigt oder ungesättigt und unsubstituiert oder substituiert sein kann, wobei anstelle von einem, zwei oder mehreren Kohlenstoffatomen gleiche oder verschiedene Heteroatome ausgewählt aus Sauerstoff, Schwefel und Stickstoff vorliegen können; oder eine Aminogruppe ist, oder, falls W für Sauerstoff oder Schwefel steht, auch Chlor sein kann, oder Salzen davon, sofern salzbildende Gruppen vorliegen, dadurch gekennzeichnet, dass man entsprechend substituierte Ausgangsmaterialien verwendet.

7. Verfahren gemäss Anspruch 6 zur Herstellung von Verbindungen der Formel I, worin R ein Acyl der Teilformel Z-C(=W)- ist, worin W Sauerstoff, oder Schwefel ist und Z $C_1$-$C_7$-Alkyl bedeutet, welches auch durch Halogen, Carboxy oder $C_1$-$C_4$-Alkoxy-carbonyl substituiert sein kann, oder Phenyl, Pyridyl, Furyl, Thienyl, Imidazolyl, Chinolyl, Isochinolyl, Benzofuranyl oder Benzimidazolyl bedeutet, welches jeweils unsubstituiert oder durch $C_1$-$C_4$-Alkyl, $C_1$-$C_4$-Alkoxy, Halogen, Nitro, Trifluormethyl, Carboxy, $C_1$-$C_4$-Alkoxy-carbonyl, Methylendioxy und/oder Cyano substituiert ist, oder Salzen davon, sofern salzbildende Gruppen vorliegen, dadurch gekennzeichnet, dass man entsprechend substituierte Ausgangsmaterialien verwendet.

8. Verfahren gemäss Anspruch 1 zur Herstellung von Verbindungen der Formel I, worin R ein von einer organischen Sulfonsäure abgeleitetes Acyl der Teilformel $R^0$-$SO_2$-, worin $R^0$ ein acylischer, carbocyclischer oder carbocyclisch-acyclischer Kohlenwasserstoffrest ist, der insgesamt höchstens 30 Kohlenstoffatome hat und gesättigt oder ungesättigt und unsubstituiert oder substituiert sein kann, wobei anstelle von einem, zwei oder mehreren Kohlenstoffatomen gleiche oder verschiedene Heteroatome ausgewählt aus Sauerstoff, Schwefel und Stickstoff vorliegen können, oder Salzen davon, sofern salzbildende Gruppen vorliegen, dadurch gekennzeichnet, dass man entsprechend substituierte Ausgangsmaterialien verwendet.

9. Verfahren gemäss Anspruch 1 zur Herstellung von Verbindungen der Formel I, worin R ein von einer gegebenenfalls veresterten Phosphorsäure abgeleitetes Acyl der Teilformel

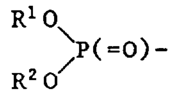

,

darstellt, worin $R^1$ und $R^2$ unabhängig voneinander je ein Wasserstoff, ein unsubstituiertes $C_1$-$C_7$-Alkyl oder ein gegebenenfalls durch $C_1$-$C_4$-Alkyl, $C_1$-$C_4$-Alkoxy, Halogen und/oder Nitro substituiertes Phenyl darstellt, oder Salzen davon, sofern salzbildende Gruppen vorliegen, dadurch gekennzeichnet, dass man entsprechend substituierte Ausgangsmaterialien verwendet.

**10.** Verfahren gemäss Anspruch 1 zur Herstellung von Verbindungen der Formel I, worin R ein Acyl der Teilformel $R_b^o$-CO- ist, worin $R_b^o$ Wasserstoff, ein $C_1$-$C_{19}$-Alkyl, das bei mehr als 5 C-Atomen eine lineare Kette aufweist, oder ein solches Alkyl substituiert durch Amino, Halogen und/oder Carboxyl in Form freier Säure, Salzes, der Cyanogruppe oder eines $C_1$-$C_4$-Alkylesters, darstellt, oder Salzen davon, sofern salzbildende Gruppen vorliegen, dadurch gekennzeichnet, dass man entsprechend substituierte Ausgangsmaterialien verwendet.

**11.** Verfahren gemäss Anspruch 1 zur Herstellung von Verbindungen der Formel I, worin R ein Acyl der Teilformel $R_b^o$-CO- ist, worin $R_b^o$ $C_1$-$C_7$-Alkyl bedeutet, welches unsubstituiert oder durch Halogen, Carboxy oder $C_1$-$C_4$-Alkoxy-carbonyl substituiert sein kann, oder Phenyl ist, welches unsubstituiert oder durch $C_1$-$C_4$-Alkyl, $C_1$-$C_4$-Alkoxy, Halogen, Nitro, Trifluormethyl, Carboxy oder $C_1$-$C_4$-Alkoxy-carbonyl substituiert sein kann, oder Salzen davon, sofern salzbildende Gruppen vorliegen, dadurch gekennzeichnet, dass man entsprechend substituierte Ausgangsmaterialien verwendet.

**12.** Verfahren gemäss Anspruch 1 zur Herstellung von Verbindungen der Formel I, worin R ein Acyl der Teilformel $R_b^o$-CO- ist, worin $R_b^o$ ein bicyclisches oder monocyclisches Aroyl, welches auch einen oder mehrere der folgenden Substituenten tragen kann: Halogenatome, Nitrogruppen, $C_1$-$C_4$-Alkylreste, Hydroxylgruppen, $C_1$-$C_4$-Alkoxy-, Phenoxy-, Methylendioxy-und/oder Carboxylgruppen (diese auch in der Salzform oder als eine Cyanogruppe oder ein $C_1$-$C_4$-Alkylester), oder ein analoges Heteroaroyl, abgeleitet von Pyridin, Furan, Thiophen oder Imidazol oder von ihren Analogen mit kondensiertem Benzoring, darstellt oder Salzen davon, sofern salzbildende Gruppen vorliegen, dadurch gekennzeichnet, dass man entsprechend substituierte Ausgangsmaterialien verwendet.

**13.** Verfahren gemäss Anspruch 1 zur Herstellung von Verbindungen der Formel I, worin R ein Acyl der Teilformel $R^0$-$SO_2$-ist, worin $R^0$ $C_1$-$C_7$-Alkyl, Phenyl, Pyridyl, Furyl, Thienyl, Imidazolyl, Chinolyl, Isochinolyl, Benzofuranyl oder Benzimidazolyl bedeutet, welches jeweils unsubstituiert oder durch $C_1$-$C_4$-Alkyl, $C_1$-$C_4$-Alkoxy, Halogen, Nitro, Trifluormethyl, Carboxy, $C_1$-$C_4$-Alkoxy-carbonyl, Methylendioxy und/oder Cyano substituiert ist, oder Salzen davon, sofern salzbildende Gruppen vorliegen, dadurch gekennzeichnet, dass man entsprechend substituierte Ausgangsmaterialien verwendet.

**14.** Verfahren gemäss Anspruch 1 zur Herstellung von Verbindungen der Formel I, worin R ein von einem Monoester der Kohlensäure abgeleitetes Acyl der Teilformel $R^0$-O-CO-darstellt, worin $R^0$ ein $C_1$-$C_{20}$-Alkyl, welches durch eine Carboxylgruppe (auch als Salz, Cyano oder $C_1$-$C_4$-Alkylester) substituiert sein kann, oder ein analoges lineares (Mono- bis Hexa-)-oxaalkyl mit 4-20 Kettengliedern, oder aber ein gegebenenfalls substituiertes Phenyl oder Benzyl ist, dadurch gekennzeichnet, dass man entsprechend substituierte Ausgangsmaterialien verwendet.

**15.** Verfahren gemäss Anspruch 1 zur Herstellung von Verbindungen der Formel I, worin R ein Acyl der Teilformel $R^0$-O-CO- ist, worin $R^0$ $C_1$-$C_7$-Alkyl, Phenyl, Pyridyl, Furyl, Thienyl, Imidazolyl, Chinolyl, Isochinolyl, Benzofuranyl oder Benzimidazolyl bedeutet, welches jeweils unsubstituiert oder durch $C_1$-$C_4$-Alkyl, $C_1$-$C_4$-Alkoxy, Halogen, Nitro, Trifluormethyl, Carboxy, $C_1$-$C_4$-Alkoxy-carbonyl, Methylendioxy und/oder Cyano substituiert ist, dadurch gekennzeichnet, dass man entsprechend substituierte Ausgangsmaterialien verwendet.

**16.** Verfahren gemäss Anspruch 1 zur Herstellung von Verbindungen der Formel I, worin R ein Amid der Kohlensäure der Formel

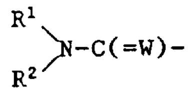

darstellt, worin W für Sauerstoff steht, einer der Reste $R^1$ und $R^2$ Wasserstoff ist und der andere ein $C_1$-$C_7$-Alkyl bedeutet, welches durch Hydroxyl, Mercapto, Methylthio, Phenyl, p-Hydroxyphenyl, p-Methoxyphenyl, 2-Indolyl, 2-Imidazolyl und/oder Carboxyl (frei oder als $C_1$-$C_4$-Alkoxycarbonyl, Carbamoyl oder Amidino), wovon eines sich in 1-Stellung befindet, substituiert sein kann, oder Salzen davon, sofern salzbildende Gruppen vorliegen, dadurch gekennzeichnet, dass man entsprechend substituierte Ausgangsmaterialien verwendet.

**17.** Verfahren gemäss Anspruch 1 zur Herstellung von Verbindungen der Formel I, worin R ein Acyl der Teilformel

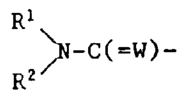

ist, worin W für Schwefel oder insbesondere Sauerstoff steht, $R_1$ Wasserstoff ist und $R_2$ $C_1$-$C_7$-Alkyl, $C_3$-$C_7$-Alkenyl, Phenyl, Pyridyl, Furyl, Thienyl, Imidazolyl, Chinolyl, Isochinolyl, Benzofuranyl oder Benzimidazolyl bedeutet, welches jeweils unsubstituiert oder durch $C_1$-$C_4$-Alkyl, $C_1$-$C_4$-Alkoxy, Halogen, Nitro, Trifluormethyl, Carboxy, $C_1$-$C_4$-Alkoxy-carbonyl, Methylendioxy und/oder Cyano substituiert ist, oder Salzen davon, sofern salzbildende Gruppen vorliegen, dadurch gekennzeichnet, dass man entsprechend substituierte Ausgangsmaterialien verwendet.

**18.** Verfahren gemäss Anspruch 1 zur Herstellung von Verbindungen der Formel I, worin R von einer α-Aminosäure ausgewählt aus Glycin, Phenylglycin, Alanin, Phenylalanin, Prolin, Leucin, Serin, Valin, Tyrosin, Arginin, Histidin und Asparagin abgeleitet ist, oder von Salzen davon, dadurch gekennzeichnet, dass man entsprechend substituierte Ausgangsmaterialien verwendet.

**19.** Verfahren nach Anspruch 1, dadurch gekennzeichnet, dass man eine Verbindung der Formel I ausgewählt aus der Gruppe bestehend aus

N-(3-Carboxypropionyl)-staurosporin,
N-Benzoyl-staurosporin,
N-Trifluoracetyl-staurosporin,
N-Methylaminothiocarbonyl-staurosporin und
N-Phenylcarbamoyl-staurosporin herstellt, dadurch gekennzeichnet, dass man entsprechend substituierte Ausgangsmaterialien verwendet.

**20.** Verfahren nach Anspruch 1, dadurch gekennzeichnet, dass man eine Verbindung der Formel I ausgewählt aus der Gruppe bestehend aus

N-(3-Nitrobenzoyl)-staurosporin,
N-(3-Fluorobenzoyl)-staurosporin,
N-tert.-Butoxycarbonyl-staurosporin,
N-(4-Carboxybenzoyl-staurosporin-natriumsalz,
N-(3,5-Dinitrobenzoyl)-staurosporin,
N-[(tert.-Butoxycarbonylamino)-acetyl]-staurosporin und
N-(2-Aminoacetyl)-staurosporin herstellt, dadurch gekennzeichnet, dass man entsprechend substituierte Ausgangsmaterialien verwendet.

**21.** Verfahren gemäss Anspruch 1, dadurch gekennzeichnet, dass man eine Verbindung der Formel I, welche ein Additionssalz der Formel

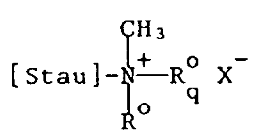
(IA)

ist, worin [Stau] und $R^0$ die in einem der Ansprüche 1-7 angegebenen Bedeutungen haben, $R^o_q$ Wasserstoff oder

ein unsubstituiertes $C_1$-$C_4$-Alkyl oder Benzyl ist und $X^-$ Anion einer anorganischen oder organischen Säure oder eines im Rest $R^0$ vorliegenden Carboxyls darstellt, dadurch gekennzeichnet, dass man entsprechend substituierte Ausgangsmaterialien verwendet.

**22.** Verfahren nach Anspruch 1 zur Herstellung von N-Carboxymethylstaurosporin, oder einem pharmazeutisch verwendbaren Salz davon, dadurch gekennzeichnet, dass man entsprechend substituierte Ausgangsmaterialien verwendet.

**23.** Verfahren nach Anspruch 1 zur Herstellung von N-Ethyl-staurosporin, dadurch gekennzeichnet, dass man entsprechend substituierte Ausgangsmaterialien verwendet.

**24.** Verfahren nach Anspruch 1 zur Herstellung von N-Benzoyl-staurosporin, dadurch gekennzeichnet, dass man entsprechend substituierte Ausgangsmaterialien verwendet.

**25.** Verfahren nach Anspruch 1 zur Herstellung von N-Trifluoracetylstaurosporin, dadurch gekennzeichnet, dass man entsprechend substituierte Ausgangsmaterialien verwendet.

**26.** Verfahren nach Anspruch 1 zur Herstellung von N-(Phenylcarbamoyl)-staurosporin, dadurch gekennzeichnet, dass man entsprechend substituierte Ausgangsmaterialien verwendet.

**27.** Verfahren nach Anspruch 1 zur Herstellung von N-Glycyl-staurosporin oder einem pharmazeutisch verwendbaren Salz davon, dadurch gekennzeichnet, dass man entsprechend substituierte Ausgangsmaterialien verwendet.

**28.** Verfahren nach Anspruch 1 zur Herstellung von N-Alanyl-staurosporin oder einem pharmazeutisch verwendbaren Salz davon, dadurch gekennzeichnet, dass man entsprechend substituierte Ausgangsmaterialien verwendet.

**29.** Verfahren zur Herstellung einer pharmazeutischen Zusammensetzung enthaltend als Wirkstoff mindestens eine in einem der Ansprüche 1-28 definierte Verbindung, dadurch gekennzeichnet, dass man diesen Wirkstoff mit mindestens einem pharmazeutischen Trägermaterial verarbeitet.

**30.** Verwendung einer in einem der Ansprüche 1-28 definierten Verbindung zur Herstellung von pharmazeutischen Zusammensetzungen zur präventiven oder kurativen Behandlung von Krankheiten, bei welchen die Hemmung von Proteinkinase C von Bedeutung ist.

## Claims

### Claims for the following Contracting States : AT, BE, CH, DE, FR, GB, IT, LI, LU, NL, SE

**1.** An N-substituted derivative of staurosporin of the general formula

$$[Stau]\text{-}N(CH_3)\text{-}R \qquad (I)$$

in which [Stau] is a residue of the partial formula

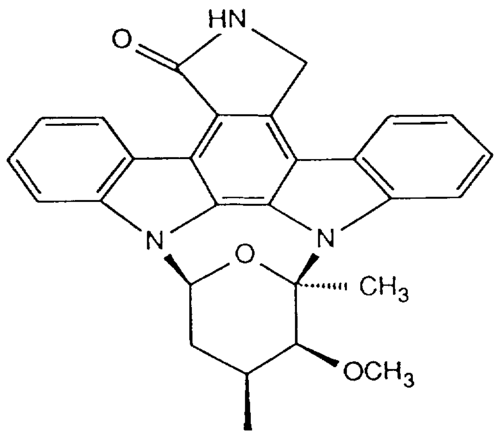

[Stau]

and R is an acyclic, carbocyclic or carbocyclic-acyclic hydrocarbon radical $R^0$ that has a total of at most 30 carbon atoms and may be saturated or unsaturated and unsubstituted or substituted, in which radical there may be present, in place of one, two or more carbon atoms, identical or different hetero atoms selected from oxygen, sulphur and nitrogen; or an acyl radical Ac that has a maximum of 30 carbon atoms; or a salt of a compound of formula I having salt-forming properties.

**2.** A compound of formula I according to claim 1, wherein R is a $C_1$-$C_{20}$alkyl radical, a $C_1$-$C_{20}$hydroxyalkyl radical of which the hydroxy group is in any position other than the 1-position, a cyano-[$C_1$-$C_{20}$]alkyl radical, a carboxy-[$C_1$-$C_{20}$] alkyl radical of which the carboxy group may also be in salt form or in the form of a $C_1$-$C_4$alkyl ester or benzyl ester, or a $C_1$-$C_{20}$alkenyl radical of which the free valency is not at the same carbon atom as the double bond.

**3.** A compound according to claim 1, wherein R is a bicyclic or monocyclic aryl radical or an analogous heterocyclyl radical having one or two hetero atoms, the free valency being located at a carbon atom, or R is one of those radicals carrying one or more of the following substituents: halogen atoms, $C_1$-$C_4$alkyl radicals, $C_1$-$C_4$alkoxy groups, methylenedioxy, nitro groups and/or carboxy groups that may be present in free or salt form or in the form of $C_1$-$C_4$alkyl ester groups.

**4.** A compound according to claim 1, wherein $R^0$ is $C_1$-$C_7$alkyl, hydroxy-$C_2$-$C_{18}$alkyl, cyano-$C_1$-$C_7$alkyl, carboxy-$C_1$-$C_7$alkyl, $C_1$-$C_7$alkoxycarbonyl-$C_1$-$C_7$alkyl, benzyloxycarbonyl-$C_1$-$C_7$alkyl, $C_3$-$C_7$alkenyl, phenyl, naphthyl, pyridyl, quinolyl, quinazolyl or phenyl-$C_1$-$C_7$alkyl, it being possible for the respective aromatic radicals also to be substituted by $C_1$-$C_7$alkyl, $C_1$-$C_7$-alkoxy, halogen, nitro, trifluoromethyl, carboxy, $C_1$-$C_4$alkoxycarbonyl, methylenedioxy and/or by cyano, or a salt thereof if salt-forming groups are present.

**5.** A compound according to claim 1, wherein $R^0$ is $C_1$-$C_4$alkyl, hydroxy-$C_2$-$C_{14}$alkyl, cyano-$C_1$-$C_4$alkyl, carboxy-$C_1$-$C_4$alkyl, carboxy-$C_1$-$C_4$alkyl, $C_1$-$C_4$alkoxycarbonyl-$C_1$-$C_4$alkyl, $C_3$-$C_7$alkenyl or phenyl, or a salt thereof if salt-forming groups are present.

**6.** A compound according to claim 1, wherein R is an acyl radical having a maximum of 30 carbon atoms of the partial formula Z-C(=W)- in which W is oxygen, sulphur or imino and Z is hydrogen; hydrocarbyl or hydrocarbyloxy wherein hydrocarbyl is an acyclic, carbocyclic or carbocyclic-acyclic hydrocarbon radical that has a total of at most 18 carbon atoms and may be saturated or unsaturated and unsubstituted or substituted, in which radical there may be present, in place of one, two or more carbon atoms, identical or different hetero atoms selected from oxygen, sulphur and nitrogen; or an amino group; or, if W is oxygen or sulphur, Z may also be chlorine, or a salt thereof if salt-forming groups are present.

**7.** A compound according to claim 6, wherein R is an acyl radical of the partial formula Z-C(=W)- in which W is oxygen or sulphur and Z is $C_1$-$C_7$alkyl, which may also be substituted by halogen, by carboxy or by $C_1$-$C_4$alkoxycarbonyl, phenyl, pyridyl, furyl, thienyl, imidazolyl, quinolyl, isoquinolyl, benzofuranyl or benzimidazolyl, each of which is unsubstituted or is substituted by $C_1$-$C_4$alkyl, $C_1$-$C_4$alkoxy, halogen, nitro, trifluoromethyl, carboxy, $C_1$-$C_4$alkoxycarbonyl, methylenedioxy and/or by cyano; or a salt thereof if salt-forming groups are present.

**8.** A compound according to claim 1, wherein R is an acyl radical, derived from an organic sulphonic acid, of the partial formula $R^0$-$SO_2$- in which $R^0$ is an acyclic, carbocyclic or carbocyclic-acyclic hydrocarbon radical that has a total of

at most 30 carbon atoms and may be saturated or unsaturated and unsubstituted or substituted, in which radical there may be present, in place of one, two or more carbon atoms, identical or different hetero atoms selected from oxygen, sulphur and nitrogen, or a salt thereof if salt-forming groups are present.

**9.** A compound according to claim 1, wherein R is an acyl radical, derived from an optionally esterified phosphoric acid, of the partial formula

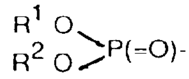

in which each of $R^1$ and $R^2$, independently of the other, is hydrogen, an unsubstituted $C_1$-$C_7$alkyl radical or a phenyl radical that is unsubstituted or is substituted by $C_1$-$C_4$alkyl, $C_1$-$C_4$alkoxy, halogen and/or by nitro, or a salt thereof if salt-forming groups are present.

**10.** A compound according to claim 1, wherein R is an acyl radical of the partial formula $R_b^o$-O- in which $R_b^o$ is hydrogen, a $C_1$-$C_{19}$alkyl group that with more than 5 carbon atoms has a linear chain, or such an alkyl group substituted by amino, halogen and/or by carboxy in the form of a free acid, a salt, a cyano group or a $C_1$-$C_4$alkyl ester, or a salt thereof if salt-forming groups are present.

**11.** A compound according to claim 1, wherein R is an acyl radical of the partial formula $R_b^o$-CO- in which $R_b^o$ is $C_1$-$C_7$alkyl, which may be unsubstituted or substituted by halogen, carboxy or by $C_1$-$C_4$alkoxycarbonyl, or is phenyl, which may be unsubstituted or substituted by $C_1$-$C_4$alkyl, $C_1$-$C_4$alkoxy, halogen, nitro, trifluoromethyl, carboxy or by $C_1$-$C_4$alkoxycarbonyl, or a salt thereof if salt-forming groups are present.

**12.** A compound according to claim 1, wherein R is a bicyclic or monocyclic aroyl radical, which may also carry one or more of the following substituents: halogen atoms, nitro groups, $C_1$-$C_4$alkyl radicals, hydroxy groups, $C_1$-$C_4$alkoxy, phenoxy, methylenedioxy and/or carboxy groups (the latter also in salt form or in the form of cyano groups or $C_1$-$C_4$alkyl ester groups), or an analogous heteroaroyl radical derived from pyridine, furan, thiophene or imidazole or from analogues thereof with a fused benzo ring, or a salt thereof if salt-forming groups are present.

**13.** A compound according to claim 1, wherein R is an acyl radical of the partial formula $R^0$-$SO_2$- in which $R^0$ is $C_1$-$C_7$alkyl, phenyl, pyridyl, furyl, thienyl, imidazolyl, quinolyl, isoquinolyl, benzofuranyl or benzimidazolyl, each of which is unsubstituted or is substituted by $C_1$-$C_4$alkyl, $C_1$-$C_4$alkoxy, halogen, nitro, trifluoromethyl, carboxy, $C_1$-$C_4$alkoxycarbonyl, methylenedioxy and/or by cyano, or a salt thereof if salt-forming groups are present.

**14.** A compound according to claim 1, wherein R is an acyl radical, derived from a monoester of carbonic acid, of the partial formula $R^0$-O-CO- in which $R^0$ is a $C_1$-$C_{20}$-alkyl radical that may be unsubstituted or substituted by a carboxy group (also in the form of a salt, a cyano group or a $C_1$-$C_4$alkyl ester group), or an analogous linear (mono- to hexa-) -oxaalkyl radical having from 4 to 20 chain members, or alternatively an unsubstituted or substituted phenyl or benzyl radical.

**15.** A compound according to claim 1, wherein R is an acyl radical of the partial formula $R^0$-O-CO- in which $R^0$ is $C_1$-$C_7$alkyl, phenyl, pyridyl, furyl, thienyl, imidazolyl, quinolyl, isoquinolyl, benzofuranyl or benzimidazolyl, each of which is unsubstituted or is substituted by $C_1$-$C_4$alkyl, $C_1$-$C_4$alkoxy, halogen, nitro, trifluoromethyl, carboxy, $C_1$-$C_4$alkoxycarbonyl, methylenedioxy and/or by cyano, or a salt thereof if salt-forming groups are present.

**16.** A compound according to claim 1, wherein R is an amide of carbonic acid of the formula

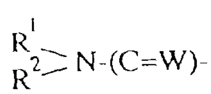

in which W is oxygen, one of the radicals $R^1$ and $R^2$ is hydrogen and the other is a $C_1$-$C_7$alkyl radical which may be unsubstituted or substituted by hydroxy, mercapto, methylthio, phenyl, p-hydroxyphenyl, p-methoxyphenyl, 2-indolyl, 2-imidazolyl and/or by carboxy (in free form or in the form of a $C_1$-$C_4$alkoxycarbonyl, carbamoyl or amidino group), one of which is in the 1-position, or a salt thereof if salt-forming groups are present.

**17.** A compound according to claim 1, wherein R is an acyl radical of the partial formula

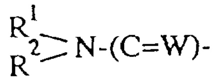

in which W is sulphur or oxygen, $R^1$ is hydrogen and $R^2$ is $C_1$-$C_7$alkyl, $C_3$-$C_7$alkenyl, phenyl, pyridyl, furyl, thienyl, imidazolyl, quinolyl, isoquinolyl, benzofuranyl or benzimidazolyl, each of which is unsubstituted or is substituted by $C_1$-$C_4$alkyl, $C_1$-$C_4$alkoxy, halogen, nitro, trifluoromethyl, carboxy, $C_1$-$C_4$alkoxycarbonyl, methylenedioxy and/or by cyano, or a salt thereof if salt-forming groups are present.

**18.** A compound according to claim 1, wherein R is derived from an $\alpha$-amino acid selected from glycine, phenylglycine, alanine, phenylalanine, proline, leucine, serine, valine, tyrosine, arginine, histidine and asparagine, or a salt thereof.

**19.** A compound selected from the group consisting of

N-(3-carboxypropionyl)-staurosporin,
N-benzoyl-staurosporin,
N-trifluoroacetyl-staurosporin,
N-methylaminothiocarbonyl-staurosporin and
N-phenylcarbamoyl-staurosporin.

**20.** A compound selected from the group consisting of

N-(3-nitrobenzoyl)-staurosporin,
N-(3-fluorobenzoyl)-staurosporin,
N-tert-butoxycarbonyl-staurosporin,
N-(4-carboxybenzoyl)-staurosporin sodium salt,
N-(3,5-dinitrobenzoyl)-staurosporin,
N-[(tert-butoxycarbonylamino)-acetyl]-staurosporin and
N-(2-aminoacetyl)-staurosporin.

**21.** A compound according to claim 1 that is an addition salt of the formula

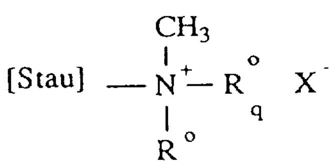

(IA)

wherein [Stau] and $R^0$ have the meanings given in any one of claims 1 to 7, $R^{o}_{q}$ is hydrogen or an unsubstituted $C_1$-$C_4$alkyl or benzyl radical and $X^-$ is an anion of an inorganic or organic acid or of a carboxy group present in the radical $R^0$.

**22.** N-Carboxymethylstaurosporin according to claim 1, or a pharmaceutically acceptable salt thereof.

**23.** N-Ethyl-staurosporin according to claim 1, or a pharmaceutically acceptable salt thereof.

**24.** N-Benzoyl-staurosporin according to claim 1.

**25.** N-Trifluoroacetyl-staurosporin according to claim 1.

**26.** N-(Phenylcarbamoyl)-staurosporin according to claim 1.

**27.** N-Glycyl-staurosporin according to claim 1, or a pharmaceutically acceptable salt thereof.

**28.** N-Alanyl-staurosporin according to claim 1, or a pharmaceutically acceptable salt thereof.

**29.** A compound according to any one of claims 1 to 28 for use as an inhibitor of protein kinase C.

**30.** A process for the preparation of a compound of formula I defined in claim 1 or a salt thereof, which comprises reacting staurosporin of the formula [Stau]-NH-$CH_3$ wherein [Stau] has the meaning given in claim 1, or an acid addition salt thereof, either

a) with a reagent of the formula R-Y (III) in which R has the meanings given in claim 1 and Y is a reactively activated hydroxy group or an additional single bond, the other end of which replaces a hydrogen atom in the radical R, or

b) to prepare a compound of formula I in which R is a radical of the partial formula H-$R^{o}_{a}$ - in which $R^{o}_{a}$ is a divalent aliphatic radical corresponding to the general structure of the acyclic hydrocarbon radical $R^0$ defined in claim 1, with a carbonyl reagent of the formula $R^{o}_{a}$=0 (IV) in which $R^{o}_{a}$ has the meaning given above, and at the same time or subsequently with a reducing agent, and,

if desired, converting a resulting compound of formula I into a different compound of formula I and/or converting a compound of formula I obtained in free form into a salt thereof and/or converting a compound of formula I obtained in the form of a salt into its free form or into a different salt.

**31.** A pharmaceutical composition comprising as active ingredient at least one of the compounds defined in any one of claims 1 to 28 and one or more pharmaceutically acceptable excipients.

**32.** The use of a compound defined in any one of claims 1 to 28 for the preparation of a pharmaceutical composition for the preventative or curative treatment of diseases in which the inhibition of protein kinase C is of importance.

**Claims for the following Contracting States : ES, GR**

**1.** A process for the preparation of an N-substituted derivative of staurosporin of the general formula

$$[Stau]\text{-}N(CH_3)\text{-}R \qquad (I)$$

in which [Stau] is a residue of the partial formula

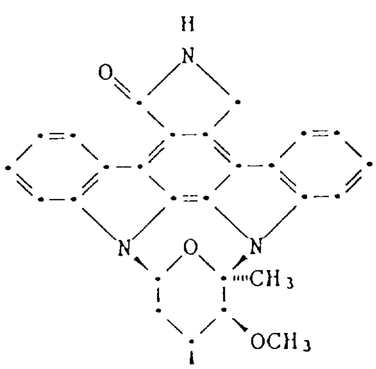

[Stau]

and R is an acyclic, carbocyclic or carbocyclic-acyclic hydrocarbon radical $R^0$ that has a total of at most 30 carbon atoms and may be saturated or unsaturated and unsubstituted or substituted, in which radical there may be present, in place of one, two or more carbon atoms, identical or different hetero atoms selected from oxygen, sulphur and nitrogen; or an acyl radical Ac that has a maximum of 30 carbon atoms; or of a salt of a compound of formula I having salt-forming properties, which comprises reacting staurosporin of the formula [Stau]-NH-$CH_3$ (II) wherein [Stau] has the meaning given in claim 1, or an acid addition salt thereof, either

a) with a reagent of the formula R-Y (III) in which R has the meanings given in claim 1 and Y is a reactively activated hydroxy group or an additional single bond, the other end of which replaces a hydrogen atom in the

radical R, or
b) to prepare a compound of formula I in which R is a radical of the partial formula H-$R_a^o$- in which $R_a^o$ is a divalent aliphatic radical corresponding to the general structure of the acyclic hydrocarbon radical $R^0$ defined in claim 1, with a carbonyl reagent of the formula $R_a^o$=O (IV) in which $R_a^o$ has the meaning given above, and at the same time or subsequently with a reducing agent, and, if desired, converting a resulting compound of formula I into a different compound of formula I and/or converting a compound of formula I obtained in free form into a salt thereof and/or converting a compound of formula I obtained in the form of a salt into its free form or into a different salt.

**2.** A process according to claim 1 for the preparation of a compound of formula I, wherein R is a $C_1$-$C_{20}$alkyl radical, a $C_2$-$C_{20}$hydroxyalkyl radical of which the hydroxy group is in any position other than the 1-position, a cyano-[$C_1$-$C_{20}$]alkyl radical, a carboxy-[$C_1$-$C_{20}$]alkyl radical of which the carboxy group may also be in salt form or in the form of a $C_1$-$C_4$alkyl ester or benzyl ester, or a $C_1$-$C_{20}$alkenyl radical of which the free valency is not at the same carbon atom as the double bond.

**3.** A process according to claim 1 for the preparation of a compound of formula I, wherein R is a bicyclic or monocyclic aryl radical or an analogous heterocyclyl radical having one or 2 hetero atoms, the free valency being located at a carbon atom, or R is one of those radicals carrying one or more of the following substituents: halogen atoms, $C_1$-$C_4$alkyl radicals, $C_1$-$C_4$alkoxy groups, methylenedioxy, nitro groups and/or carboxy groups that may be present in free or salt form or in the form of $C_1$-$C_4$alkyl ester groups.

**4.** A process according to claim 1 for the preparation of a compound of formula I, wherein $R^0$ is $C_1$-$C_7$alkyl, hydroxy-$C_2$-$C_{18}$alkyl, cyano-$C_1$-$C_7$alkyl, carboxy-$C_1$-$C_7$alkyl, $C_1$-$C_7$alkoxycarbonyl-$C_1$-$C_7$alkyl, benzyloxycarbonyl-$C_1$-$C_7$alkyl, $C_3$-$C_7$alkenyl, phenyl, naphthyl, pyridyl, quinolyl, quinazolyl or phenyl-$C_1$-$C_7$alkyl, it being possible for the respective aromatic radicals also to be substituted by $C_1$-$C_7$alkyl, $C_1$-$C_7$alkoxy, halogen, nitro, trifluoromethyl, carboxy, $C_1$-$C_4$alkoxycarbonyl, methylenedioxy and/or by cyano, or a salt thereof if salt-forming groups are present, which comprises using appropriately substituted starting materials.

**5.** A process according to claim 1 for the preparation of a compound of formula I, wherein $R^0$ is $C_1$-$C_4$alkyl, hydroxy-$C_2$-$C_{14}$alkyl, cyano-$C_1$-$C_4$alkyl, carboxy-$C_1$-$C_4$alkyl, $C_1$-$C_4$alkoxycarbonyl-$C_1$-$C_4$alkyl, $C_3$-$C_7$alkenyl or phenyl, or a salt thereof if salt-forming groups are present, which comprises using appropriately substituted starting materials.

**6.** A process according to claim 1 for the preparation of a compound of formula I, wherein R is an acyl radical having a maximum of 30 carbon atoms of the partial formula Z-C(=W)- in which W is oxygen, sulphur or imino and Z is hydrogen; hydrocarbyl or hydrocarbyloxy wherein hydrocarbyl is an acyclic, carbocyclic or carbocyclic-acyclic hydrocarbon radical that has a total of at most 18 carbon atoms and may be saturated or unsaturated and unsubstituted or substituted, in which radical there may be present, in place of one, two or more carbon atoms, identical or different hetero atoms selected from oxygen, sulphur and nitrogen; or an amino group; or, if W is oxygen or sulphur, Z may also be chlorine; or a salt thereof if salt-forming groups are present, which comprises using appropriately substituted starting materials.

**7.** A process according to claim 6 for the preparation of a compound of formula I, wherein R is an acyl radical of the partial formula Z-C(=W)- in which W is oxygen or sulphur and Z is $C_1$-$C_7$alkyl, which may also be substituted by halogen, by carboxy or by $C_1$-$C_4$alkoxycarbonyl, phenyl, pyridyl, furyl, thienyl, imidazolyl, quinolyl, isoquinolyl, benzofuranyl or benzimidazolyl, each of which is unsubstituted or is substituted by $C_1$-$C_4$alkyl, $C_1$-$C_4$alkoxy, halogen, nitro, trifluoromethyl, carboxy, $C_1$-$C_4$alkoxycarbonyl, methylenedioxy and/or by cyano; or a salt thereof if salt-forming groups are present, which comprises using appropriately substituted starting materials.

**8.** A process according to claim 1 for the preparation of a compound of formula I, wherein R is an acyl radical, derived from an organic sulphonic acid, of the partial formula $R^0$-$SO_2$- in which $R^0$ is an acyclic, carbocyclic or carbocyclic-acyclic hydrocarbon radical that has a total of at most 30 carbon atoms and may be saturated or unsaturated and unsubstituted or substituted, in which radical there may be present, in place of one, two or more carbon atoms, identical or different hetero atoms selected from oxygen, sulphur and nitrogen, or a salt thereof if salt-forming groups are present, which comprises using appropriately substituted starting materials.

**9.** A process according to claim 1 for the preparation of a compound of formula I, wherein R is an acyl radical, derived from an optionally esterified phosphoric acid, of the partial formula

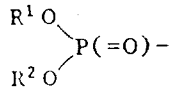
,

in which each of $R^1$ and $R^2$, independently of the other, is hydrogen, an unsubstituted $C_1$-$C_7$alkyl radical or a phenyl radical that is unsubstituted or is substituted by $C_1$-$C_4$alkyl, $C_1$-$C_4$alkoxy, halogen and/or by nitro, or a salt thereof if salt-forming groups are present, which comprises using appropriately substituted starting materials.

**10.** A process according to claim 1 for the preparation of a compound of formula I, wherein R is an acyl radical of the partial formula $R_b^o$-CO- in which $R_b^o$ is hydrogen, a $C_1$-$C_{19}$alkyl group that with more than 5 carbon atoms has a linear chain, or such an alkyl group substituted by amino, halogen and/or by carboxy in the form of a free acid, a salt, a cyano group or a $C_1$-$C_4$alkyl ester, or a salt thereof if salt-forming groups are present, which comprises using appropriately substituted starting materials.

**11.** A process according to claim 1 for the preparation of a compound of formula I, wherein R is an acyl radical of the partial formula $R_b^o$-CO- in which $R_b^o$ is $C_1$-$C_7$alkyl, which may be unsubstituted or substituted by halogen, carboxy or by $C_1$-$C_4$alkoxycarbonyl, or is phenyl, which may be unsubstituted or substituted by $C_1$-$C_4$alkyl, $C_1$-$C_4$alkoxy, halogen, nitro, trifluoromethyl, carboxy or by $C_1$-$C_4$alkoxycarbonyl, or a salt thereof if salt-forming groups are present, which comprises using appropriately substituted starting materials.

**12.** A process according to claim 1 for the preparation of a compound of formula I, wherein R is an acyl radical of the partial formula $R_b^o$-CO- in which $R_b^o$ is a bicyclic or monocyclic aroyl radical, which may also carry one or more of the following substituents: halogen atoms, nitro groups, $C_1$-$C_4$alkyl radicals, hydroxy groups, $C_1$-$C_4$alkoxy, phenoxy, methylenedioxy and/or carboxy groups (the latter also in salt form or in the form of cyano groups or $C_1$-$C_4$alkyl ester groups), or an analogous heteroaroyl radical derived from pyridine, furan, thiophene or imidazole or from analogues thereof with a fused benzo ring, or a salt thereof if salt-forming groups are present, which comprises using appropriately substituted starting materials.

**13.** A process according to claim 1 for the preparation of a compound of formula I, wherein R is an acyl radical of the partial formula $R^0$-$SO_2$- in which $R^0$ is $C_1$-$C_7$alkyl, phenyl, pyridyl, furyl, thienyl, imidazolyl, quinolyl, isoquinolyl, benzofuranyl or benzimidazolyl, each of which is unsubstituted or is substituted by $C_1$-$C_4$alkyl, $C_1$-$C_4$alkoxy, halogen, nitro, trifluoromethyl, carboxy, $C_1$-$C_4$alkoxycarbonyl, methylenedioxy and/or by cyano, or a salt thereof if salt-forming groups are present, which comprises using appropriately substituted starting materials.

**14.** A process according to claim 1 for the preparation of a compound of formula I, wherein R is an acyl radical, derived from a monoester of carbonic acid, of the partial formula $R^0$-O-CO- in which $R^0$ is a $C_1$-$C_{20}$alkyl radical that may be substituted by a carboxy group (also in the form of a salt, a cyano group or a $C_1$-$C_4$alkyl ester group), or an analogous linear (mono- to hexa-)-oxaalkyl radical having from 4 to 20 chain members, or alternatively an unsubstituted or substituted phenyl or benzyl radical, which comprises using appropriately substituted starting materials.

**15.** A process according to claim 1 for the preparation of a compound of formula I, wherein R is an acyl radical of the partial formula $R^0$-O-CO- in which $R^0$ is $C_1$-$C_7$alkyl, phenyl pyridyl, furyl, thienyl, imidazolyl, quinolyl, isoquinolyl, benzofuranyl or benzimidazolyl, each of which is unsubstituted or is substituted by $C_1$-$C_4$alkyl, $C_1$-$C_4$alkoxy, halogen, nitro, trifluoromethyl, carboxy, $C_1$-$C_4$alkoxycarbonyl, methylenedioxy and/or by cyano, which comprises using appropriately substituted starting materials.

**16.** A process according to claim 1 for the preparation of a compound of formula I, wherein R is an amide of carbonic acid of the formula

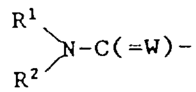

in which W is oxygen, one of the radicals $R^1$ and $R^2$ is hydrogen and the other is a $C_1$-$C_7$alkyl radical which may be substituted by hydroxy, mercapto, methylthio, phenyl, p-hydroxyphenyl, p-methoxyphenyl, 2-indolyl, 2-imidazolyl and/or by carboxy (in free form or in the form of a $C_1$-$C_4$alkoxycarbonyl, carbamoyl or amidino group), one of which is in the 1-position, or a salt thereof if salt-forming groups are present, which comprises using appropriately substituted starting materials.

**17.** A process according to claim 1 for the preparation of a compound of formula I, wherein R is an acyl radical of the partial formula

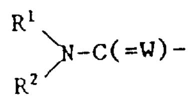

in which W is sulphur or especially oxygen, $R^1$ is hydrogen and $R^2$ is $C_1$-$C_7$alkyl, $C_3$-$C_7$alkenyl, phenyl, pyridyl, furyl, thienyl, imidazolyl, quinolyl, isoquinolyl, benzofuranyl or benzimidazolyl, each of which is unsubstituted or is substituted by $C_1$-$C_4$alkyl, $C_1$-$C_4$alkoxy, halogen, nitro, trifluoromethyl, carboxy, $C_1$-$C_4$alkoxycarbonyl, methylenedioxy and/or by cyano, or a salt thereof if salt-forming groups are present, which comprises using appropriately substituted starting materials.

**18.** A process according to claim 1 for the preparation of a compound of formula I, wherein R is derived from an $\alpha$-amino acid selected from glycine, phenylglycine, alanine, phenylalanine, proline, leucine, serine, valine, tyrosine, arginine, histidine and asparagine, or a salt thereof, which comprises using appropriately substituted starting materials.

**19.** A process according to claim 1, which comprises preparing a compound of formula I selected from the group consisting of

N-(3-carboxypropionyl)-staurosporin,
N-benzoyl-staurosporin,
N-trifluoroacetyl-staurosporin,
N-methylaminothiocarbonyl-staurosporin and
N-phenylcarbamoyl-staurosporin, and which comprises using appropriately substituted starting materials.

**20.** A process according to claim 1, which comprises preparing a compound of formula I selected from the group consisting of

N-(3-nitrobenzoyl)-staurosporin,
N-(3-fluorobenzoyl)-staurosporin,
N-tert-butoxycarbonyl-staurosporin,
N-(4-carboxybenzoyl)-staurosporin sodium salt,
N-(3,5-dinitrobenzoyl)-staurosporin,
N-[(tert-butoxycarbonylamino)-acetyl]-staurosporin and
N-(2-aminoacetyl)-staurosporin, and which comprises using appropriately substituted starting materials.

**21.** A process according to claim 1, which comprises preparing a compound of formula I that is an addition salt of the formula

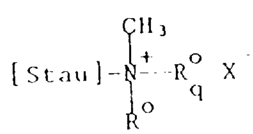

(IA)

wherein [Stau] and $R^0$ have the meanings given in any one of claims 1 to 7, $R_q^o$ is hydrogen or an unsubstituted

$C_1$-$C_4$alkyl or benzyl radical and $X^-$ is an anion of an inorganic or organic acid or of a carboxy group present in the radical $R^0$, and which comprises using appropriately substituted starting materials.

**22.** A process according to claim 1 for the preparation of N-carboxymethylstaurosporin, or a pharmaceutically acceptable salt thereof, which comprises using appropriately substituted starting materials.

**23.** A process according to claim 1 for the preparation of N-ethyl-staurosporin, which comprises using appropriately substituted starting materials.

**24.** A process according to claim 1 for the preparation of N-benzoyl-staurosporin, which comprises using appropriately substituted starting materials.

**25.** A process according to claim 1 for the preparation of N-trifluoroacetyl-staurosporin, which comprises using appropriately substituted starting materials.

**26.** A process according to claim 1 for the preparation of N-(phenylcarbamoyl)-staurosporin, which comprises using appropriately substituted starting materials.

**27.** A process according to claim 1 for the preparation of N-glycyl-staurosporin, or a pharmaceutically acceptable salt thereof, which comprises using appropriately substituted starting materials.

**28.** A process according to claim 1 for the preparation of N-alanyl-staurosporin, or a pharmaceutically acceptable salt thereof, which comprises using appropriately substituted starting materials.

**29.** A process for the preparation of a pharmaceutical composition comprising as active ingredient at least one of the compounds defined in any one of claims 1 to 28, which comprises processing that active ingredient with at least one pharmaceutical carrier.

**30.** The use of a compound defined in any one of claims 1 to 28 for the preparation of a pharmaceutical composition for the preventative or curative treatment of diseases in which the inhibition of protein kinase C is of importance.

**Revendications**

**Revendications pour les Etats contractants suivants : AT, BE, CH, DE, FR, GB, IT, LI, LU, NL, SE**

**1.** Dérivés N-substitués de staurosporine de formule générale

$$[Stau]\text{-}N(CH_3)\text{-}R \quad (I),$$

où [Stau] est le reste de formule partielle

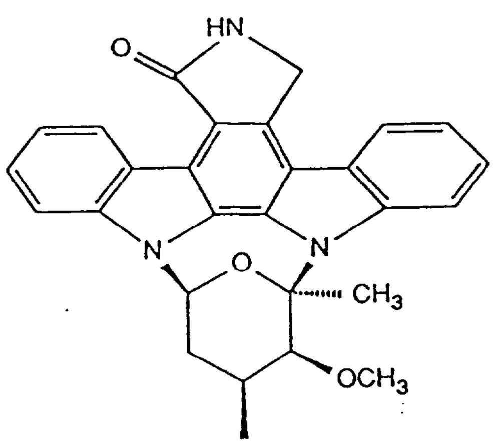

[Stau]

et R est un reste hydrocarboné $R^0$ acyclique, carbocyclique ou carbocyclique-acyclique, qui a au total au plus 30

atomes de carbone et peut être saturé ou insaturé et substitué ou non-substitué, tandis que peuvent être présents à la place d'un, deux ou plus de deux atomes de carbone des hétéroatomes identiques ou différents choisis parmi l'oxygène, le soufre et l'azote; ou un acyle Ac, qui présente au plus 30 atomes de carbone; ainsi que les sels des composés de formule I ayant des propriétés salifiantes.

**2.** Composé de formule I selon la revendication 1, dans lequel R représente un alkyle en $C_1$-$C_{20}$, un hydroxyalkyle en $C_1$-$C_{20}$, dont le groupe hydroxyle est en une position quelconque à l'exception de la position 1, un cyano(alkyle en $C_1$-$C_{20}$), un carboxy(alkyle en $C_1$-$C_{20}$), dont le groupe carboxyle peut également être présent sous forme de sel ou sous forme d'un ester d'alkyle en $C_1$-$C_4$ ou d'ester de benzyle, ou un alcényle en $C_1$-$C_{20}$, dont la valence libre n'est pas sur le même atome de carbone que la double liaison.

**3.** Composé selon la revendication 1, dans lequel R représente un aryle bicyclique ou monocyclique, ou un hétérocyclyle analogue ayant un ou deux hétéroatomes, tandis que la valence libre est localisée sur un atome de carbone, ou l'un de ces restes, qui porte un ou plusieurs des substituants suivants: atomes d'halogène, restes alkyle en $C_1$-$C_4$, groupes alcoxy en $C_1$-$C_4$, méthylènedioxy, groupes nitro et/ou groupes carboxy, qui peuvent être libres, sous forme de sel ou sous forme d'ester d'alkyle en $C_1$-$C_4$.

**4.** Composé selon la revendication 1, dans lequel $R^0$ désigne un groupe alkyle en $C_1$-$C_7$, hydroxy(alkyle en $C_2$-$C_{18}$), cyano(alkyle en $C_1$-$C_7$), carboxy(alkyle en $C_1$-$C_7$), alcoxy en $C_1$-$C_7$-carbonyl(alkyle en $C_1$-$C_7$), benzyloxycarbonyl (alkyle en $C_1$-$C_7$), alcényle en $C_3$-$C_7$, phényle, naphtyle, pyridyle, quinolyle et quinazolyle, ou phényl(alkyle en $C_1$-$C_7$), tandis que les restes aromatiques respectifs peuvent en outre être également substitués par des groupes alkyle en $C_1$-$C_7$, alcoxy en $C_1$-$C_7$, halogène, nitro, trifluorométhyle, carboxy, (alcoxy en $C_1$-$C_4$)carbonyle, méthylènedioxy et/ou cyano, ou ses sels, dans la mesure où il existe des groupes salifiables.

**5.** Composé selon la revendication 1, dans lequel $R^0$ désigne un groupe alkyle en $C_1$-$C_4$, hydroxy(alkyle en $C_2$-$C_{14}$), cyano(alkyle en $C_1$-$C_4$), carboxy(alkyle en $C_1$-$C_4$), carboxy(alkyle en $C_1$-$C_4$), alcoxy en $C_1$-$C_4$-carbonyl(alkyle en $C_1$-$C_4$), alcényle en $C_3$-$C_7$ ou phényle, ou ses sels, dans la mesure où il existe des groupes salifiables.

**6.** Composé selon la revendication 1, dans lequel R est un groupe acyle ayant au plus 30 atomes de carbone de formule partielle Z-C(=W)-, où W est l'oxygène, le soufre ou un radical imino et Z est l'hydrogène; hydrocarbyle ou hydrocarbyloxy, dans lequel le radical hydrocarbyle est un reste hydrocarboné acyclique, carbocyclique ou carbocyclique-acyclique, qui a au total au plus 18 atomes de carbone et peut être saturé ou insaturé et non-substitué ou substitué, tandis qu'à la place d'un, deux ou plus de deux atomes de carbone peuvent être présents des hétéroatomes identiques ou différents, choisis parmi l'oxygène, le soufre et l'azote; ou un groupe amino; ou, dans le cas où W désigne l'oxygène ou le soufre, peut également être du chlore, ou ses sels, dans la mesure où il existe des groupes salifiables.

**7.** Composé selon la revendication 6, dans lequel R est un acyle de formule partielle Z-C(=W)-, où W est l'oxygène ou le soufre et Z représente un alkyle en $C_1$-$C_7$, qui peut également être substitué par un radical halogène, carboxy ou alcoxy($C_1$-$C_4$)carbonyle; phényle, pyridyle, furyle, thiényle, imidazolyle, quinolyle, isoquinolyle, benzofurannyle ou benzimidazolyle, qui peut dans chaque cas être non-substitué ou substitué par un radical alkyle en $C_1$-$C_4$, alcoxy en $C_1$-$C_4$, halogène, nitro, trifluorométhyle, carboxy, alcoxy($C_1$-$C_4$)carbonyle, méthylènedioxy et/ou cyano, ou ses sels, dans la mesure où il existe des groupes salifiables.

**8.** Composé selon la revendication 1, dans lequel R représente un acyle dérivé d'un acide sulfonique organique de formule partielle $R^0$-$SO_2$-, dans laquelle $R^0$ est un reste hydrocarboné acyclique, carbocyclique ou carbocyclique-acyclique, qui a au total au plus 30 atomes de carbone et peut être saturé ou insaturé et non-substitué ou susbtitué, tandis qu'à la place d'un, deux ou plus de deux atomes de carbone sont présents des hétéroatomes identiques ou différents, choisis parmi l'oxygène, le soufre et l'azote, ou ses sels, dans la mesure où il existe des groupes salifiables.

**9.** Composé selon la revendication 1, dans lequel R représente un acyle dérivé d'acide phosphorique éventuellement estérifié de formule partielle

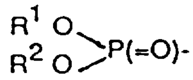

où $R^1$ et $R^2$ représentent chacun, indépendamment l'un de l'autre, un hydrogène, un alkyle en $C_1$-$C_7$ non-substitué

ou un phényle non-substitué ou substitué par un radical alkyle en $C_1$-$C_4$, alcoxy en $C_1$-$C_4$, halogène et/ou nitro, ou ses sels, dans la mesure où il existe des groupes salifiables.

**10.** Composé selon la revendication 1, dans lequel R est un acyle de formule partielle $R^0_b$-CO-, dans laquelle $R^0_b$ représente l'hydrogène, un alkyle en $C_1$-$C_{19}$, qui pour plus de 5 atomes de carbone présente une chaîne linéaire, ou un tel alkyle substitué par un radical amino, halogène et/ou carboxyle sous forme de l'acide libre, du sel, du groupe cyano ou d'un ester d'alkyle en $C_1$-$C_4$, ou ses sels, dans la mesure où il existe des groupes salifiables.

**11.** Composé selon la revendication 1, dans lequel R est un acyle de formule partielle $R^0_b$-CO-, où $R^0_b$ désigne un alkyle en $C_1$-$C_7$, qui peut être non-substitué ou substitué par un radical halogène, carboxy ou alcoxy($C_1$-$C_4$)carbonyle, ou est un phényle qui peut être non-substitué ou substitué par un radical alkyle en $C_1$-$C_4$, alcoxy en $C_1$-$C_4$, halogène, nitro, trifluorométhyle, carboxy ou alcoxy($C_1$-$C_4$)carbonyle, ou ses sels, dans la mesure où il existe des groupes salifiables.

**12.** Composé selon la revendication 1, dans lequel R représente un aroyle bicyclique ou monocyclique, qui peut également porter un ou plusieurs des substituants suivants: atomes d'halogène, groupes nitro, restes alkyle en $C_1$-$C_4$, groupes hydroxyle, groupes alcoxy en $C_1$-$C_4$, phénoxy, méthylènedioxy et/où carboxyle (ceux-ci également sous forme de sel, en tant que groupe cyano ou qu'ester d'alkyle en $C_1$-$C_4$), ou un hétéroaroyle analogue, dérivé de pyridine, furanne, thiophène ou imidazole ou de leurs analogues ayant un noyau benzénique condensé, ou leurs sels, dans la mesure où il existe des groupes salifiables.

**13.** Composé selon la revendication 1, dans lequel R est un acyle de formule partielle $R^0$-$SO_2$-, où $R^0$ désigne un radical alkyle en $C_1$-$C_7$, phényle, pyridyle, furyle, thiényle, imidazolyle, quinolyle, isoquinolyle, benzofurannyle ou benzimidazolyle, qui peut à chaque fois être non-substitué ou substitué par un groupe alkyle en $C_1$-$C_4$, alcoxy en $C_1$-$C_4$, halogène, nitro, trifluorométhyle, carboxy, alcoxy($C_1$-$C_4$)carbonyle, méthylènedioxy et/ou cyano, ou ses sels, dans la mesure où il existe des groupes salifiables.

**14.** Composé selon la revendication 1, dans lequel R représente un acyle de formule partielle $R^0$-O-CO- dérivé d'un monoester d'acide carboxylique, où $R^0$ est un alkyle en $C_1$-$C_{20}$, qui peut être non-substitué ou substitué par un groupe carboxyle (également en tant que sel, cyano ou ester d'alkyle en $C_1$-$C_4$), ou un (mono- à hexa-)-oxaalkyle linéaire analogue ayant 4-20 chaînons, ou même un phényle ou un benzyle éventuellement substitué.

**15.** Composé selon la revendication 1, dans lequel R est un acyle de formule partielle $R^0$-O-CO-, où $R^0$ désigne un radical alkyle en $C_1$-$C_7$, phényle, pyridyle, furyle, thiényle, imidazolyle, quinolyle, isoquinolyle, benzofurannyle ou benzimidazolyle, qui peut à chaque fois être non-substitué ou substitué par un groupe alkyle en $C_1$-$C_4$, alcoxy en $C_1$-$C_4$, halogène, nitro, trifluorométhyle, carboxy, alcoxy($C_1$-$C_4$)carbonyle, méthylènedioxy et/ou cyano, ou ses sels, dans la mesure où il existe des groupes salifiables.

**16.** Composé selon la revendication 1, dans lequel R représente un amide d'acide carboxylique de formule

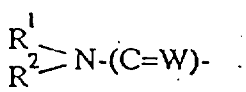

où W désigne l'oxygène, l'un des restes $R^1$ et $R^2$ est l'hydrogène et l'autre un alkyle en $C_1$-$C_7$, qui peut être non-substitué ou substitué par un radical hydroxyle, mercapto, méthylthio, phényle, p-hydroxyphényle, p-méthoxyphényle, 2-indolyle, 2-imidazolyle et/ou carboxyle (libre ou en tant qu'alcoxycarbonyle en $C_1$-$C_4$, carbamoyle ou amidino), dont l'un se trouve en position 1, ou ses sels, dans la mesure où il existe des groupes salifiables.

**17.** Composé selon la revendication 1, dans lequel R est un acyle de formule partielle

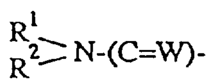

où W désigne le soufre ou l'oxygène, $R^1$ est l'hydrogène et $R^2$ représente un radical alkyle en $C_1$-$C_7$, alcényle en $C_3$-$C_7$, phényle, pyridyle, furyle, thiényle, imidazolyle, quinolyle, isoquinolyle, benzofurannyle ou benzimidazolyle, qui peut à chaque fois être non-substitué ou substitué par un groupe alkyle en $C_1$-$C_4$, alcoxy en $C_1$-$C_4$, halogène, nitro, trifluorométhyle, carboxy, alcoxy($C_1$-$C_4$)carbonyle, méthylènedioxy et/ou cyano, ou ses sels, dans la mesure où il existe des groupes salifiables.

**18.** Composé selon la revendication 1, dans lequel R est dérivé d'un acide $\alpha$-aminé choisi parmi la glycine, la phénylglycine, l'alanine, la phénylalanine, la proline, la leucine, la sérine, la valine, la tyrosine, l'arginine, l'histidine et l'asparagine, ou l'un de ses sels.

**19.** Composé choisi dans le groupe consistant en:

N-(3-carbxoypropionyl)-staurosporine,
N-benzoyl-staurosporine,
N-tridluoroacétyl-staurosporine,
N-méthylaminothiocarbonyl-staurosporine et
N-phénylcarbamoyl-staurosporine.

**20.** Composé choisi dans le groupe consistant en:

N-(3-nitrobenzoyl)-staurosporine,
N-(3-fluorobenzoyl)-staurosporine,
N-tert-butoxycarbonyl-staurosporine, sel de sodium de N-(4-carboxybenzoyl)-staurosporine,
N-(3,5-dinitrobenzoyl)-staurosporine,
N-[(tert-butoxycarbonylamino)-acétyl]-staurosporine et
N-(2-aminoacétyl)-staurosporine.

**21.** Composé selon la revendication 1, qui est un sel d'addition de formule

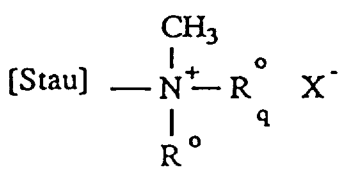

(IA)

où [Stau] et $R^0$ ont les significations indiquées dans les revendications 1 à 7, $R^0_q$ est l'hydrogène ou un radical alkyle en $C_1$-$C_4$ ou benzyle non-substitué et $X^-$ représente un anion d'un acide inorganique ou organique ou d'un carboxyle présent dans le reste $R^0$.

**22.** N-carboxyméthylstaurosporine selon la revendication 1, ou l'un de ses sels pharmaceutiquement acceptables.

**23.** N-éthyl-staurosporine selon la revendication 1, ou l'un de ses sels pharmaceutiquement acceptables.

**24.** N-benzoyl-staurosporine selon la revendication 1.

**25.** N-trifluoroacétyl-staurosporine selon la revendication 1.

**26.** N-(phénylcarbamoyl)-staurosporine selon la revendication 1.

**27.** N-glycyl-staurosporine selon la revendication 1, ou l'un de ses sels pharmaceutiquement acceptables.

**28.** N-alanyl-staurosporine selon la revendication 1, ou l'un de ses sels pharmaceutiquement acceptables.

**29.** Composé selon l'une quelconque des revendications 1 à 28 pour utilisation comme inhibiteur de la protéine-kinase C.

**30.** Procédé pour la préparation d'un composé défini dans la revendication 1 de formule I ou d'un de ses sels, caractérisé en ce qu'on fait réagir de la staurosporine de formule [Stau]-NH-$CH_3$, où [Stau] a les significations indiquées dans la revendication 1, ou un de ses sels d'addition d'acide, soit

a) avec un réactif de formule R-Y (III), où R a les significations indiquées dans la revendication 1 et Y représente un groupe hydroxyle activé réactif ou une liaison simple additionnelle, dont l'autre extrémité remplace un hydrogène dans le reste R, soit
b) pour la préparation de composés de formule I, dans lesquels R représente le reste de formule partielle H-$R^0_a$,

où $R^0_a$ désigne un reste aliphatique bivalent, correspondant à la structure générale du reste hydrocarboné acyclique $R^0$ défini dans la revendication 1, avec un réactif carbonylé de formule $R^0_a$=O (IV), où $R^0_a$ a la signification susdite, et simultanément ou subséquemment on fait réagir avec un agent réducteur, et si on le souhaite, on convertit un composé obtenu de formule I en un autre composé de formule I et/ou on transforme un composé de formule I obtenu sous forme libre en l'un de ses sels et/ou on transforme un composé de formule I obtenu en tant que sel en sa forme libre ou en un autre sel.

**31.** Composition pharmaceutique contenant comme agent actif au moins un composé défini dans l'une quelconque des revendications 1 à 28 et un ou plusieurs adjuvants pharmaceutiquement acceptables.

**32.** Utilisation d'un composé défini dans l'une quelconque des revendications 1 - 28 pour l'obtention de préparations pharmaceutiques pour le traitement préventif ou curatif de maladies, dans lesquelles l'inhibition de la protéine-kinase C a de l'importance.

**Revendications pour les Etats contractants suivants : ES, GR**

**1.** Procédé pour la préparation de dérivés N-substitués de staurosporine de formule générale

$$[Stau]-N(CH_3)-R \quad (I),$$

où [Stau] est le reste de formule partielle

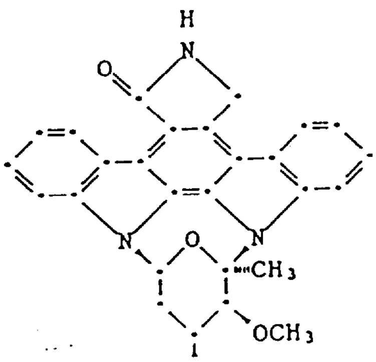

[Stau]

et R est un reste hydrocarboné $R^0$ acyclique, carbocyclique ou carbocyclique-acyclique, qui a au total au plus 30 atomes de carbone et peut être saturé ou insaturé et substitué ou non-substitué, tandis que peuvent être présents à la place d'un, deux ou plus de deux atomes de carbone des hétéroatomes identiques ou différents choisis parmi l'oxygène, le soufre et l'azote; ou un acyle Ac, qui présente au plus 30 atomes de carbone; ainsi que les sels des composés de formule I ayant des propriétés salifiantes, caractérisé en ce qu'on fait réagir de la staurosporine de formule [Stau]-NH-$CH_3$ (II), où [Stau] a la signification indiquée dans la revendication 1, ou un de ses sels d'addition d'acide, soit

a) avec un réactif de formule R-Y (III), où R a les significations indiquées dans la revendication 1 et Y représente un groupe hydroxyle activé réactif ou une liaison simple additionnelle, dont l'autre extrémité remplace un hydrogène dans le reste R, soit

b) pour la préparation de composés de formule I, dans lesquels R représente le reste de formule partielle H-$R^0_a$, où $R^0_a$ désigne un reste aliphatique bivalent, correspondant à la structure générale du reste hydrocarboné acyclique $R^0$ défini dans la revendication 1, avec un réactif carbonylé de formule $R^0_a$=O (IV), où $R^0_a$ a la signification susdite, et simultanément ou subséquemment on fait réagir avec un agent réducteur, et si on le souhaite, on convertit un composé obtenu de formule I en un autre composé de formule I et/ou on transforme un composé de formule I obtenu sous forme libre en l'un de ses sels et/ou on transforme un composé de formule I obtenu en tant que sel en sa forme libre ou en un autre sel.

**2.** Procédé selon la revendication 1 pour la préparation de composés de formule I, où R représente un alkyle en $C_1$-$C_{20}$, un hydroxyalkyle en $C_1$-$C_{20}$, dont le groupe hydroxyle est en une position quelconque à l'exception de la position 1, un cyano(alkyle en $C_1$-$C_{20}$), un carboxy(alkyle en $C_1$-$C_{20}$), dont le groupe carboxyle peut également être présent sous forme de sel ou sous forme d'un ester d'alkyle en $C_1$-$C_4$ ou d'ester de benzyle, ou un alcényle en

$C_1$-$C_{20}$, dont la valence libre n'est pas sur le même atome de carbone que la double liaison.

**3.** Procédé selon la revendication 1 pour la préparation de composés de formule I, où R représente un aryle bicyclique ou monocyclique, ou un hétérocyclyle analogue ayant un ou deux hétéroatomes, tandis que la valence libre est localisée sur un atome de carbone, ou l'un de ces restes, qui porte un ou plusieurs des substituants suivants: atomes d'halogène, restes alkyle en $C_1$-$C_4$, groupes alcoxy en $C_1$-$C_4$, méthylènedioxy, groupes nitro et/ou groupes carboxy, qui peuvent être libres, sous forme de sel ou sous forme d'ester d'alkyle en $C_1$-$C_4$.

**4.** Procédé selon la revendication 1 pour la préparation de composés de formule I, où $R^0$ désigne un groupe alkyle en $C_1$-$C_7$, hydroxy(alkyle en $C_2$-$C_{18}$), cyano(alkyle en $C_1$-$C_7$), carboxy(alkyle en $C_1$-$C_7$), alcoxy en $C_1$-$C_7$-carbonyl (alkyle en $C_1$-$C_7$), benzyloxy-carbonyl(alkyle en $C_1$-$C_7$), alcényle en $C_3$-$C_7$, phényle, naphtyle, pyridyle, quinolyle et quinazolyle, ou phényl(alkyle en $C_1$-$C_7$), tandis que les restes aromatiques respectifs peuvent en outre être également substitués par des groupes alkyle en $C_1$-$C_7$, alcoxy en $C_1$-$C_7$, halogène, nitro, trifluorométhyle, carboxy, (alcoxy en $C_1$-$C_4$)carbonyre, méthylènedioxy et/ou cyano, ou de leurs sels, dans la mesure où il existe des groupes salifiables, caractérisé en ce qu'on utilise des produits de départ substitués de manière correspondante.

**5.** Procédé selon la revendication 1 pour la préparation de composés de formule I, où $R^0$ désigne un groupe alkyle en $C_1$-$C_4$, hydroxy(alkyle en $C_2$-$C_{14}$), cyano(alkyle en $C_1$-$C_4$), carboxy(alkyle en $C_1$-$C_4$), carboxy(alkyle en $C_1$-$C_4$), alcoxy en $C_1$-$C_4$-carbonyl(alkyle en $C_1$-$C_4$), alcényle en $C_3$-$C_7$ ou phényle, ou leurs sels, dans la mesure où il existe des groupes salifiables, caractérisé en ce qu'on utilise des produits de départ substitués de manière correspondante.

**6.** Procédé selon la revendication 1 pour la préparation de composés de formule I, où R est un groupe acyle ayant au plus 30 atomes de carbone de formule partielle Z-C(=W)-, où W est l'oxygène, le soufre ou un radical imino et Z est l'hydrogène; hydrocarbyle ou hydrocarbyloxy, dans lequel le radical hydrocarbyle est un reste hydrocarboné acyclique, carbocyclique ou carbocyclique-acyclique, qui a au total au plus 18 atomes de carbone et peut être saturé ou insaturé et non-substitué ou substitué, tandis qu'à la place d'un, deux ou plus de deux atomes de carbone peuvent être présents des hétéroatomes identiques ou différents, choisis parmi l'oxygène, le soufre et l'azote; ou un groupe amino; ou, dans le cas où W désigne l'oxygène ou le soufre, peut également être du chlore, ou leurs sels, dans la mesure où il existe des groupes salifiables, caractérisé en ce qu'on utilise des produits de départ substitués de manière correspondante.

**7.** Procédé selon la revendication 6 pour la préparation de composés de formule I, où R est un acyle de formule partielle Z-C(=W)-, où W est l'oxygène ou le soufre et Z représente un alkyle en $C_1$-$C_7$, qui peut également être substitué par un radical halogène, carboxy ou alcoxy($C_1$-$C_4$)carbonyle; phényle, pyridyle, furyle, thiényle, imidazolyle, quinolyle, isoquinolyle, benzofurannyle ou benzimidazolyle, qui peut dans chaque cas être non-substitué ou substitué par un radical alkyle en $C_1$-$C_4$, alcoxy en $C_1$-$C_4$, halogène, nitro, trifluorométhyle, carboxy, alcoxy($C_1$-$C_4$) carbonyle, méthylènedioxy et/ou cyano, ou leurs sels, dans la mesure où il existe des groupes salifiables, caractérisé en ce qu'on utilise des produits de départ substitués de manière correspondante.

**8.** Procédé selon la revendication 1 pour la préparation de composés de formule I, où R représente un acyle dérivé d'un acide sulfonique organique de formule partielle $R^0$-$SO_2$-, dans laquelle $R^0$ est un reste hydrocarboné acyclique, carbocyclique ou carbocyclique-acyclique, qui a au total au plus 30 atomes de carbone et peut être saturé ou insaturé et non-substitué ou susbtitué, tandis qu'à la place d'un, deux ou plus de deux atomes de carbone sont présents des hétéroatomes identiques ou différents, choisis parmi l'oxygène, le soufre et l'azote, ou leurs sels, dans la mesure où il existe des groupes salifiables, caractérisé en ce qu'on utilise des produits de départ substitués de manière correspondante.

**9.** Procédé selon la revendication 1 pour la préparation de composés de formule I, où R représente un acyle dérivé d'acide phosphorique éventuellement estérifié de formule partielle

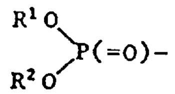

,

où $R^1$ et $R^2$ représentent chacun, indépendamment l'un de l'autre, un hydrogène, un alkyle en $C_1$-$C_7$ non-substitué ou un phényle non-substitué ou substitué par un radical alkyle en $C_1$-$C_4$, alcoxy en $C_1$-$C_4$, halogène et/ou nitro, ou leurs sels, dans la mesure où il existe des groupes salifiables, caractérisé en ce qu'on utilise des produits de départ substitués de manière correspondante.

**10.** Procédé selon la revendication 1 pour la préparation de composés de formule I, où R est un acyle de formule partielle $R^0{}_b$-CO-, dans laquelle $R^0{}_b$ représente l'hydrogène, un alkyle en $C_1$-$C_{19}$, qui pour plus de 5 atomes de carbone présente une chaîne linéaire, ou un tel alkyle substitué par un radical amino, halogène et/ou carboxyle sous forme de l'acide libre, du sel, du groupe cyano ou d'un ester d'alkyle en $C_1$-$C_4$, ou leurs sels, dans la mesure où il existe des groupes salifiables, caractérisé en ce qu'on utilise des produits de départ substitués de manière correspondante.

**11.** Procédé selon la revendication 1 pour la préparation de composés de formule I, où R est un acyle de formule partielle $R^0{}_b$-CO-, où $R^0{}_b$ désigne un alkyle en $C_1$-$C_7$, qui peut être non-substitué ou substitué par un radical halogène, carboxy ou alcoxy($C_1$-$C_4$)carbonyle, ou est un phényle qui peut être non-substitué ou substitué par un radical alkyle en $C_1$-$C_4$, alcoxy en $C_1$-$C_4$, halogène, nitro, trifluorométhyle, carboxy ou alcoxy($C_1$-$C_4$)carbonyle, ou leurs sels, dans la mesure où il existe des groupes salifiables, caractérisé en ce qu'on utilise des produits de départ substitués de manière correspondante.

**12.** Procédé selon la revendication 1 pour la préparation de composés de formule I, où R représente un aroyle bicyclique ou monocyclique, qui peut également porter un ou plusieurs des substituants suivants: atomes d'halogène, groupes nitro, restes alkyle en $C_1$-$C_4$, groupes hydroxyle, groupes alcoxy en $C_1$-$C_4$, phénoxy, méthylènedioxy et/ou carboxyle (ceux-ci également sous forme de sel, en tant que groupe cyano ou qu'ester d'alkyle en $C_1$-$C_4$), ou un hétéroaroyle analogue, dérivé de pyridine, furanne, thiophène ou imidazole ou de leurs analogues ayant un noyau benzénique condensé, ou leurs sels, dans la mesure où il existe des groupes salifiables, caractérisé en ce qu'on utilise des produits de départ substitués de manière correspondante.

**13.** Procédé selon la revendication 1 pour la préparation de composés de formule I, où R est un acyle de formule partielle $R^0$-$SO_2$-, où $R^0$ désigne un radical alkyle en $C_1$-$C_7$, phényle, pyridyle, furyle, thiényle, imidazolyle, quinolyle, isoquinolyle, benzo-furannyle ou benzimidazolyle, qui peut à chaque fois être non-substitué ou substitué par un groupe alkyle en $C_1$-$C_4$, alcoxy en $C_1$-$C_4$, halogène, nitro, trifluorométhyle, carboxy, alcoxy($C_1$--$C_4$)carbonyle, méthylènedioxy et/ou cyano, ou leurs sels, dans la mesure où il existe des groupes salifiables, caractérisé en ce qu'on utilise des produits de départ substitués de manière correspondante.

**14.** Procédé selon la revendication 1 pour la préparation de composés de formule I, où R représente un acyle de formule partielle $R^0$-O-CO- dérivé d'un monoester d'acide carboxylique, où $R^0$ est un alkyle en $C_1$-$C_{20}$, qui peut être non-substitué ou substitué par un groupe carboxyle (également en tant que sel, cyano ou ester d'alkyle en $C_1$-$C_4$), ou un (mono- à hexa-)-oxaalkyle linéaire analogue ayant 4-20 chaînons, ou même un phényle ou un benzyle éventuellement substitué, caractérisé en ce qu'on utilise des produits de départ substitués de manière correspondante.

**15.** Procédé selon la revendication 1 pour la préparation de composés de formule I, où R est un acyle de formule partielle $R^0$-O-CO-, où $R^0$ désigne un radical alkyle en $C_1$-$C_7$, phényle, pyridyle, furyle, thiényle, imidazolyle, quinolyle, isoquinolyle, benzo-furannyle ou benzimidazolyle, qui peut à chaque fois être non-substitué ou substitué par un groupe alkyle en $C_1$-$C_4$, alcoxy en $C_1$-$C_4$, halogène, nitro, trifluorométhyle, carboxy, alcoxy($C_1$-$C_4$)carbonyle, méthylènedioxy et/ou cyano, caractérisé en ce qu'on utilise des produits de départ substitués de manière correspondante.

**16.** Procédé selon la revendication 1 pour la préparation de composés de formule I, où R représente un amide d'acide carboxylique de formule

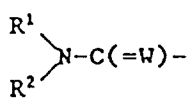

où W désigne l'oxygène, l'un des restes $R^1$ et $R^2$ est l'hydrogène et l'autre un alkyle en $C_1$-$C_7$, qui peut être non-substitué ou substitué par un radical hydroxyle, mercapto, méthylthio, phényle, p-hydroxyphényle, p-méthoxyphényle, 2-indolyle, 2-imidazolyle et/ou carboxyle (libre ou en tant qu'alcoxycarbonyle en $C_1$-$C_4$, carbamoyle ou amidino), dont l'un se trouve en position 1, ou leurs sels, dans la mesure où il existe des groupes salifiables, caractérisé en ce qu'on utilise des produits de départ substitués de manière correspondante.

**17.** Procédé selon la revendication 1 pour la préparation de composés de formule I où R est un acyle de formule partielle

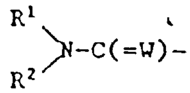

où W désigne le soufre ou l'oxygène, $R^1$ est l'hydrogène et $R^2$ représente un radical alkyle en $C_1$-$C_7$, alcényle en $C_3$-$C_7$, phényle, pyridyle, furyle, thiényle, imidazolyle, quinolyle, isoquinolyle, benzofurannyle ou benzimidazolyle, qui peut à chaque fois être non-substitué ou substitué par un groupe alkyle en $C_1$-$C_4$, alcoxy en $C_1$-$C_4$, halogène, nitro, trifluorométhyle, carboxy, alcoxy($C_1$-$C_4$)carbonyle, méthylènedioxy et/ou cyano, ou leurs sels, dans la mesure où il existe des groupes salifiables, caractérisé en ce qu'on utilise des produits de départ substitués de manière correspondante.

**18.** Procédé selon la revendication 1 pour la préparation de composés de formule I, où R est dérivé d'un acide $\alpha$-aminé choisi parmi la glycine, la phénylglycine, l'alanine, la phénylalanine, la proline, la leucine, la sérine, la valine, la tyrosine, l'arginine, l'histidine et l'asparagine, ou leurs sels, caractérisé en ce qu'on utilise des produits de départ substitués de manière correspondante.

**19.** Procédé selon la revendication 1, caractérisé en ce qu'on prépare un composé de formule I choisi dans le groupe consistant en:

N-(3-carbxoypropionyl)-staurosporine,
N-benzoyl-staurosporine,
N-tridluoroacétyl-staurosporine,
N-méthylaminothiocarbonyl-staurosporine et
N-phénylcarbamoyl-staurosporine, caractérisé en ce qu'on utilise des produits de départ substitués de manière correspondante.

**20.** Procédé selon la revendication 1, caractérisé en ce qu'on prépare un composé de formule I choisi dans le groupe consistant en:

N-(3-nitrobenzoyl)-staurosporine,
N-(3-fluorobenzoyl)-staurosporine,
N-tert-butoxycarbonyl-staurosporine, sel de sodium de N-(4-carboxybenzoyl)-staurosporine,
N-(3,5-dinitrobenzoyl)-staurosporine,
N-[(tert-butoxycarbonylamino)-acétyl]-staurosporine et
N-(2-aminoacétyl)-staurosporine, caractérisé en ce qu'on utilise des produits de départ substitués de manière correspondante.

**21.** Procédé selon la revendication 1, caractérisé en ce qu'on prépare un composé de formule I qui est un sel d'addition de formule

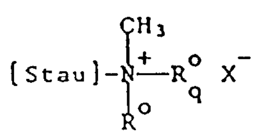

(IA)

où [Stau] et $R^0$ ont les significations indiquées dans les revendications 1 à 7, $R^0_q$ est l'hydrogène ou un radical alkyle en $C_1$-$C_4$ ou benzyle non-substitué et $X^-$ représente un anion d'un acide inorganique ou organique ou d'un carboxyle présent dans le reste $R^0$, caractérisé en ce qu'on utilise des produits de départ substitués de manière correspondante.

**22.** Procédé selon la revendication 1 pour la préparation de N-carboxyméthylstaurosporine ou de l'un de ses sels pharmaceutiquement acceptables, caractérisé en ce qu'on utilise des produits de départ substitués de manière correspondante.

**23.** Procédé selon la revendication 1 pour la préparation de N-éthyl-staurosporine, caractérisé en ce qu'on utilise des produits de départ substitués de manière correspondante.

**24.** Procédé selon la revendication 1 pour la préparation de N-benzoyl-staurosporine, caractérisé en ce qu'on utilise

des produits de départ substitués de manière correspondante.

**25.** Procédé selon la revendication 1 pour la préparation de N-trifluoroacétyl-staurosporine, caractérisé en ce qu'on utilise des produits de départ substitués de manière correspondante.

**26.** Procédé selon la revendication 1 pour la préparation de N-(phénylcarbamoyl)-staurosporine, caractérisé en ce qu'on utilise des produits de départ substitués de manière correspondante.

**27.** Procédé selon la revendication 1 pour la préparation de N-glycyl-staurosporine ou de l'un de ses sels pharmaceutiquement acceptables, caractérisé en ce qu'on utilise des produits de départ substitués de manière correspondante.

**28.** Procédé selon la revendication 1 pour la préparation de N-alanyl-staurosporine ou de l'un de ses sels pharmaceutiquement acceptables, caractérisé en ce qu'on utilise des produits de départ substitués de manière correspondante.

**29.** Procédé pour la préparation d'une composition pharmaceutique contenant comme agent actif au moins un composé défini dans l'une quelconque des revendications 1-28, caractérisé en ce qu'on traite ce composé avec au moins une matière de support pharmaceutique.

**30.** Utilisation d'un composé défini dans l'une quelconque des revendications 1 - 28 pour l'obtention de compositions pharmaceutiques pour le traitement préventif ou curatif de maladies, dans lesquelles l'inhibition de la protéine-kinase C a de l'importance.